# FASCICULE DE BREVET EUROPEEN

(11) **EP 2 563 399 B1**
(45) Date de publication et mention de la délivrance du brevet: **11.06.2014**
(21) Numéro de dépôt: 11719343.3
(22) Date de dépôt: 07.04.2011
(51) Int. Cl.: A61K 47/00, C12N 15/62, A61K 47/42

(54) **UTILISATION DE PEPTIDES COMME TRANSPORTEURS DESTINES A L'INTERNALISATION DE MOLECULES D'INTERET DANS DES CELLULES CIBLES**
VERWENDUNG VON PEPTIDEN ALS TRANSPORTER ZUR INTERNALISIERUNG VON BESTIMMTEN MOKEKÜLEN IN ZIELZELLEN
USE OF PEPTIDES AS TRANSPORTERS INTENDED FOR THE INTERNALIZATION OF MOLECULES OF INTEREST INTO TARGET CELLS

(30) Priorité: 26.04.2010 FR 1053179
(43) Date de publication de la demande: 06.03.2013
(73) Titulaire: Université Joseph Fourier, 38041 Grenoble Cedex 09 (FR)
(72) Inventeur: LENORMAND, Jean-Luc, Erwan, Claude, Francis, F-38700 La Tronche (FR); ROTHE-WALTHER,Romy, F-31500 Toulouse (FR)
(74) Mandataire: Grosset-Fournier, Chantal Catherine
(86) Numéro de dépôt international: PCT/FR2011/050794
(87) Numéro de publication internationale: WO 2011/135222

(56) Documents cités:
- ROMY ROTHE ET AL: "Characterization of the Cell-penetrating Properties of the Epstein-Barr Virus ZEBRA trans-Activator", JOURNAL OF BIOLOGICAL CHEMISTRY, AMERICAN SOCIETY FOR BIOCHEMISTRY AND MOLECULAR BIOLOGY, INC, US, vol. 285, no. 26, 9 avril 2010 (2010-04-09), - 25 juin 2010 (2010-06-25), pages 20224-20233, XP007916069, e-pub 09-04-2010 ISSN: 0021-9258, DOI: DOI:10.1074/JBC.M110.101550 [extrait le 2010-04-09]
- HARADA HIROSHI ET AL: "Antitumor protein therapy; application of the protein transduction domain to the development of a protein drug for cancer treatment.", BREAST CANCER (TOKYO, JAPAN) 2006 LNKD- PUBMED:16518058, vol. 13, no. 1, 2006, pages 16-26, XP002544010, ISSN: 1340-6868
- ROTHE ROMY ET AL: "Expression and purification of ZEBRA fusion proteins and applications for the delivery of macromolecules into mammalian cells.", CURRENT PROTOCOLS IN PROTEIN SCIENCE / EDITORIAL BOARD, JOHN E. COLIGAN ... [ET AL.] NOV 2008 LNKD- PUBMED:19016434, vol. Chapter 18, novembre 2008 (2008-11), XP007916063, ISSN: 1934-3663
- PHAN ET AL: "Identification of a translation initiation factor 3 (eIF3) core complex, conserved in yeast and mammals, that interacts with eIF5.", MOLECULAR AND CELLULAR BIOLOGY, vol. 18, no. 8, 1 août 1998 (1998-08-01), pages 4935-4946, XP55007751, ISSN: 0270-7306
- MAMIKO MASUTANI ET AL: "Reconstitution reveals the functional core of mammalian eIF3", THE EMBO JOURNAL, vol. 26, no. 14, 25 juillet 2007 (2007-07-25), pages 3373-3383, XP55008262, ISSN: 0261-4189, DOI: 10.1038/sj.emboj.7601765
- A. K. LEFEBVRE: "Translation Initiation Factor eIF4G-1 Binds to eIF3 through the eIF3e Subunit", JOURNAL OF BIOLOGICAL CHEMISTRY, vol. 281, no. 32, 1 janvier 2006 (2006-01-01), pages 22917-22932, XP55007747, ISSN: 0021-9258, DOI: 10.1074/jbc.M605418200
- BROWNING K S ET AL: "Unified nomenclature for the subunits of eukaryotic initiation factor 3<1>", TRENDS IN BIOCHEMICAL SCIENCES, ELSEVIER, HAYWARDS, GB, vol. 26, no. 5, 1 mai 2001 (2001-05-01), page 284, XP004241870, ISSN: 0968-0004, DOI: 10.1016/S0968-0004(01)01825-4
- SNYDER ERIC L ET AL: "Enhanced targeting and killing of tumor cells expressing the CXC chemokine receptor 4 by transducible anticancer peptides.", CANCER RESEARCH 1 DEC 2005 LNKD- PUBMED:16322205, vol. 65, no. 23, 1 décembre 2005 (2005-12-01), pages 10646-10650, XP002613447, ISSN: 0008-5472
- FOGED CAMILLA ET AL: "Cell-penetrating peptides for drug delivery across membrane barriers", EXPERT OPINION ON DRUG DELIVERY, INFORMA HEALTHCARE, GB, vol. 5, no. 1, 1 janvier 2008 (2008-01-01) , pages 105-117, XP008090485, ISSN: 1742-5247, DOI: DOI:10.1517/17425247.5.1.105
- MURRIEL CHRISTOPHER L ET AL: "Influence of protein transduction domains on intracellular delivery of macromolecules", EXPERT OPINION ON DRUG DELIVERY, INFORMA HEALTHCARE, GB, vol. 3, no. 6, 1 novembre 2006 (2006-11-01), pages 739-746, XP008107388, ISSN: 1742-5247, DOI: DOI:10.1517/17425247.3.6.739
- MORRIS M C ET AL: "A peptide carrier for the delivery of biologically active proteins into mammalian cells", NATURE BIOTECHNOLOGY, NATURE PUBLISHING GROUP, NEW YORK, NY, US, vol. 19, 1 décembre 2001 (2001-12-01), pages 1173-1176, XP002559236, ISSN: 1087-0156, DOI: DOI:10.1038/NBT1201-1173
- DONG Z ET AL: "Initiation factor eIF3 and regulation of mRNA translation, cell growth, and cancer", CRITICAL REVIEWS IN ONCOLOGY / HEMATOLOGY, ELSEVIER SCIENCE IRELAND LTD., LIMERICK, IE, vol. 59, no. 3, 1 septembre 2006 (2006-09-01), pages 169-180, XP024907211, ISSN: 1040-8428, DOI: 10.1016/J.CRITREVONC.2006.03.005 [extrait le 2006-09-01]
- FAIS S ET AL: "The role of FAS to ezrin association in FAS-mediated apoptosis", APOPTOSIS ; AN INTERNATIONAL JOURNAL ON PROGRAMMED CELL DEATH, KLUWER ACADEMIC PUBLISHERS, BO, vol. 10, no. 5, 1 octobre 2005 (2005-10-01), pages 941-947, XP019204742, ISSN: 1573-675X, DOI: 10.1007/S10495-005-0478-2
- CHAUHAN S ET AL: "Androgen regulation of the human FERM domain encoding gene EHM2 in a cell model of steroid-induced differentiation", BIOCHEMICAL AND BIOPHYSICAL RESEARCH COMMUNICATIONS, ACADEMIC PRESS INC. ORLANDO, FL, US, vol. 310, no. 2, 17 octobre 2003 (2003-10-17), pages 421-432, XP004458960, ISSN: 0006-291X, DOI: 10.1016/J.BBRC.2003.08.147
- SSANG-TAEK LIM ET AL: "FERM control of FAK function: implications for cancer therapy.", CELL CYCLE, vol. 7, no. 15, 1 août 2008 (2008-08-01), pages 2306-2314, XP55018297, ISSN: 1538-4101

### SEQUENCE LISTING

<110> Université Joseph Fourier
<120> Utilisation de peptide comme transporteurs destinés à l'internalisation de molécules d'intérêt dans des cellules cibles
<130> 09 BH UJF ZEBR
<160> 56
<170> PatentIn version 3.5
<210> 1
   <211> 51
   <212> PRT
   <213> Epstein Barr virus
<400> 1
   Glu Cys Asp Ser Glu Leu Glu Ile Lys Arg Tyr Lys Asn Arg Val Ala
   1               5                   10                  15
   Ser Arg Lys Cys Arg Ala Lys Phe Lys Gln Leu Leu Gln His Tyr Arg
               20                  25                  30
   Glu Val Ala Ala Ala Lys Ser Ser Glu Asn Asp Arg Leu Arg Leu Leu
           35                  40                  45
   Leu Lys Gln
       50
<210> 2
   <211> 298
   <212> PRT
   <213> Xenopus
<400> 2
   Met Arg His Met Gln Ser Ala Thr Arg Ala Thr Leu Val Cys Gly Ser
   1               5                   10                  15
   Gly Val Lys Gln Ile Ile Ala Lys Gly His Pro Asn Thr Arg Val Phe
               20                  25                  30
   Gly Ala Arg Lys Ala Arg Ile Pro Glu Arg Glu Val Leu Ala Ala Lys
           35                  40                  45
   Pro Lys Ile Thr Arg Ile Thr His Leu Asn Leu Gln Pro Gln Glu Arg
       50                  55                  60
   Gln Ala Phe Tyr Arg Leu Leu Glu Asn Glu Leu Ile Gln Glu Phe Leu
   65                  70                  75                  80
   Ser Met Asp Ser Cys Leu Lys Ile Ser Asp Lys Tyr Leu Ile Ala Met
                   85                  90                  95
   Val Leu Ala Tyr Phe Lys Arg Ala Gly Leu Tyr Thr Gly Glu Tyr Thr
   100 105 110
   Thr Met Asn Phe Phe Val Ala Leu Tyr Leu Ala Asn Asp Met Glu Glu
   115 120 125
   Asp Glu Glu Asp Tyr Lys Tyr Glu Ile Phe Pro Trp Ala Leu Gly Asp
   130 135 140
   Ser Trp Arg Glu Phe Phe Pro Gln Phe Leu Arg Leu Arg Asp Asn Phe
   145 150 155 160
   Trp Ala Lys Met Asn Tyr Arg Ala Val Val Ser Arg Arg Cys Cys Asp
   165 170 175
   Glu Val Met Ala Lys Asp Pro Thr His Trp Ala Trp Leu Arg Asp Arg
   180 185 190
   Pro Ile His His Ser Gly Ala Leu Arg Gly Tyr Leu Arg Asn Glu Asp
   195 200 205
   Asp Phe Phe Pro Arg Gly Pro Gly Leu Thr Pro Ala Ser Cys Ala Leu
   210 215 220
   Cys His Lys Ala Ser Val Cys Asp Ser Gly Gly Val Ala His Asp Asn
   225 230 235 240
   Ser Ser Pro Glu Gln Glu Ile Phe His Tyr Thr Asn Arg Glu Trp Ser
   245 250 255
   Gln Glu Leu Leu Ile Leu Pro Pro Glu Leu Leu Leu Asp Pro Glu Ser
   260 265 270
   Thr Tyr Asp Ile His Ile Phe Gln Glu Pro Leu Val Gly Leu Glu Pro
   275 280 285
   Asp Gly Ala Ala Leu Glu Trp His His Leu
   290 295
<210> 3
   <211> 131
   <212> PRT
   <213> Homo sapiens
<400> 3
   Gln Glu Val Gln Ala Phe Leu Ser Leu Leu Glu Asp Ser Phe Val Gln
   1 5 10 15
   Glu Phe Leu Ser Lys Asp Pro Cys Phe Gln Ile Ser Asp Lys Tyr Leu
   20 25 30
   Leu Ala Met Val Leu Val Tyr Phe Gln Arg Ala His Leu Lys Leu Ser
   35 40 45
   Glu Tyr Thr His Ser Ser Leu Phe Leu Ala Leu Tyr Leu Ala Asn Asp
   50 55 60
   Met Glu Glu Asp Leu Glu Gly Pro Lys Cys Glu Ile Phe Pro Trp Ala
   65 70 75 80
   Leu Gly Lys Asp Trp Cys Leu Arg Val Gly Lys Phe Leu His Gln Arg
   85 90 95
   Asp Lys Leu Trp Ala Arg Met Gly Phe Arg Ala Val Val Ser Arg Gln
   100 105 110
   Cys Cys Glu Glu Val Met Ala Lys Glu Pro Phe His Trp Ala Trp Thr
   115 120 125
   Arg Asp Arg
   130
<210> 4
   <211> 131
   <212> PRT
   <213> Mus musculus
<400> 4
   Gln Glu Met Thr Ala Phe Phe Lys Leu Phe Asp Asp Asp Leu Ile Gln
   1               5                   10                  15
   Asp Phe Leu Trp Met Asp Cys Cys Cys Lys Ile Ala Asp Lys Tyr Leu
               20                  25                  30
   Leu Ala Met Thr Phe Val Tyr Phe Lys Arg Ala Lys Phe Thr Ile Asn
           35                  40                  45
   Glu His Thr Arg Ile Asn Phe Phe Ile Ala Leu Tyr Leu Ala Asn Thr
       50                  55                  60
   Val Glu Glu Asp Glu Glu Glu Ala Lys Tyr Glu Ile Phe Pro Trp Ala
   65                  70                  75                  80
   Leu Gly Lys Asn Trp Arg Lys Leu Phe Pro Asn Phe Leu Lys Leu Arg
                   85                  90                  95
   Asp Gln Leu Trp Asp Arg Ile Asp Tyr Arg Ala Ile Val Ser Arg Arg
               100                 105                 110
   Cys Cys Glu Glu Val Met Ala Ile Ala Pro Thr His Tyr Ile Trp Gln
           115                 120                 125
   Arg Glu Arg
       130
<210> 5
   <211> 133
   <212> PRT
   <213> Xenopus
<400> 5
   Pro Gln Glu Arg Gln Ala Phe Tyr Arg Leu Leu Glu Asn Glu Leu Ile
   1 5 10 15
   Gln Glu Phe Leu Ser Met Asp Ser Cys Leu Lys Ile Ser Asp Lys Tyr
   20 25 30
   Leu Ile Ala Met Val Leu Ala Tyr Phe Lys Arg Ala Gly Leu Tyr Thr
   35 40 45
   Gly Glu Tyr Thr Thr Met Asn Phe Phe Val Ala Leu Tyr Leu Ala Asn
   50 55 60
   Asp Met Glu Glu Asp Glu Glu Asp Tyr Lys Tyr Glu Ile Phe Pro Trp
   65 70 75 80
   Ala Leu Gly Asp Ser Trp Arg Glu Phe Phe Pro Gln Phe Leu Arg Leu
   85 90 95
   Arg Asp Asn Phe Trp Ala Lys Met Asn Tyr Arg Ala Val Val Ser Arg
   100 105 110
   Arg Cys Cys Asp Glu Val Met Ala Lys Asp Pro Thr His Trp Ala Trp
   115 120 125
   Leu Arg Asp Arg Pro
   130
<210> 6
   <211> 133
   <212> PRT
   <213> Xenopus
<400> 6
   Pro Gln Glu Arg Gln Ala Phe Tyr Arg Leu Leu Glu Asn Glu Leu Ile
   1 5 10 15
   Gln Glu Phe Leu Ser Met Asp Ser Cys Leu Lys Ile Ser Asp Lys Tyr
   20 25 30
   Leu Ile Ala Met Val Leu Ala Tyr Phe Lys Arg Ala Gly Leu Tyr Thr
   35 40 45
   Gly Glu Tyr Thr Thr Met Asn Phe Phe Val Ala Leu Tyr Leu Ala Asn
   50 55 60
   Asp Met Glu Glu Asp Glu Glu Asp Tyr Lys Tyr Glu Ile Phe Pro Trp
   65 70 75 80
   Ala Leu Gly Asp Ser Trp Arg Glu Phe Phe Pro Gln Phe Leu Arg Leu
   85 90 95
   Arg Asp Asn Phe Trp Ala Lys Met Asn Tyr Arg Ala Val Val Ser Arg
   100 105 110
   Arg Cys Cys Asp Glu Val Met Ala Lys Asp Pro Thr His Trp Ala Trp
   115 120 125
   Leu Arg Asp Arg Pro
   130
<210> 7
   <211> 131
   <212> PRT
   <213> Drosophila
<400> 7
   Gln Glu Met Thr Ala Phe Phe Lys Leu Phe Asp Asp Asp Leu Ile Gln
   1               5                   10                  15
   Asp Phe Leu Trp Met Asp Cys Cys Cys Lys Ile Ala Asp Lys Tyr Leu
               20                  25                  30
   Leu Ala Met Thr Phe Val Tyr Phe Lys Arg Ala Lys Phe Thr Ile Asn
           35                  40                  45
   Glu His Thr Arg Ile Asn Phe Phe Ile Ala Leu Tyr Leu Ala Asn Thr
       50                  55                  60
   Val Glu Glu Asp Glu Glu Glu Ala Lys Tyr Glu Ile Phe Pro Trp Ala
   65                  70                  75                  80
   Leu Gly Lys Asn Trp Arg Lys Leu Phe Pro Asn Phe Leu Lys Leu Arg
                   85                  90                  95
   Asp Gln Leu Trp Asp Arg Ile Asp Tyr Arg Ala Ile Val Ser Arg Arg
               100                 105                 110
   Cys Cys Glu Glu Val Met Ala Ile Ala Pro Thr His Tyr Ile Trp Gln
           115                 120                 125
   Arg Glu Arg
       130
<210> 8
   <211> 133
   <212> PRT
   <213> Artificial sequence
<220>
   <223> dérivé de protéine humaine
<220>
   <221> MISC_FEATURE
   <222> (1) .. (6)
   <223> X est choisi parmi A, R, N, D, C, E, S, Q, G, H, I, L, K, M, F, P, S, T, W, Y, V,
<220>
   <221> MISC_FEATURE
   <222> (7) .. (8)
   <223> X est choisi parmi A, R, N, D, C, E, S, Q, G, H, I, L, K, M, F, P, S, T, W, Y, V,
<220>
   <221> misc_feature
   <222> (9)..(9)
   <223> Xaa can be any naturally occurring amino acid
<220>
   <221> MISC_FEATURE
   <222> (10) .. (17)
   <223> X est choisi parmi A, R, N, D, C, E, S, Q, G, H, I, L, K, M, F, P, S, T, W, Y, V,
<220>
   <221> misc_feature
   <222> (18) .. (18)
   <223> Xaa can be any naturally occurring amino acid
<220>
   <221> MISC_FEATURE
   <222> (20) .. (21)
   <223> X est choisi parmi A, R, N, D, C, E, S, Q, G, H, I, L, K, M, F, P, S, T, W, Y, V,
<220>
   <221> misc_feature
   <222> (22) .. (22)
   <223> Xaa can be any naturally occurring amino acid
<220>
   <221> MISC_FEATURE
   <222> (23) .. (28)
   <223> X est choisi parmi A, R, N, D, C, E, S, Q, G, H, I, L, K, M, F, P, S, T, W, Y, V,
<220>
   <221> misc_feature
   <222> (29) .. (29)
   <223> Xaa can be any naturally occurring amino acid
<220>
   <221> MISC_FEATURE
   <222> (33) .. (34)
   <223> X est choisi parmi A, R, N, D, C, E, S, Q, G, H, I, L, K, M, F, P, S, T, W, Y, V,
<220>
   <221> misc_feature
   <222> (35) .. (35)
   <223> Xaa can be any naturally occurring amino acid
<220>
   <221> MISC_FEATURE
   <222> (36) .. (38)
   <223> X est choisi parmi A, R, N, D, C, E, S, Q, G, H, I, L, K, M, F, P, S, T, W, Y, V,
<220>
   <221> misc_feature
   <222> (39) .. (39)
   <223> Xaa can be any naturally occurring amino acid
<220>
   <221> MISC_FEATURE
   <222> (41) .. (41)
   <223> X est choisi parmi A, R, N, D, C, E, S, Q, G, H, I, L, K, M, F, P, S, T, W, Y, V,
<220>
   <221> misc_feature
   <222> (42)..(42)
   <223> Xaa can be any naturally occurring amino acid
<220>
   <221> MISC_FEATURE
   <222> (44) .. (55)
   <223> X est choisi parmi A, R, N, D, C, E, S, Q, G, H, I, L, K, M, F, P, S, T, W, Y, V,
<220>
   <221> misc_feature
   <222> (56)..(56)
   <223> Xaa can be any naturally occurring amino acid
<220>
   <221> MISC_FEATURE
   <222> (57) .. (57)
   <223> X est choisi parmi A, R, N, D, C, E, S, Q, G, H, I, L, K, M, F, P, S, T, W, Y, V,
<220>
   <221> misc_feature
   <222> (58) .. (58)
   <223> Xaa can be any naturally occurring amino acid
<220>
   <221> MISC_FEATURE
   <222> (64)..(66)
   <223> X est choisi parmi A, R, N, D, C, E, S, Q, G, H, I, L, K, M, F, P, S, T, W, Y, V,
<220>
   <221> misc_feature
   <222> (67) .. (67)
   <223> Xaa can be any naturally occurring amino acid
<220>
   <221> MISC_FEATURE
   <222> (68) .. (72)
   <223> X est choisi parmi A, R, N, D, C, E, S, Q, G, H, I, L, K, M, F, P, S, T, W, Y, V,
<220>
   <221> misc_feature
   <222> (73) .. (73)
   <223> Xaa can be any naturally occurring amino acid
<220>
   <221> MISC_FEATURE
   <222> (74) .. (75)
   <223> X est choisi parmi A, R, N, D, C, E, S, Q, G, H, I, L, K, M, F, P, S, T, W, Y, V,
<220>
   <221> MISC_FEATURE
   <222> (78) .. (81)
   <223> X est choisi parmi A, R, N, D, C, E, S, Q, G, H, I, L, K, M, F, P, S, T, W, Y, V,
<220>
   <221> misc_feature
   <222> (83) .. (83)
   <223> Xaa can be any naturally occurring amino acid
<220>
   <221> MISC_FEATURE
   <222> (84) .. (93)
   <223> X est choisi parmi A, R, N, D, C, E, S, Q, G, H, I, L, K, M, F, P, S, T, W, Y, V,
<220>
   <221> misc_feature
   <222> (94)..(94)
   <223> Xaa can be any naturally occurring amino acid
<220>
   <221> MISC_FEATURE
   <222> (95) .. (97)
   <223> X est choisi parmi A, R, N, D, C, E, S, Q, G, H, I, L, K, M, F, P, S, T, W, Y, V,
<220>
   <221> misc_feature
   <222> (98) .. (98)
   <223> Xaa can be any naturally occurring amino acid
<220>
   <221> MISC_FEATURE
   <222> (99) .. (110)
   <223> X est choisi parmi A, R, N, D, C, E, S, Q, G, H, I, L, K, M, F, P, S, T, W, Y, V,
<220>
   <221> misc_feature
   <222> (113)..(113)
   <223> Xaa can be any naturally occurring amino acid
<220>
   <221> MISC_FEATURE
   <222> (114)..(115)
   <223> X est choisi parmi A, R, N, D, C, E, S, Q, G, H, I, L, K, M, F, P, S, T, W, Y, V,
<220>
   <221> misc_feature
   <222> (116)..(116)
   <223> Xaa can be any naturally occurring amino acid
<220>
   <221> MISC_FEATURE
   <222> (117)..(117)
   <223> X est choisi parmi A, R, N, D, C, E, S, Q, G, H, I, L, K, M, F, P, S, T, W, Y, V,
<220>
   <221> misc_feature
   <222> (118)..(118)
   <223> Xaa can be any naturally occurring amino acid
<220>
   <221> MISC_FEATURE
   <222> (119)..(128)
   <223> X est choisi parmi A, R, N, D, C, E, S, Q, G, H, I, L, K, M, F, P, S, T, W, Y, V,
<220>
   <221> misc_feature
   <222> (129)..(129)
   <223> Xaa can be any naturally occurring amino acid
<220>
   <221> MISC_FEATURE
   <222> (130)..(130)
   <223> X est choisi parmi A, R, N, D, C, E, S, Q, G, H, I, L, K, M, F, P, S, T, W, Y, V,
<220>
   <221> misc_feature
   <222> (131)..(131)
   <223> Xaa can be any naturally occurring amino acid
<220>
   <221> MISC_FEATURE
   <222> (132)..(132)
   <223> X est choisi parmi A, R, N, D, C, E, S, Q, G, H, I, L, K, M, F, P, S, T, W, Y, V,
<220>
   <221> misc_feature
   <222> (133)..(133)
   <223> Xaa can be any naturally occurring amino acid
<220>
   <221> MISC_FEATURE
   <222> (134)..(134)
   <223> X est choisi parmi A, R, N, D, C, E, S, Q, G, H, I, L, K, M, F, P, S, T, W, Y, V,
<400> 8
   Xaa Xaa Xaa Xaa Xaa Xaa Phe Xaa Xaa Leu Xaa Xaa Xaa Xaa Xaa Xaa
   1               5                   10                  15
   Xaa Xaa Leu Phe Xaa Xaa Asp Xaa Xaa Xaa Xaa Xaa Xaa Asp Lys Tyr
               20                  25                  30
   Leu Xaa Xaa Met Xaa Xaa Xaa Tyr Phe Xaa Arg Ala Xaa Xaa Xaa Xaa
           35                  40                  45
   Xaa Xaa Xaa Xaa Xaa Xaa Xaa Xaa Phe Xaa Ala Leu Tyr Leu Ala Asn
       50                  55                  60
   Xaa Xaa Xaa Glu Asp Xaa Xaa Xaa Xaa Lys Xaa Ile Phe Xaa Xaa Xaa
   65                  70                  75                  80
   Xaa Gly Xaa Xaa Xaa Xaa Xaa Xaa Xaa Xaa Xaa Xaa Phe Xaa Xaa Xaa
                   85                  90                  95
   Arg Xaa Xaa Xaa Xaa Xaa Xaa Xaa Xaa Xaa Xaa Xaa Xaa Val Ser Arg
               100                 105                 110
   Xaa Xaa Cys Xaa Glu Xaa Xaa Xaa Xaa Xaa Xaa Xaa Xaa Xaa Xaa Trp
           115                 120                 125
   Xaa Arg Xaa Arg Xaa
       130
<210> 9
   <211> 411
   <212> PRT
   <213> Rattus norvegicus
<400> 9
   Met Pro Arg Glu Arg Lys Glu Arg Asp Ser Lys Asp His Ser Asn Met
   1               5                   10                  15
   Lys Glu Glu Gly Gly Ser Asp Leu Ser Val Arg Ser Arg Lys Arg Lys
               20                  25                  30
   Ala Asn Val Ala Val Phe Leu Gln Asp Pro Asp Glu Glu Ile Ala Lys
           35                  40                  45
   Ile Asp Lys Thr Val Lys Ser Gln Asp Ser Ser Gln Pro Trp Asp Asp
       50                  55                  60
   Asp Ser Ala Cys Val Asp Pro Cys Ser Phe Ile Pro Thr Pro Asn Lys
   65                  70                  75                  80
   Glu Glu Asp Asn Glu Leu Glu Tyr Pro Lys Thr Ala Phe Gln Pro Arg
                   85                  90                  95
   Lys Ile Arg Pro Pro Arg Ala Ser Pro Leu Pro Val Leu Asn Trp Gly
               100                 105                 110
   Asn Arg Glu Glu Val Trp Arg Ile Met Leu Asn Lys Glu Lys Thr Tyr
           115                 120                 125
   Leu Arg Asp Glu His Phe Leu Gln Arg His Pro Leu Leu Gln Ala Arg
       130                 135                 140
   Met Arg Ala Val Leu Leu Asp Trp Leu Met Glu Val Cys Glu Val Tyr
   145                 150                 155                 160
   Lys Leu His Arg Glu Thr Phe Tyr Leu Ala Gln Asp Phe Phe Asp Arg
                   165                 170                 175
   Tyr Met Ala Ser Gln Gln Asn Ile Ile Lys Thr Leu Leu Gln Leu Ile
               180                 185                 190
   Gly Ile Ser Ala Leu Phe Ile Ala Ser Lys Leu Glu Glu Ile Tyr Pro
           195                 200                 205
   Pro Lys Leu His Gln Phe Ala Tyr Val Thr Asp Gly Ala Cys Ser Gly
       210                 215                 220
   Asp Glu Ile Leu Thr Met Glu Leu Met Met Met Lys Ala Leu Lys Trp
   225                 230                 235                 240
   Arg Leu Ser Pro Leu Thr Ile Val Ser Trp Leu Asn Val Tyr Val Gln
                   245                 250                 255
   Val Ala Tyr Val Asn Asp Thr Gly Glu Val Leu Met Pro Gln Tyr Pro
               260                 265                 270
   Gln Gln Val Phe Val Gln Ile Ala Glu Leu Leu Asp Leu Cys Val Leu
           275                 280                 285
   Asp Val Gly Cys Leu Glu Phe Pro Tyr Gly Val Leu Ala Ala Ser Ala
       290                 295                 300
   Leu Tyr His Phe Ser Ser Leu Glu Leu Met Gln Lys Val Ser Gly Tyr
   305                 310                 315                 320
   Gln Trp Cys Asp Ile Glu Lys Cys Val Lys Trp Met Val Pro Phe Ala
                   325                 330                 335
   Met Val Ile Arg Glu Met Gly Ser Ser Lys Leu Lys His Phe Arg Gly
               340                 345                 350
   Val Pro Met Glu Asp Ser His Asn Ile Gln Thr His Thr Asn Ser Leu
   355 360 365
   Asp Leu Leu Asp Lys Ala Gln Ala Lys Lys Ala Ile Leu Ser Glu Gln
   370 375 380
   Asn Arg Ile Ser Pro Pro Pro Ser Gly Val Leu Thr Pro Pro His Ser
   385 390 395 400
   Ser Lys Lys Gln Ser Ser Glu Gln Glu Thr Glu
   405 410
<210> 10
   <211> 20
   <212> PRT
   <213> Rattus norvegicus
<400> 10
   Glu Leu Met Met Met Lys Ala Leu Lys Trp Arg Leu Ser Pro Leu Thr
   1 5 10 15
   Ile Val Ser Trp
   20
<210> 11
   <211> 20
   <212> PRT
   <213> Mus musculus
<400> 11
   Glu Leu Met Met Met Lys Ala Leu Lys Trp Arg Leu Ser Pro Leu Thr
   1 5 10 15
   Ile Val Ser Trp
   20
<210> 12
   <211> 20
   <212> PRT
   <213> Mus musculus
<400> 12
   Glu Leu Met Met Met Lys Ala Leu Lys Trp Arg Leu Ser Pro Leu Thr
   1 5 10 15
   Ile Val Ser Trp
   20
<210> 13
   <211> 20
   <212> PRT
   <213> Homo sapiens
<400> 13
   Glu Leu Met Ile Met Lys Ala Leu Lys Trp Arg Leu Ser Pro Leu Thr
   1               5                   10                  15
   Ile Val Ser Trp
               20
<210> 14
   <211> 20
   <212> PRT
   <213> Homo sapiens
<400> 14
   Glu Leu Met Ile Met Lys Ala Leu Lys Trp Arg Leu Ser Pro Leu Thr
   1               5                   10                  15
   Ile Val Ser Trp
               20
<210> 15
   <211> 319
   <212> PRT
   <213> Mus musculus
<400> 15
   Met Asp Leu Ser Ala Ile Tyr Glu Ser Leu Gln Ser Met Ser His Asp
   1               5                   10                  15
   Leu Ser Ser Asp His Gly Gly Thr Glu Ser Leu Gly Gly Leu Trp Asn
               20                  25                  30
   Ile Asn Ser Asp Ser Ile Pro Ser Gly Val Thr Ser Arg Leu Thr Gly
           35                  40                  45
   Arg Ser Thr Ser Leu Val Glu Gly Arg Ser Cys Gly Trp Val Pro Pro
       50                  55                  60
   Pro Pro Gly Phe Ala Pro Leu Ala Pro Arg Pro Gly Pro Glu Leu Ser
   65                  70                  75                  80
   Pro Ser Pro Thr Ser Pro Thr Ala Thr Pro Thr Thr Ser Ser Arg Tyr
                   85                  90                  95
   Lys Thr Glu Leu Cys Arg Thr Tyr Ser Glu Ser Gly Arg Cys Arg Tyr
               100                 105                 110
   Gly Ala Lys Cys Gln Phe Ala His Gly Leu Gly Glu Leu Arg Gln Ala
           115                 120                 125
   Asn Arg His Pro Lys Tyr Lys Thr Glu Leu Cys His Lys Phe Tyr Leu
   130 135 140
   Gln Gly Arg Cys Pro Tyr Gly Ser Arg Cys His Phe Ile His Asn Pro
   145 150 155 160
   Thr Glu Asp Leu Ala Leu Pro Gly Gln Pro His Val Leu Arg Gln Ser
   165 170 175
   Ile Ser Phe Ser Gly Leu Pro Ser Gly Arg Arg Ser Ser Pro Pro Pro
   180 185 190
   Pro Gly Phe Ser Gly Pro Ser Leu Ser Ser Cys Ser Phe Ser Pro Ser
   195 200 205
   Ser Ser Pro Pro Pro Pro Gly Asp Leu Pro Leu Ser Pro Ser Ala Phe
   210 215 220
   Ser Ala Ala Pro Gly Thr Pro Val Thr Arg Arg Asp Pro Asn Gln Ala
   225 230 235 240
   Cys Cys Pro Ser Cys Arg Arg Ser Thr Thr Pro Ser Thr Ile Trp Gly
   245 250 255
   Pro Leu Gly Gly Leu Ala Arg Ser Pro Ser Ala His Ser Leu Gly Ser
   260 265 270
   Asp Pro Asp Asp Tyr Ala Ser Ser Gly Ser Ser Leu Gly Gly Ser Asp
   275 280 285
   Ser Pro Val Phe Glu Ala Gly Val Phe Gly Pro Pro Gln Thr Pro Ala
   290 295 300
   Pro Pro Arg Arg Leu Pro Ile Phe Asn Arg Ile Ser Val Ser Glu
   305 310 315
<210> 16
   <211> 326
   <212> PRT
   <213> Homo sapiens
<400> 16
   Met Asp Leu Thr Ala Ile Tyr Glu Ser Leu Leu Ser Leu Ser Pro Asp
   1               5                   10                  15
   Val Pro Val Pro Ser Asp His Gly Gly Thr Glu Ser Ser Pro Gly Trp
               20                  25                  30
   Gly Ser Ser Gly Pro Trp Ser Leu Ser Pro Ser Asp Ser Ser Pro Ser
           35                  40                  45
   Gly Val Thr Ser Arg Leu Pro Gly Arg Ser Thr Ser Leu Val Glu Gly
       50                  55                  60
   Arg Ser Cys Gly Trp Val Pro Pro Pro Pro Gly Phe Ala Pro Leu Ala
   65                  70                  75                  80
   Pro Arg Leu Gly Pro Glu Leu Ser Pro Ser Pro Thr Ser Pro Thr Ala
                   85                  90                  95
   Thr Ser Thr Thr Pro Ser Arg Tyr Lys Thr Glu Leu Cys Arg Thr Phe
               100                 105                 110
   Ser Glu Ser Gly Arg Cys Arg Tyr Gly Ala Lys Cys Gln Phe Ala His
           115                 120                 125
   Gly Leu Gly Glu Leu Arg Gln Ala Asn Arg His Pro Lys Tyr Lys Thr
       130                 135                 140
   Glu Leu Cys His Lys Phe Tyr Leu Gln Gly Arg Cys Pro Tyr Gly Ser
   145                 150                 155                 160
   Arg Cys His Phe Ile His Asn Pro Ser Glu Asp Leu Ala Ala Pro Gly
                   165                 170                 175
   His Pro Pro Val Leu Arg Gln Ser Ile Ser Phe Ser Gly Leu Pro Ser
               180                 185                 190
   Gly Arg Arg Thr Ser Pro Pro Pro Pro Gly Leu Ala Gly Pro Ser Leu
           195                 200                 205
   Ser Ser Ser Ser Phe Ser Pro Ser Ser Ser Pro Pro Pro Pro Gly Asp
       210                 215                 220
   Leu Pro Leu Ser Pro Ser Ala Phe Ser Ala Ala Pro Gly Thr Pro Leu
   225                 230                 235                 240
   Ala Arg Arg Asp Pro Thr Pro Val Cys Cys Pro Ser Cys Arg Arg Ala
                   245                 250                 255
   Thr Pro Ile Ser Val Trp Gly Pro Leu Gly Gly Leu Val Arg Thr Pro
               260                 265                 270
   Ser Val Gln Ser Leu Gly Ser Asp Pro Asp Glu Tyr Ala Ser Ser Gly
           275                 280                 285
   Ser Ser Leu Gly Gly Ser Asp Ser Pro Val Phe Glu Ala Gly Val Phe
       290                 295                 300
   Ala Pro Pro Gln Pro Val Ala Ala Pro Arg Arg Leu Pro Ile Phe Asn
   305 310 315 320
   Arg Ile Ser Val Ser Glu
   325
<210> 17
   <211> 355
   <212> PRT
   <213> Homo sapiens
<400> 17
   Met Pro Phe Leu Gly Gln Asp Trp Arg Ser Pro Gly Gln Asn Trp Val
   1               5                   10                  15
   Lys Thr Ala Asp Gly Trp Lys Arg Phe Leu Asp Glu Lys Ser Gly Ser
               20                  25                  30
   Phe Val Ser Asp Leu Ser Ser Tyr Cys Asn Lys Glu Val Tyr Asn Lys
           35                  40                  45
   Glu Asn Leu Phe Asn Ser Leu Asn Tyr Asp Val Ala Ala Lys Lys Arg
       50                  55                  60
   Lys Lys Asp Met Leu Asn Ser Lys Thr Lys Thr Gln Tyr Phe His Gln
   65                  70                  75                  80
   Glu Lys Trp Ile Tyr Val His Lys Gly Ser Thr Lys Glu Arg His Gly
                   85                  90                  95
   Tyr Cys Thr Leu Gly Glu Ala Phe Asn Arg Leu Asp Phe Ser Thr Ala
               100                 105                 110
   Ile Leu Asp Ser Arg Arg Phe Asn Tyr Val Val Arg Leu Leu Glu Leu
           115                 120                 125
   Ile Ala Lys Ser Gln Leu Thr Ser Leu Ser Gly Ile Ala Gln Lys Asn
       130                 135                 140
   Phe Met Asn Ile Leu Glu Lys Val Val Leu Lys Val Leu Glu Asp Gln
   145                 150                 155                 160
   Gln Asn Ile Arg Leu Ile Arg Glu Leu Leu Gln Thr Leu Tyr Thr Ser
                   165                 170                 175
   Leu Cys Thr Leu Val Gln Arg Val Gly Lys Ser Val Leu Val Gly Asn
               180                 185                 190
   Ile Asn Met Trp Val Tyr Arg Met Glu Thr Ile Leu His Trp Gln Gln
           195                 200                 205
   Gln Leu Asn Asn Ile Gln Ile Thr Arg Pro Ala Phe Lys Gly Leu Thr
   210 215 220
   Phe Thr Asp Leu Pro Leu Cys Leu Gln Leu Asn Ile Met Gln Arg Leu
   225 230 235 240
   Ser Asp Gly Arg Asp Leu Val Ser Leu Gly Gln Ala Ala Pro Asp Leu
   245 250 255
   His Val Leu Ser Glu Asp Arg Leu Leu Trp Lys Lys Leu Cys Gln Tyr
   260 265 270
   His Phe Ser Glu Arg Gln Ile Arg Lys Arg Leu Ile Leu Ser Asp Lys
   275 280 285
   Gly Gln Leu Asp Trp Lys Lys Met Tyr Phe Lys Leu Val Arg Cys Tyr
   290 295 300
   Pro Arg Lys Glu Gln Tyr Gly Asp Thr Leu Gln Leu Cys Lys His Cys
   305 310 315 320
   His Ile Leu Ser Trp Lys Gly Thr Asp His Pro Cys Thr Ala Asn Asn
   325 330 335
   Pro Glu Ser Cys Ser Val Ser Leu Ser Pro Gln Asp Phe Ile Asn Leu
   340 345 350
   Phe Lys Phe
   355
<210> 18
   <211> 355
   <212> PRT
   <213> Mus musculus
<400> 18
   Met Pro Phe Leu Gly Gln Asp Trp Arg Ser Pro Gly Gln Ser Trp Val
   1 5 10 15
   Lys Thr Ala Asp Gly Trp Lys Arg Phe Leu Asp Glu Lys Ser Gly Ser
   20 25 30
   Phe Val Ser Asp Leu Ser Ser Tyr Cys Asn Lys Glu Val Tyr Ser Lys
   35 40 45
   Glu Asn Leu Phe Ser Ser Leu Asn Tyr Asp Val Ala Ala Lys Lys Arg
   50 55 60
   Lys Lys Asp Ile Gln Asn Ser Lys Thr Lys Thr Gln Tyr Phe His Gln
   65                  70                  75                  80
   Glu Lys Trp Ile Tyr Val His Lys Gly Ser Thr Lys Glu Arg His Gly
                   85                  90                  95
   Tyr Cys Thr Leu Gly Glu Ala Phe Asn Arg Leu Asp Phe Ser Thr Ala
               100                 105                 110
   Ile Leu Asp Ser Arg Arg Phe Asn Tyr Val Val Arg Leu Leu Glu Leu
           115                 120                 125
   Ile Ala Lys Ser Gln Leu Thr Ser Leu Ser Gly Ile Ala Gln Lys Asn
       130                 135                 140
   Phe Met Asn Ile Leu Glu Lys Val Val Leu Lys Val Leu Glu Asp Gln
   145                 150                 155                 160
   Gln Asn Ile Arg Leu Ile Arg Glu Leu Leu Gln Thr Leu Tyr Thr Ser
                   165                 170                 175
   Leu Cys Thr Leu Val Gln Arg Val Gly Lys Ser Val Leu Val Gly Asn
               180                 185                 190
   Ile Asn Met Trp Val Tyr Arg Met Glu Thr Ile Leu His Trp Gln Gln
           195                 200                 205
   Gln Leu Asn Ser Ile Gln Ile Ser Arg Pro Ala Phe Lys Gly Leu Thr
       210                 215                 220
   Ile Thr Asp Leu Pro Val Cys Leu Gln Leu Asn Ile Met Gln Arg Leu
   225                 230                 235                 240
   Ser Asp Gly Arg Asp Leu Val Ser Leu Gly Gln Ala Ala Pro Asp Leu
                   245                 250                 255
   His Val Leu Ser Glu Asp Arg Leu Leu Trp Lys Arg Leu Cys Gln Tyr
               260                 265                 270
   His Phe Ser Glu Arg Gln Ile Arg Lys Arg Leu Ile Leu Ser Asp Lys
           275                 280                 285
   Gly Gln Leu Asp Trp Lys Lys Met Tyr Phe Lys Leu Val Arg Cys Tyr
       290                 295                 300
   Pro Arg Arg Glu Gln Tyr Gly Val Thr Leu Gln Leu Cys Lys His Cys
   305                 310                 315                 320
   His Ile Leu Ser Trp Lys Gly Thr Asp His Pro Cys Thr Ala Asn Asn
   325 330 335
   Pro Glu Ser Cys Ser Val Ser Leu Ser Pro Gln Asp Phe Ile Asn Leu
   340 345 350
   Phe Lys Phe
   355
<210> 19
   <211> 361
   <212> PRT
   <213> Mus musculus
<400> 19
   Met Ala Ser Pro Ala Val Pro Ala Asn Val Pro Pro Ala Thr Ala Ala
   1               5                   10                  15
   Ala Ala Pro Ala Pro Val Val Thr Ala Ala Pro Ala Ser Ala Pro Thr
               20                  25                  30
   Pro Ser Thr Pro Ala Pro Thr Pro Ala Ala Thr Pro Ala Ala Ser Pro
           35                  40                  45
   Ala Pro Val Ser Ser Asp Pro Ala Val Ala Ala Pro Ala Ala Pro Gly
       50                  55                  60
   Gln Thr Pro Ala Ser Ala Pro Ala Pro Ala Gln Thr Pro Ala Pro Ser
   65                  70                  75                  80
   Gln Pro Gly Pro Ala Leu Pro Gly Pro Phe Pro Gly Gly Arg Val Val
                   85                  90                  95
   Arg Leu His Pro Val Ile Leu Ala Ser Ile Val Asp Ser Tyr Glu Arg
               100                 105                 110
   Arg Asn Glu Gly Ala Ala Arg Val Ile Gly Thr Leu Leu Gly Thr Val
           115                 120                 125
   Asp Lys His Ser Val Glu Val Thr Asn Cys Phe Ser Val Pro His Asn
       130                 135                 140
   Glu Ser Glu Asp Glu Val Ala Val Asp Met Glu Phe Ala Lys Asn Met
   145                 150                 155                 160
   Tyr Glu Leu His Lys Lys Val Ser Pro Asn Glu Leu Ile Leu Gly Trp
                   165                 170                 175
   Tyr Ala Thr Gly His Asp Ile Thr Glu His Ser Val Leu Ile His Glu
               180                 185                 190
   Tyr Tyr Ser Arg Glu Ala Pro Asn Pro Ile His Leu Thr Val Asp Thr
   195 200 205
   Gly Leu Gln His Gly Arg Met Ser Ile Lys Ala Tyr Val Ser Thr Leu
   210 215 220
   Met Gly Val Pro Gly Arg Thr Met Gly Val Met Phe Thr Pro Leu Thr
   225 230 235 240
   Val Lys Tyr Ala Tyr Tyr Asp Thr Glu Arg Ile Gly Val Asp Leu Ile
   245 250 255
   Met Lys Thr Cys Phe Ser Pro Asn Arg Val Ile Gly Leu Ser Ser Asp
   260 265 270
   Leu Gln Gln Val Gly Gly Ala Ser Ala Arg Ile Gln Asp Ala Leu Ser
   275 280 285
   Thr Val Leu Gln Tyr Ala Glu Asp Val Leu Ser Gly Lys Val Ser Ala
   290 295 300
   Asp Asn Thr Val Gly Arg Phe Leu Met Ser Leu Val Asn Gln Val Pro
   305 310 315 320
   Lys Ile Val Pro Asp Asp Phe Glu Thr Met Leu Asn Ser Asn Ile Asn
   325 330 335
   Asp Leu Leu Met Val Thr Tyr Leu Ala Asn Leu Thr Gln Ser Gln Ile
   340 345 350
   Ala Leu Asn Glu Lys Leu Val Asn Leu
   355 360
<210> 20
   <211> 298
   <212> PRT
   <213> Homo sapiens
<400> 20
   Gly Gln Thr Pro Ala Ser Ala Gln Ala Pro Ala Gln Thr Pro Ala Pro
   1 5 10 15
   Ala Leu Pro Gly Pro Ala Leu Pro Gly Pro Phe Pro Gly Gly Arg Val
   20 25 30
   Val Arg Leu His Pro Val Ile Leu Ala Ser Ile Val Asp Ser Tyr Glu
   35 40 45
   Arg Arg Asn Glu Gly Ala Ala Arg Val Ile Gly Thr Leu Leu Gly Thr
   50 55 60
   Val Asp Lys His Ser Val Glu Val Thr Asn Cys Phe Ser Val Pro His
   65 70 75 80
   Asn Glu Ser Glu Asp Glu Val Ala Val Asp Met Glu Phe Ala Lys Asn
   85 90 95
   Met Tyr Glu Leu His Lys Lys Val Ser Pro Asn Glu Leu Ile Leu Gly
   100 105 110
   Trp Tyr Ala Thr Gly His Asp Ile Thr Glu His Ser Val Leu Ile His
   115 120 125
   Glu Tyr Tyr Ser Arg Glu Ala Pro Asn Pro Ile His Leu Thr Val Asp
   130 135 140
   Thr Ser Leu Gln Asn Gly Arg Met Ser Ile Lys Ala Tyr Val Ser Thr
   145 150 155 160
   Leu Met Gly Val Pro Gly Arg Thr Met Gly Val Met Phe Thr Pro Leu
   165 170 175
   Thr Val Lys Tyr Ala Tyr Tyr Asp Thr Glu Arg Ile Gly Val Asp Leu
   180 185 190
   Ile Met Lys Thr Cys Phe Ser Pro Asn Arg Val Ile Gly Leu Ser Ser
   195 200 205
   Asp Leu Gln Gln Val Gly Gly Ala Ser Ala Arg Ile Gln Asp Ala Leu
   210 215 220
   Ser Thr Val Leu Gln Tyr Ala Glu Asp Val Leu Ser Gly Lys Val Ser
   225 230 235 240
   Ala Asp Asn Thr Val Gly Arg Phe Leu Met Ser Leu Val Asn Gln Val
   245 250 255
   Pro Lys Ile Val Pro Asp Asp Phe Glu Thr Met Leu Asn Ser Asn Ile
   260 265 270
   Asn Asp Leu Leu Met Val Thr Tyr Leu Ala Asn Leu Thr Gln Ser Gln
   275 280 285
   Ile Ala Leu Asn Glu Lys Leu Val Asn Leu
   290 295
<210> 21
   <211> 206
   <212> PRT
   <213> Homo sapiens
<400> 21
   Met Asn Phe Gln Gln Arg Leu Gln Ser Leu Trp Thr Leu Ala Arg Pro
   1               5                   10                  15
   Phe Cys Pro Pro Leu Leu Ala Thr Ala Ser Gln Met Gln Met Val Val
               20                  25                  30
   Leu Pro Cys Leu Gly Phe Thr Leu Leu Leu Trp Ser Gln Val Ser Gly
           35                  40                  45
   Ala Gln Gly Gln Glu Phe His Phe Gly Pro Cys Gln Val Lys Gly Val
       50                  55                  60
   Val Pro Gln Lys Leu Trp Glu Ala Phe Trp Ala Val Lys Asp Thr Met
   65                  70                  75                  80
   Gln Ala Gln Asp Asn Ile Thr Ser Ala Arg Leu Leu Gln Gln Glu Val
                   85                  90                  95
   Leu Gln Asn Val Ser Asp Ala Glu Ser Cys Tyr Leu Val His Thr Leu
               100                 105                 110
   Leu Glu Phe Tyr Leu Lys Thr Val Phe Lys Asn Tyr His Asn Arg Thr
           115                 120                 125
   Val Glu Val Arg Thr Leu Lys Ser Phe Ser Thr Leu Ala Asn Asn Phe
       130                 135                 140
   Val Leu Ile Val Ser Gln Leu Gln Pro Ser Gln Glu Asn Glu Met Phe
   145                 150                 155                 160
   Ser Ile Arg Asp Ser Ala His Arg Arg Phe Leu Leu Phe Arg Arg Ala
                   165                 170                 175
   Phe Lys Gln Leu Asp Val Glu Ala Ala Leu Thr Lys Ala Leu Gly Glu
               180                 185                 190
   Val Asp Ile Leu Leu Thr Trp Met Gln Lys Phe Tyr Lys Leu
           195                 200                 205
<210> 22
   <211> 164
   <212> PRT
   <213> Mus musculus
<400> 22
   Trp Asn Gln Val Pro Gly Leu Glu Gly Gln Glu Phe Arg Phe Gly Ser
   1               5                   10                  15
   Cys Gln Val Thr Gly Val Val Leu Pro Glu Leu Trp Glu Ala Phe Trp
               20                  25                  30
   Thr Val Lys Asn Thr Val Gln Thr Gln Asp Asp Ile Thr Ser Ile Arg
           35                  40                  45
   Leu Leu Lys Pro Gln Val Leu Arg Asn Val Ser Gly Ala Glu Ser Cys
       50                  55                  60
   Tyr Leu Ala His Ser Leu Leu Lys Phe Tyr Leu Asn Thr Val Phe Lys
   65                  70                  75                  80
   Asn Tyr His Ser Lys Ile Ala Lys Phe Lys Val Leu Arg Ser Phe Ser
                   85                  90                  95
   Thr Leu Ala Asn Asn Phe Ile Val Ile Met Ser Gln Leu Gln Pro Ser
               100                 105                 110
   Lys Asp Asn Ser Met Leu Pro Ile Ser Glu Ser Ala His Gln Arg Phe
           115                 120                 125
   Leu Leu Phe Arg Arg Ala Phe Lys Gln Leu Asp Thr Glu Val Ala Leu
       130                 135                 140
   Val Lys Ala Phe Gly Glu Val Asp Ile Leu Leu Thr Trp Met Gln Lys
   145                 150                 155                 160
   Phe Tyr His Leu
<210> 23
   <211> 154
   <212> PRT
   <213> Homo sapiens
<400> 23
   Met Asn Phe Gln Gln Arg Leu Gln Ser Leu Trp Thr Leu Ala Ser Arg
   1 5 10 15
   Pro Phe Cys Pro Pro Leu Leu Ala Thr Ala Ser Gln Met Gln Met Val
   20 25 30
   Val Leu Pro Cys Leu Gly Phe Thr Leu Leu Leu Trp Ser Gln Val Ser
   35 40 45
   Gly Ala Gln Gly Gln Glu Phe His Phe Gly Pro Cys Gln Val Lys Gly
   50 55 60
   Val Val Pro Gln Lys Leu Trp Glu Ala Phe Trp Ala Val Lys Asp Thr
   65 70 75 80
   Met Gln Ala Gln Asp Asn Ile Thr Ser Ala Arg Leu Leu Gln Gln Glu
   85 90 95
   Val Leu Gln Asn Val Ser Gln Glu Asn Glu Met Phe Ser Ile Arg Asp
   100 105 110
   Ser Ala His Arg Arg Phe Leu Leu Phe Arg Arg Ala Phe Lys Gln Leu
   115 120 125
   Asp Val Glu Ala Ala Leu Thr Lys Ala Leu Gly Glu Val Asp Ile Leu
   130 135 140
   Leu Thr Trp Met Gln Lys Phe Tyr Lys Leu
   145 150
<210> 24
   <211> 784
   <212> PRT
   <213> Homo sapiens
<400> 24
   Met Gln Arg Leu Met Met Leu Leu Ala Thr Ser Gly Ala Cys Leu Gly
   1               5                   10                  15
   Leu Leu Ala Val Ala Ala Val Ala Ala Ala Gly Ala Asn Pro Ala Gln
               20                  25                  30
   Arg Asp Thr His Ser Leu Leu Pro Thr His Arg Arg Gln Lys Arg Asp
           35                  40                  45
   Trp Ile Trp Asn Gln Met His Ile Asp Glu Glu Lys Asn Thr Ser Leu
       50                  55                  60
   Pro His His Val Gly Lys Ile Lys Ser Ser Val Ser Arg Lys Asn Ala
   65                  70                  75                  80
   Lys Tyr Leu Leu Lys Gly Glu Tyr Val Gly Lys Val Phe Arg Val Asp
                   85                  90                  95
   Ala Glu Thr Gly Asp Val Phe Ala Ile Glu Arg Leu Asp Arg Glu Asn
               100                 105                 110
   Ile Ser Glu Tyr His Leu Thr Ala Val Ile Val Asp Lys Asp Thr Gly
           115                 120                 125
   Glu Asn Leu Glu Thr Pro Ser Ser Phe Thr Ile Lys Val His Asp Val
   130 135 140
   Asn Asp Asn Trp Pro Val Phe Thr His Arg Leu Phe Asn Ala Ser Val
   145 150 155 160
   Pro Glu Ser Ser Ala Val Gly Thr Ser Val Ile Ser Val Thr Ala Val
   165 170 175
   Asp Ala Asp Asp Pro Thr Val Gly Asp His Ala Ser Val Met Tyr Gln
   180 185 190
   Ile Leu Lys Gly Lys Glu Tyr Phe Ala Ile Asp Asn Ser Gly Arg Ile
   195 200 205
   Ile Thr Ile Thr Lys Ser Leu Asp Arg Glu Lys Gln Ala Arg Tyr Glu
   210 215 220
   Ile Val Val Glu Ala Arg Asp Ala Gln Gly Leu Arg Gly Asp Ser Gly
   225 230 235 240
   Thr Ala Thr Val Leu Val Thr Leu Gln Asp Ile Asn Asp Asn Phe Pro
   245 250 255
   Phe Phe Thr Gln Thr Lys Tyr Thr Phe Val Val Pro Glu Asp Thr Arg
   260 265 270
   Val Gly Thr Ser Val Gly Ser Leu Phe Val Glu Asp Pro Asp Glu Pro
   275 280 285
   Gln Asn Arg Met Thr Lys Tyr Ser Ile Leu Arg Gly Asp Tyr Gln Asp
   290 295 300
   Ala Phe Thr Ile Glu Thr Asn Pro Ala His Asn Glu Gly Ile Ile Lys
   305 310 315 320
   Pro Met Lys Pro Leu Asp Tyr Glu Tyr Ile Gln Gln Tyr Ser Phe Ile
   325 330 335
   Val Glu Ala Thr Asp Pro Thr Ile Asp Leu Arg Tyr Met Ser Pro Pro
   340 345 350
   Ala Gly Asn Arg Ala Gln Val Ile Ile Asn Ile Thr Asp Val Asp Glu
   355 360 365
   Pro Pro Ile Phe Gln Gln Pro Phe Tyr His Phe Gln Leu Lys Glu Asn
   370 375 380
   Gln Lys Lys Pro Leu Ile Gly Thr Val Leu Ala Met Asp Pro Asp Ala
   385 390 395 400
   Ala Arg His Ser Ile Gly Tyr Ser Ile Arg Arg Thr Ser Asp Lys Gly
   405 410 415
   Gln Phe Phe Arg Val Thr Lys Lys Gly Asp Ile Tyr Asn Glu Lys Glu
   420 425 430
   Leu Asp Arg Glu Val Tyr Pro Trp Tyr Asn Leu Thr Val Glu Ala Lys
   435 440 445
   Glu Leu Asp Ser Thr Gly Thr Pro Thr Gly Lys Glu Ser Ile Val Gln
   450 455 460
   Val His Ile Glu Val Leu Asp Glu Asn Asp Asn Ala Pro Glu Phe Ala
   465 470 475 480
   Lys Pro Tyr Gln Pro Lys Val Cys Glu Asn Ala Val His Gly Gln Leu
   485 490 495
   Val Leu Gln Ile Ser Ala Ile Asp Lys Asp Ile Thr Pro Arg Asn Val
   500 505 510
   Lys Phe Lys Phe Thr Leu Asn Thr Glu Asn Asn Phe Thr Leu Thr Asp
   515 520 525
   Asn His Asp Asn Thr Ala Asn Ile Thr Val Lys Tyr Gly Gln Phe Asp
   530 535 540
   Arg Glu His Thr Lys Val His Phe Leu Pro Val Val Ile Ser Asp Asn
   545 550 555 560
   Gly Met Pro Ser Arg Thr Gly Thr Ser Thr Leu Thr Val Ala Val Cys
   565 570 575
   Lys Cys Asn Glu Gln Gly Glu Phe Thr Phe Cys Glu Asp Met Ala Ala
   580 585 590
   Gln Val Gly Val Ser Ile Gln Ala Val Val Ala Ile Leu Leu Cys Ile
   595 600 605
   Leu Thr Ile Thr Val Ile Thr Leu Leu Ile Phe Leu Arg Arg Arg Leu
   610 615 620
   Arg Lys Gln Ala Arg Ala His Gly Lys Ser Val Pro Glu Ile His Glu
   625 630 635 640
   Gln Leu Val Thr Tyr Asp Glu Glu Gly Gly Gly Glu Met Asp Thr Thr
   645 650 655
   Ser Tyr Asp Val Ser Val Leu Asn Ser Val Arg Arg Gly Gly Ala Lys
   660 665 670
   Pro Pro Arg Pro Ala Leu Asp Ala Arg Pro Ser Leu Tyr Ala Gln Val
   675 680 685
   Gln Lys Pro Pro Arg His Ala Pro Gly Ala His Gly Gly Pro Gly Glu
   690 695 700
   Met Ala Ala Met Ile Glu Val Lys Lys Asp Glu Ala Asp His Asp Gly
   705 710 715 720
   Asp Gly Pro Pro Tyr Asp Thr Leu His Ile Tyr Gly Tyr Glu Gly Ser
   725 730 735
   Glu Ser Ile Ala Glu Ser Leu Ser Ser Leu Gly Thr Asp Ser Ser Asp
   740 745 750
   Ser Asp Val Asp Tyr Asp Phe Leu Asn Asp Trp Gly Pro Arg Phe Lys
   755 760 765
   Met Leu Ala Glu Leu Tyr Gly Ser Asp Pro Arg Glu Glu Leu Leu Tyr
   770 775 780
<210> 25
   <211> 9
   <212> PRT
   <213> Homo sapiens
<220>
   <221> SITE
   <222> (5)..(5)
   <223> la tyrosine Y685 est phosphorylée par la src kinase
<400> 25
   Arg Pro Ser Leu Tyr Ala Gln Val Gln
   1 5
<210> 26
   <211> 393
   <212> PRT
   <213> Homo sapiens
<400> 26
<210> 27
   <211> 320
   <212> PRT
   <213> Homo sapiens
<400> 27
   Met Pro Lys Pro Ile Asn Val Arg Val Thr Thr Met Asp Ala Glu Leu
   1               5                   10                  15
   Glu Phe Ala Ile Gln Pro Asn Thr Thr Gly Lys Gln Leu Phe Asp Gln
               20                  25                  30
   Val Val Lys Thr Ile Gly Leu Arg Glu Val Trp Tyr Phe Gly Leu His
           35                  40                  45
   Tyr Val Asp Asn Lys Gly Phe Pro Thr Trp Leu Lys Leu Asp Lys Lys
       50                  55                  60
   Val Ser Ala Gln Glu Val Arg Lys Glu Asn Pro Leu Gln Phe Lys Phe
   65                  70                  75                  80
   Arg Ala Lys Phe Tyr Pro Glu Asp Val Ala Glu Glu Leu Ile Gln Asp
                   85                  90                  95
   Ile Thr Gln Lys Leu Phe Phe Leu Gln Val Lys Glu Gly Ile Leu Ser
               100                 105                 110
   Asp Glu Ile Tyr Cys Pro Pro Glu Thr Ala Val Leu Leu Gly Ser Tyr
           115                 120                 125
   Ala Val Gln Ala Lys Phe Gly Asp Tyr Asn Lys Glu Val His Lys Ser
       130                 135                 140
   Gly Tyr Leu Ser Ser Glu Arg Leu Ile Pro Gln Arg Val Met Asp Gln
   145                 150                 155                 160
   His Lys Leu Thr Arg Asp Gln Trp Glu Asp Arg Ile Gln Val Trp His
                   165                 170                 175
   Ala Glu His Arg Gly Met Leu Lys Asp Asn Ala Met Leu Glu Tyr Leu
               180                 185                 190
   Lys Ile Ala Gln Asp Leu Glu Met Tyr Gly Ile Asn Tyr Phe Glu Ile
           195                 200                 205
   Lys Asn Lys Lys Gly Thr Asp Leu Trp Leu Gly Val Asp Ala Leu Gly
       210                 215                 220
   Leu Asn Ile Tyr Glu Lys Asp Asp Lys Leu Thr Pro Lys Ile Gly Phe
   225                 230                 235                 240
   Pro Trp Ser Glu Ile Arg Asn Ile Ser Phe Asn Asp Lys Lys Phe Val
                   245                 250                 255
   Ile Lys Pro Ile Asp Lys Lys Ala Pro Asp Phe Val Phe Tyr Ala Pro
               260                 265                 270
   Arg Leu Arg Ile Asn Lys Arg Ile Leu Gln Leu Cys Met Gly Asn His
           275                 280                 285
   Glu Leu Tyr Met Arg Arg Arg Lys Pro Asp Thr Ile Glu Val Gln Gln
       290                 295                 300
   Met Lys Ala Gln Ala Arg Glu Glu Lys His Gln Lys Gln Leu Glu Arg
   305                 310                 315                 320
<210> 28
   <211> 200
   <212> PRT
   <213> Artificial sequence
<220>
   <223> dérivé de protéine humaine
<400> 28
   Met His His His His His His His His Lys Arg Tyr Lys Asn Arg Val
   1 5 10 15
   Ala Ser Arg Lys Cys Arg Ala Lys Phe Lys Gln Leu Leu Gln His Tyr
   20 25 30
   Arg Glu Val Ala Ala Ala Lys Ser Ser Glu Asn Asp Arg Leu Arg Leu
   35 40 45
   Leu Leu Lys Gln Met Cys Lys Leu Ala Lys Phe Val Ala Ala Trp Thr
   50 55 60
   Leu Lys Ala Ala Ala Gly Ser Asp Glu Val Ser Gly Leu Glu Gln Leu
   65 70 75 80
   Glu Ser Ile Ile Asn Phe Glu Lys Leu Thr Glu Trp Thr Ser Ser Asn
   85 90 95
   Val Met Glu Glu Arg Lys Ile Lys Val Tyr Leu Pro Arg Met Lys Met
   100 105 110
   Glu Glu Lys Tyr Asn Leu Thr Ser Val Leu Met Ala Met Gly Ile Thr
   115 120 125
   Asp Val Phe Ser Ser Ser Ala Asn Leu Ser Gly Ile Ser Ser Ala Glu
   130 135 140
   Ser Leu Lys Ile Ser Gln Ala Val His Ala Ala His Ala Glu Ile Asn
   145 150 155 160
   Glu Ala Gly Arg Glu Val Val Gly Ser Ala Glu Ala Gly Val Asp Ala
   165 170 175
   Ala Ser Val Ser Glu Glu Phe Arg Ala Asp His Pro Phe Leu Phe Cys
   180 185 190
   Ile Lys His Ile Ala Thr Asn Ala
   195 200
<210> 29
   <211> 349
   <212> PRT
   <213> Artificial sequence
<220>
   <223> dérivé de protéine humaine
<400> 29
   Glu Cys Asp Ser Glu Leu Glu Ile Lys Arg Tyr Lys Asn Arg Val Ala
   1               5                   10                  15
   Ser Arg Lys Cys Arg Ala Lys Phe Lys Gln Leu Leu Gln His Tyr Arg
               20                  25                  30
   Glu Val Ala Ala Ala Lys Ser Ser Glu Asn Asp Arg Leu Arg Leu Leu
               35                  40                  45
   Leu Lys Gln Met Arg His Met Gln Ser Ala Thr Arg Ala Thr Leu Val
       50                  55                  60
   Cys Gly Ser Gly Val Lys Gln Ile Ile Ala Lys Gly His Pro Asn Thr
   65                  70                  75                  80
   Arg Val Phe Gly Ala Arg Lys Ala Arg Ile Pro Glu Arg Glu Val Leu
                   85                  90                  95
   Ala Ala Lys Pro Lys Ile Thr Arg Ile Thr His Leu Asn Leu Gln Pro
               100                 105                 110
   Gln Glu Arg Gln Ala Phe Tyr Arg Leu Leu Glu Asn Glu Leu Ile Gln
           115                 120                 125
   Glu Phe Leu Ser Met Asp Ser Cys Leu Lys Ile Ser Asp Lys Tyr Leu
       130                 135                 140
   Ile Ala Met Val Leu Ala Tyr Phe Lys Arg Ala Gly Leu Tyr Thr Gly
   145                 150                 155                 160
   Glu Tyr Thr Thr Met Asn Phe Phe Val Ala Leu Tyr Leu Ala Asn Asp
                   165                 170                 175
   Met Glu Glu Asp Glu Glu Asp Tyr Lys Tyr Glu Ile Phe Pro Trp Ala
               180                 185                 190
   Leu Gly Asp Ser Trp Arg Glu Phe Phe Pro Gln Phe Leu Arg Leu Arg
           195                 200                 205
   Asp Asn Phe Trp Ala Lys Met Asn Tyr Arg Ala Val Val Ser Arg Arg
       210                 215                 220
   Cys Cys Asp Glu Val Met Ala Lys Asp Pro Thr His Trp Ala Trp Leu
   225                 230                 235                 240
   Arg Asp Arg Pro Ile His His Ser Gly Ala Leu Arg Gly Tyr Leu Arg
                   245                 250                 255
   Asn Glu Asp Asp Phe Phe Pro Arg Gly Pro Gly Leu Thr Pro Ala Ser
               260                 265                 270
   Cys Ala Leu Cys His Lys Ala Ser Val Cys Asp Ser Gly Gly Val Ala
           275                 280                 285
   His Asp Asn Ser Ser Pro Glu Gln Glu Ile Phe His Tyr Thr Asn Arg
       290                 295                 300
   Glu Trp Ser Gln Glu Leu Leu Ile Leu Pro Pro Glu Leu Leu Leu Asp
   305 310 315 320
   Pro Glu Ser Thr Tyr Asp Ile His Ile Phe Gln Glu Pro Leu Val Gly
   325 330 335
   Leu Glu Pro Asp Gly Ala Ala Leu Glu Trp His His Leu
   340 345
<210> 30
   <211> 182
   <212> PRT
   <213> Artificial sequence
<220>
   <223> dérivé de protéine humaine
<400> 30
   Glu Cys Asp Ser Glu Leu Glu Ile Lys Arg Tyr Lys Asn Arg Val Ala
   1 5 10 15
   Ser Arg Lys Cys Arg Ala Lys Phe Lys Gln Leu Leu Gln His Tyr Arg
   20 25 30
   Glu Val Ala Ala Ala Lys Ser Ser Glu Asn Asp Arg Leu Arg Leu Leu
   35 40 45
   Leu Lys Gln Gln Glu Val Gln Ala Phe Leu Ser Leu Leu Glu Asp Ser
   50 55 60
   Phe Val Gln Glu Phe Leu Ser Lys Asp Pro Cys Phe Gln Ile Ser Asp
   65 70 75 80
   Lys Tyr Leu Leu Ala Met Val Leu Val Tyr Phe Gln Arg Ala His Leu
   85 90 95
   Lys Leu Ser Glu Tyr Thr His Ser Ser Leu Phe Leu Ala Leu Tyr Leu
   100 105 110
   Ala Asn Asp Met Glu Glu Asp Leu Glu Gly Pro Lys Cys Glu Ile Phe
   115 120 125
   Pro Trp Ala Leu Gly Lys Asp Trp Cys Leu Arg Val Gly Lys Phe Leu
   130 135 140
   His Gln Arg Asp Lys Leu Trp Ala Arg Met Gly Phe Arg Ala Val Val
   145 150 155 160
   Ser Arg Gln Cys Cys Glu Glu Val Met Ala Lys Glu Pro Phe His Trp
   165 170 175
   Ala Trp Thr Arg Asp Arg
   180
<210> 31
   <211> 462
   <212> PRT
   <213> Artificial sequence
<220>
   <223> dérivé de protéine humaine
<400> 31
   Glu Cys Asp Ser Glu Leu Glu Ile Lys Arg Tyr Lys Asn Arg Val Ala
   1               5                   10                  15
   Ser Arg Lys Cys Arg Ala Lys Phe Lys Gln Leu Leu Gln His Tyr Arg
               20                  25                  30
   Glu Val Ala Ala Ala Lys Ser Ser Glu Asn Asp Arg Leu Arg Leu Leu
           35                  40                  45
   Leu Lys Gln Met Pro Arg Glu Arg Lys Glu Arg Asp Ser Lys Asp His
       50                  55                  60
   Ser Asn Met Lys Glu Glu Gly Gly Ser Asp Leu Ser Val Arg Ser Arg
   65                  70                  75                  80
   Lys Arg Lys Ala Asn Val Ala Val Phe Leu Gln Asp Pro Asp Glu Glu
                   85                  90                  95
   Ile Ala Lys Ile Asp Lys Thr Val Lys Ser Gln Asp Ser Ser Gln Pro
               100                 105                 110
   Trp Asp Asp Asp Ser Ala Cys Val Asp Pro Cys Ser Phe Ile Pro Thr
           115                 120                 125
   Pro Asn Lys Glu Glu Asp Asn Glu Leu Glu Tyr Pro Lys Thr Ala Phe
       130                 135                 140
   Gln Pro Arg Lys Ile Arg Pro Pro Arg Ala Ser Pro Leu Pro Val Leu
   145                 150                 155                 160
   Asn Trp Gly Asn Arg Glu Glu Val Trp Arg Ile Met Leu Asn Lys Glu
                   165                 170                 175
   Lys Thr Tyr Leu Arg Asp Glu His Phe Leu Gln Arg His Pro Leu Leu
               180                 185                 190
   Gln Ala Arg Met Arg Ala Val Leu Leu Asp Trp Leu Met Glu Val Cys
   195 200 205
   Glu Val Tyr Lys Leu His Arg Glu Thr Phe Tyr Leu Ala Gln Asp Phe
   210 215 220
   Phe Asp Arg Tyr Met Ala Ser Gln Gln Asn Ile Ile Lys Thr Leu Leu
   225 230 235 240
   Gln Leu Ile Gly Ile Ser Ala Leu Phe Ile Ala Ser Lys Leu Glu Glu
   245 250 255
   Ile Tyr Pro Pro Lys Leu His Gln Phe Ala Tyr Val Thr Asp Gly Ala
   260 265 270
   Cys Ser Gly Asp Glu Ile Leu Thr Met Glu Leu Met Met Met Lys Ala
   275 280 285
   Leu Lys Trp Arg Leu Ser Pro Leu Thr Ile Val Ser Trp Leu Asn Val
   290 295 300
   Tyr Val Gln Val Ala Tyr Val Asn Asp Thr Gly Glu Val Leu Met Pro
   305 310 315 320
   Gln Tyr Pro Gln Gln Val Phe Val Gln Ile Ala Glu Leu Leu Asp Leu
   325 330 335
   Cys Val Leu Asp Val Gly Cys Leu Glu Phe Pro Tyr Gly Val Leu Ala
   340 345 350
   Ala Ser Ala Leu Tyr His Phe Ser Ser Leu Glu Leu Met Gln Lys Val
   355 360 365
   Ser Gly Tyr Gln Trp Cys Asp Ile Glu Lys Cys Val Lys Trp Met Val
   370 375 380
   Pro Phe Ala Met Val Ile Arg Glu Met Gly Ser Ser Lys Leu Lys His
   385 390 395 400
   Phe Arg Gly Val Pro Met Glu Asp Ser His Asn Ile Gln Thr His Thr
   405 410 415
   Asn Ser Leu Asp Leu Leu Asp Lys Ala Gln Ala Lys Lys Ala Ile Leu
   420 425 430
   Ser Glu Gln Asn Arg Ile Ser Pro Pro Pro Ser Gly Val Leu Thr Pro
   435 440 445
   Pro His Ser Ser Lys Lys Gln Ser Ser Glu Gln Glu Thr Glu
   450 455 460
<210> 33
   <211> 377
   <212> PRT
   <213> Artificial sequence
<220>
   <223> dérivé de protéine humaine
<400> 33
   Glu Cys Asp Ser Glu Leu Glu Ile Lys Arg Tyr Lys Asn Arg Val Ala
   1               5                   10                  15
   Ser Arg Lys Cys Arg Ala Lys Phe Lys Gln Leu Leu Gln His Tyr Arg
               20                  25                  30
   Glu Val Ala Ala Ala Lys Ser Ser Glu Asn Asp Arg Leu Arg Leu Leu
           35                  40                  45
   Leu Lys Gln Met Asp Leu Thr Ala Ile Tyr Glu Ser Leu Leu Ser Leu
       50                  55                  60
   Ser Pro Asp Val Pro Val Pro Ser Asp His Gly Gly Thr Glu Ser Ser
   65                  70                  75                  80
   Pro Gly Trp Gly Ser Ser Gly Pro Trp Ser Leu Ser Pro Ser Asp Ser
                   85                  90                  95
   Ser Pro Ser Gly Val Thr Ser Arg Leu Pro Gly Arg Ser Thr Ser Leu
               100                 105                 110
   Val Glu Gly Arg Ser Cys Gly Trp Val Pro Pro Pro Pro Gly Phe Ala
           115                 120                 125
   Pro Leu Ala Pro Arg Leu Gly Pro Glu Leu Ser Pro Ser Pro Thr Ser
       130                 135                 140
   Pro Thr Ala Thr Ser Thr Thr Pro Ser Arg Tyr Lys Thr Glu Leu Cys
   145                 150                 155                 160
   Arg Thr Phe Ser Glu Ser Gly Arg Cys Arg Tyr Gly Ala Lys Cys Gln
                   165                 170                 175
   Phe Ala His Gly Leu Gly Glu Leu Arg Gln Ala Asn Arg His Pro Lys
               180                 185                 190
   Tyr Lys Thr Glu Leu Cys His Lys Phe Tyr Leu Gln Gly Arg Cys Pro
           195                 200                 205
   Tyr Gly Ser Arg Cys His Phe Ile His Asn Pro Ser Glu Asp Leu Ala
       210                 215                 220
   Ala Pro Gly His Pro Pro Val Leu Arg Gln Ser Ile Ser Phe Ser Gly
   225                 230                 235                 240
   Leu Pro Ser Gly Arg Arg Thr Ser Pro Pro Pro Pro Gly Leu Ala Gly
                   245                 250                 255
   Pro Ser Leu Ser Ser Ser Ser Phe Ser Pro Ser Ser Ser Pro Pro Pro
               260                 265                 270
   Pro Gly Asp Leu Pro Leu Ser Pro Ser Ala Phe Ser Ala Ala Pro Gly
           275                 280                 285
   Thr Pro Leu Ala Arg Arg Asp Pro Thr Pro Val Cys Cys Pro Ser Cys
       290                 295                 300
   Arg Arg Ala Thr Pro Ile Ser Val Trp Gly Pro Leu Gly Gly Leu Val
   305                 310                 315                 320
   Arg Thr Pro Ser Val Gln Ser Leu Gly Ser Asp Pro Asp Glu Tyr Ala
                   325                 330                 335
   Ser Ser Gly Ser Ser Leu Gly Gly Ser Asp Ser Pro Val Phe Glu Ala
               340                 345                 350
   Gly Val Phe Ala Pro Pro Gln Pro Val Ala Ala Pro Arg Arg Leu Pro
   355 360 365
   Ile Phe Asn Arg Ile Ser Val Ser Glu
   370 375
<210> 34
   <211> 406
   <212> PRT
   <213> Artificial sequence
<220>
   <223> dérivé de protéine humaine
<400> 34
   Glu Cys Asp Ser Glu Leu Glu Ile Lys Arg Tyr Lys Asn Arg Val Ala
   1               5                   10                  15
   Ser Arg Lys Cys Arg Ala Lys Phe Lys Gln Leu Leu Gln His Tyr Arg
               20                  25                  30
   Glu Val Ala Ala Ala Lys Ser Ser Glu Asn Asp Arg Leu Arg Leu Leu
           35                  40                  45
   Leu Lys Gln Met Pro Phe Leu Gly Gln Asp Trp Arg Ser Pro Gly Gln
       50                  55                  60
   Asn Trp Val Lys Thr Ala Asp Gly Trp Lys Arg Phe Leu Asp Glu Lys
   65                  70                  75                  80
   Ser Gly Ser Phe Val Ser Asp Leu Ser Ser Tyr Cys Asn Lys Glu Val
                   85                  90                  95
   Tyr Asn Lys Glu Asn Leu Phe Asn Ser Leu Asn Tyr Asp Val Ala Ala
               100                 105                 110
   Lys Lys Arg Lys Lys Asp Met Leu Asn Ser Lys Thr Lys Thr Gln Tyr
           115                 120                 125
   Phe His Gln Glu Lys Trp Ile Tyr Val His Lys Gly Ser Thr Lys Glu
       130                 135                 140
   Arg His Gly Tyr Cys Thr Leu Gly Glu Ala Phe Asn Arg Leu Asp Phe
   145                 150                 155                 160
   Ser Thr Ala Ile Leu Asp Ser Arg Arg Phe Asn Tyr Val Val Arg Leu
                   165                 170                 175
   Leu Glu Leu Ile Ala Lys Ser Gln Leu Thr Ser Leu Ser Gly Ile Ala
               180                 185                 190
   Gln Lys Asn Phe Met Asn Ile Leu Glu Lys Val Val Leu Lys Val Leu
   195 200 205
   Glu Asp Gln Gln Asn Ile Arg Leu Ile Arg Glu Leu Leu Gln Thr Leu
   210 215 220
   Tyr Thr Ser Leu Cys Thr Leu Val Gln Arg Val Gly Lys Ser Val Leu
   225 230 235 240
   Val Gly Asn Ile Asn Met Trp Val Tyr Arg Met Glu Thr Ile Leu His
   245 250 255
   Trp Gln Gln Gln Leu Asn Asn Ile Gln Ile Thr Arg Pro Ala Phe Lys
   260 265 270
   Gly Leu Thr Phe Thr Asp Leu Pro Leu Cys Leu Gln Leu Asn Ile Met
   275 280 285
   Gln Arg Leu Ser Asp Gly Arg Asp Leu Val Ser Leu Gly Gln Ala Ala
   290 295 300
   Pro Asp Leu His Val Leu Ser Glu Asp Arg Leu Leu Trp Lys Lys Leu
   305 310 315 320
   Cys Gln Tyr His Phe Ser Glu Arg Gln Ile Arg Lys Arg Leu Ile Leu
   325 330 335
   Ser Asp Lys Gly Gln Leu Asp Trp Lys Lys Met Tyr Phe Lys Leu Val
   340 345 350
   Arg Cys Tyr Pro Arg Lys Glu Gln Tyr Gly Asp Thr Leu Gln Leu Cys
   355 360 365
   Lys His Cys His Ile Leu Ser Trp Lys Gly Thr Asp His Pro Cys Thr
   370 375 380
   Ala Asn Asn Pro Glu Ser Cys Ser Val Ser Leu Ser Pro Gln Asp Phe
   385 390 395 400
   Ile Asn Leu Phe Lys Phe
   405
<210> 35
   <211> 349
   <212> PRT
   <213> Artificial sequence
<220>
   <223> dérivé de protéine humaine
<400> 35
   Glu Cys Asp Ser Glu Leu Glu Ile Lys Arg Tyr Lys Asn Arg Val Ala
   1               5                   10                  15
   Ser Arg Lys Cys Arg Ala Lys Phe Lys Gln Leu Leu Gln His Tyr Arg
               20                  25                  30
   Glu Val Ala Ala Ala Lys Ser Ser Glu Asn Asp Arg Leu Arg Leu Leu
           35                  40                  45
   Leu Lys Gln Gly Gln Thr Pro Ala Ser Ala Gln Ala Pro Ala Gln Thr
       50                  55                  60
   Pro Ala Pro Ala Leu Pro Gly Pro Ala Leu Pro Gly Pro Phe Pro Gly
   65                  70                  75                  80
   Gly Arg Val Val Arg Leu His Pro Val Ile Leu Ala Ser Ile Val Asp
                   85                  90                  95
   Ser Tyr Glu Arg Arg Asn Glu Gly Ala Ala Arg Val Ile Gly Thr Leu
               100                 105                 110
   Leu Gly Thr Val Asp Lys His Ser Val Glu Val Thr Asn Cys Phe Ser
           115                 120                 125
   Val Pro His Asn Glu Ser Glu Asp Glu Val Ala Val Asp Met Glu Phe
       130                 135                 140
   Ala Lys Asn Met Tyr Glu Leu His Lys Lys Val Ser Pro Asn Glu Leu
   145                 150                 155                 160
   Ile Leu Gly Trp Tyr Ala Thr Gly His Asp Ile Thr Glu His Ser Val
                   165                 170                 175
   Leu Ile His Glu Tyr Tyr Ser Arg Glu Ala Pro Asn Pro Ile His Leu
               180                 185                 190
   Thr Val Asp Thr Ser Leu Gln Asn Gly Arg Met Ser Ile Lys Ala Tyr
           195                 200                 205
   Val Ser Thr Leu Met Gly Val Pro Gly Arg Thr Met Gly Val Met Phe
       210                 215                 220
   Thr Pro Leu Thr Val Lys Tyr Ala Tyr Tyr Asp Thr Glu Arg Ile Gly
   225                 230                 235                 240
   Val Asp Leu Ile Met Lys Thr Cys Phe Ser Pro Asn Arg Val Ile Gly
                   245                 250                 255
   Leu Ser Ser Asp Leu Gln Gln Val Gly Gly Ala Ser Ala Arg Ile Gln
               260                 265                 270
   Asp Ala Leu Ser Thr Val Leu Gln Tyr Ala Glu Asp Val Leu Ser Gly
           275                 280                 285
   Lys Val Ser Ala Asp Asn Thr Val Gly Arg Phe Leu Met Ser Leu Val
       290                 295                 300
   Asn Gln Val Pro Lys Ile Val Pro Asp Asp Phe Glu Thr Met Leu Asn
   305                 310                 315                 320
   Ser Asn Ile Asn Asp Leu Leu Met Val Thr Tyr Leu Ala Asn Leu Thr
                   325                 330                 335
   Gln Ser Gln Ile Ala Leu Asn Glu Lys Leu Val Asn Leu
               340                 345
<210> 36
   <211> 257
   <212> PRT
   <213> Artificial sequence
<220>
   <223> dérivé de protéine humaine
<400> 36
   Glu Cys Asp Ser Glu Leu Glu Ile Lys Arg Tyr Lys Asn Arg Val Ala
   1               5                   10                  15
   Ser Arg Lys Cys Arg Ala Lys Phe Lys Gln Leu Leu Gln His Tyr Arg
               20                  25                  30
   Glu Val Ala Ala Ala Lys Ser Ser Glu Asn Asp Arg Leu Arg Leu Leu
           35                  40                  45
   Leu Lys Gln Met Asn Phe Gln Gln Arg Leu Gln Ser Leu Trp Thr Leu
       50                  55                  60
   Ala Arg Pro Phe Cys Pro Pro Leu Leu Ala Thr Ala Ser Gln Met Gln
   65                  70                  75                  80
   Met Val Val Leu Pro Cys Leu Gly Phe Thr Leu Leu Leu Trp Ser Gln
                   85                  90                  95
   Val Ser Gly Ala Gln Gly Gln Glu Phe His Phe Gly Pro Cys Gln Val
               100                 105                 110
   Lys Gly Val Val Pro Gln Lys Leu Trp Glu Ala Phe Trp Ala Val Lys
   115 120 125
   Asp Thr Met Gln Ala Gln Asp Asn Ile Thr Ser Ala Arg Leu Leu Gln
   130 135 140
   Gln Glu Val Leu Gln Asn Val Ser Asp Ala Glu Ser Cys Tyr Leu Val
   145 150 155 160
   His Thr Leu Leu Glu Phe Tyr Leu Lys Thr Val Phe Lys Asn Tyr His
   165 170 175
   Asn Arg Thr Val Glu Val Arg Thr Leu Lys Ser Phe Ser Thr Leu Ala
   180 185 190
   Asn Asn Phe Val Leu Ile Val Ser Gln Leu Gln Pro Ser Gln Glu Asn
   195 200 205
   Glu Met Phe Ser Ile Arg Asp Ser Ala His Arg Arg Phe Leu Leu Phe
   210 215 220
   Arg Arg Ala Phe Lys Gln Leu Asp Val Glu Ala Ala Leu Thr Lys Ala
   225 230 235 240
   Leu Gly Glu Val Asp Ile Leu Leu Thr Trp Met Gln Lys Phe Tyr Lys
   245 250 255
   Leu
<210> 37
   <211> 835
   <212> PRT
   <213> Artificial sequence
<220>
   <223> dérivé de protéine humaine
<400> 37
   Glu Cys Asp Ser Glu Leu Glu Ile Lys Arg Tyr Lys Asn Arg Val Ala
   1               5                   10                  15
   Ser Arg Lys Cys Arg Ala Lys Phe Lys Gln Leu Leu Gln His Tyr Arg
               20                  25                  30
   Glu Val Ala Ala Ala Lys Ser Ser Glu Asn Asp Arg Leu Arg Leu Leu
           35                  40                  45
   Leu Lys Gln Met Gln Arg Leu Met Met Leu Leu Ala Thr Ser Gly Ala
       50                  55                  60
   Cys Leu Gly Leu Leu Ala Val Ala Ala Val Ala Ala Ala Gly Ala Asn
   65 70 75 80
   Pro Ala Gln Arg Asp Thr His Ser Leu Leu Pro Thr His Arg Arg Gln
   85 90 95
   Lys Arg Asp Trp Ile Trp Asn Gln Met His Ile Asp Glu Glu Lys Asn
   100 105 110
   Thr Ser Leu Pro His His Val Gly Lys Ile Lys Ser Ser Val Ser Arg
   115 120 125
   Lys Asn Ala Lys Tyr Leu Leu Lys Gly Glu Tyr Val Gly Lys Val Phe
   130 135 140
   Arg Val Asp Ala Glu Thr Gly Asp Val Phe Ala Ile Glu Arg Leu Asp
   145 150 155 160
   Arg Glu Asn Ile Ser Glu Tyr His Leu Thr Ala Val Ile Val Asp Lys
   165 170 175
   Asp Thr Gly Glu Asn Leu Glu Thr Pro Ser Ser Phe Thr Ile Lys Val
   180 185 190
   His Asp Val Asn Asp Asn Trp Pro Val Phe Thr His Arg Leu Phe Asn
   195 200 205
   Ala Ser Val Pro Glu Ser Ser Ala Val Gly Thr Ser Val Ile Ser Val
   210 215 220
   Thr Ala Val Asp Ala Asp Asp Pro Thr Val Gly Asp His Ala Ser Val
   225 230 235 240
   Met Tyr Gln Ile Leu Lys Gly Lys Glu Tyr Phe Ala Ile Asp Asn Ser
   245 250 255
   Gly Arg Ile Ile Thr Ile Thr Lys Ser Leu Asp Arg Glu Lys Gln Ala
   260 265 270
   Arg Tyr Glu Ile Val Val Glu Ala Arg Asp Ala Gln Gly Leu Arg Gly
   275 280 285
   Asp Ser Gly Thr Ala Thr Val Leu Val Thr Leu Gln Asp Ile Asn Asp
   290 295 300
   Asn Phe Pro Phe Phe Thr Gln Thr Lys Tyr Thr Phe Val Val Pro Glu
   305 310 315 320
   Asp Thr Arg Val Gly Thr Ser Val Gly Ser Leu Phe Val Glu Asp Pro
   325 330 335
   Asp Glu Pro Gln Asn Arg Met Thr Lys Tyr Ser Ile Leu Arg Gly Asp
   340 345 350
   Tyr Gln Asp Ala Phe Thr Ile Glu Thr Asn Pro Ala His Asn Glu Gly
   355 360 365
   Ile Ile Lys Pro Met Lys Pro Leu Asp Tyr Glu Tyr Ile Gln Gln Tyr
   370 375 380
   Ser Phe Ile Val Glu Ala Thr Asp Pro Thr Ile Asp Leu Arg Tyr Met
   385 390 395 400
   Ser Pro Pro Ala Gly Asn Arg Ala Gln Val Ile Ile Asn Ile Thr Asp
   405 410 415
   Val Asp Glu Pro Pro Ile Phe Gln Gln Pro Phe Tyr His Phe Gln Leu
   420 425 430
   Lys Glu Asn Gln Lys Lys Pro Leu Ile Gly Thr Val Leu Ala Met Asp
   435 440 445
   Pro Asp Ala Ala Arg His Ser Ile Gly Tyr Ser Ile Arg Arg Thr Ser
   450 455 460
   Asp Lys Gly Gln Phe Phe Arg Val Thr Lys Lys Gly Asp Ile Tyr Asn
   465 470 475 480
   Glu Lys Glu Leu Asp Arg Glu Val Tyr Pro Trp Tyr Asn Leu Thr Val
   485 490 495
   Glu Ala Lys Glu Leu Asp Ser Thr Gly Thr Pro Thr Gly Lys Glu Ser
   500 505 510
   Ile Val Gln Val His Ile Glu Val Leu Asp Glu Asn Asp Asn Ala Pro
   515 520 525
   Glu Phe Ala Lys Pro Tyr Gln Pro Lys Val Cys Glu Asn Ala Val His
   530 535 540
   Gly Gln Leu Val Leu Gln Ile Ser Ala Ile Asp Lys Asp Ile Thr Pro
   545 550 555 560
   Arg Asn Val Lys Phe Lys Phe Thr Leu Asn Thr Glu Asn Asn Phe Thr
   565 570 575
   Leu Thr Asp Asn His Asp Asn Thr Ala Asn Ile Thr Val Lys Tyr Gly
   580 585 590
   Gln Phe Asp Arg Glu His Thr Lys Val His Phe Leu Pro Val Val Ile
   595 600 605
   Ser Asp Asn Gly Met Pro Ser Arg Thr Gly Thr Ser Thr Leu Thr Val
   610 615 620
   Ala Val Cys Lys Cys Asn Glu Gln Gly Glu Phe Thr Phe Cys Glu Asp
   625 630 635 640
   Met Ala Ala Gln Val Gly Val Ser Ile Gln Ala Val Val Ala Ile Leu
   645 650 655
   Leu Cys Ile Leu Thr Ile Thr Val Ile Thr Leu Leu Ile Phe Leu Arg
   660 665 670
   Arg Arg Leu Arg Lys Gln Ala Arg Ala His Gly Lys Ser Val Pro Glu
   675 680 685
   Ile His Glu Gln Leu Val Thr Tyr Asp Glu Glu Gly Gly Gly Glu Met
   690 695 700
   Asp Thr Thr Ser Tyr Asp Val Ser Val Leu Asn Ser Val Arg Arg Gly
   705 710 715 720
   Gly Ala Lys Pro Pro Arg Pro Ala Leu Asp Ala Arg Pro Ser Leu Tyr
   725 730 735
   Ala Gln Val Gln Lys Pro Pro Arg His Ala Pro Gly Ala His Gly Gly
   740 745 750
   Pro Gly Glu Met Ala Ala Met Ile Glu Val Lys Lys Asp Glu Ala Asp
   755 760 765
   His Asp Gly Asp Gly Pro Pro Tyr Asp Thr Leu His Ile Tyr Gly Tyr
   770 775 780
   Glu Gly Ser Glu Ser Ile Ala Glu Ser Leu Ser Ser Leu Gly Thr Asp
   785 790 795 800
   Ser Ser Asp Ser Asp Val Asp Tyr Asp Phe Leu Asn Asp Trp Gly Pro
   805 810 815
   Arg Phe Lys Met Leu Ala Glu Leu Tyr Gly Ser Asp Pro Arg Glu Glu
   820 825 830
   Leu Leu Tyr
   835
<210> 38
   <211> 60
   <212> PRT
   <213> Artificial sequence
<220>
   <223> dérivé de protéine humaine
<220>
   <221> SITE
   <222> (56) .. (56)
   <223> la tyrosine Y56 est phosphorylée par la src kinase
<400> 38
   Glu Cys Asp Ser Glu Leu Glu Ile Lys Arg Tyr Lys Asn Arg Val Ala
   1               5                   10                  15
   Ser Arg Lys Cys Arg Ala Lys Phe Lys Gln Leu Leu Gln His Tyr Arg
               20                  25                  30
   Glu Val Ala Ala Ala Lys Ser Ser Glu Asn Asp Arg Leu Arg Leu Leu
           35                  40                  45
   Leu Lys Gln Arg Pro Ser Leu Tyr Ala Gln Val Gln
       50                  55                  60
<210> 39
   <211> 444
   <212> PRT
   <213> Artificial sequence
<220>
   <223> dérivé de protéine humaine
<400> 39
   Glu Cys Asp Ser Glu Leu Glu Ile Lys Arg Tyr Lys Asn Arg Val Ala
   1               5                   10                  15
   Ser Arg Lys Cys Arg Ala Lys Phe Lys Gln Leu Leu Gln His Tyr Arg
               20                  25                  30
   Glu Val Ala Ala Ala Lys Ser Ser Glu Asn Asp Arg Leu Arg Leu Leu
           35                  40                  45
   Leu Lys Gln Met Glu Glu Pro Gln Ser Asp Pro Ser Val Glu Pro Pro
       50                  55                  60
   Leu Ser Gln Glu Thr Phe Ser Asp Leu Trp Lys Leu Leu Pro Glu Asn
   65                  70                  75                  80
   Asn Val Leu Ser Pro Leu Pro Ser Gln Ala Met Asp Asp Leu Met Leu
                   85                  90                  95
   Ser Pro Asp Asp Ile Glu Gln Trp Phe Thr Glu Asp Pro Gly Pro Asp
   100 105 110
   Glu Ala Pro Arg Met Pro Glu Ala Ala Pro Pro Val Ala Pro Ala Pro
   115 120 125
   Ala Ala Pro Thr Pro Ala Ala Pro Ala Pro Ala Pro Ser Trp Pro Leu
   130 135 140
   Ser Ser Ser Val Pro Ser Gln Lys Thr Tyr Gln Gly Ser Tyr Gly Phe
   145 150 155 160
   Arg Leu Gly Phe Leu His Ser Gly Thr Ala Lys Ser Val Thr Cys Thr
   165 170 175
   Tyr Ser Pro Ala Leu Asn Lys Met Phe Cys Gln Leu Ala Lys Thr Cys
   180 185 190
   Pro Val Gln Leu Trp Val Asp Ser Thr Pro Pro Pro Gly Thr Arg Val
   195 200 205
   Arg Ala Met Ala Ile Tyr Lys Gln Ser Gln His Met Thr Glu Val Val
   210 215 220
   Arg Arg Cys Pro His His Glu Arg Cys Ser Asp Ser Asp Gly Leu Ala
   225 230 235 240
   Pro Pro Gln His Leu Ile Arg Val Glu Gly Asn Leu Arg Val Glu Tyr
   245 250 255
   Leu Asp Asp Arg Asn Thr Phe Arg His Ser Val Val Val Pro Tyr Glu
   260 265 270
   Pro Pro Glu Val Gly Ser Asp Cys Thr Thr Ile His Tyr Asn Tyr Met
   275 280 285
   Cys Asn Ser Ser Cys Met Gly Gly Met Asn Arg Arg Pro Ile Leu Thr
   290 295 300
   Ile Ile Thr Leu Glu Asp Ser Ser Gly Asn Leu Leu Gly Arg Asn Ser
   305 310 315 320
   Phe Glu Val Arg Val Cys Ala Cys Pro Gly Arg Asp Arg Arg Thr Glu
   325 330 335
   Glu Glu Asn Leu Arg Lys Lys Gly Glu Pro His His Glu Leu Pro Pro
   340 345 350
   Gly Ser Thr Lys Arg Ala Leu Pro Asn Asn Thr Ser Ser Ser Pro Gln
           355                 360                 365
   Pro Lys Lys Lys Pro Leu Asp Gly Glu Tyr Phe Thr Leu Gln Ile Arg
       370                 375                 380
   Gly Arg Glu Arg Phe Glu Met Phe Arg Glu Leu Asn Glu Ala Leu Glu
   385                 390                 395                 400
   Leu Lys Asp Ala Gln Ala Gly Lys Glu Pro Gly Gly Ser Arg Ala His
                   405                 410                 415
   Ser Ser His Leu Lys Ser Lys Lys Gly Gln Ser Thr Ser Arg His Lys
               420                 425                 430
   Lys Leu Met Phe Lys Thr Glu Gly Pro Asp Ser Asp
           435                 440
<210> 40
   <211> 371
   <212> PRT
   <213> Séquence artificielle
<400> 40
   Met Pro Lys Pro Ile Asn Val Arg Val Thr Thr Met Asp Ala Glu Leu
   1 5 10 15
   Glu Phe Ala Ile Gln Pro Asn Thr Thr Gly Lys Gln Leu Phe Asp Gln
   20 25 30
   Val Val Lys Thr Ile Gly Leu Arg Glu Val Trp Tyr Phe Gly Leu His
   35 40 45
   Tyr Val Asp Asn Lys Gly Phe Pro Thr Trp Leu Lys Leu Asp Lys Lys
   50 55 60
   Val Ser Ala Gln Glu Val Arg Lys Glu Asn Pro Leu Gln Phe Lys Phe
   65 70 75 80
   Arg Ala Lys Phe Tyr Pro Glu Asp Val Ala Glu Glu Leu Ile Gln Asp
   85 90 95
   Ile Thr Gln Lys Leu Phe Phe Leu Gln Val Lys Glu Gly Ile Leu Ser
   100 105 110
   Asp Glu Ile Tyr Cys Pro Pro Glu Thr Ala Val Leu Leu Gly Ser Tyr
   115 120 125
   Ala Val Gln Ala Lys Phe Gly Asp Tyr Asn Lys Glu Val His Lys Ser
   130 135 140
   Gly Tyr Leu Ser Ser Glu Arg Leu Ile Pro Gln Arg Val Met Asp Gln
   145 150 155 160
   His Lys Leu Thr Arg Asp Gln Trp Glu Asp Arg Ile Gln Val Trp His
   165 170 175
   Ala Glu His Arg Gly Met Leu Lys Asp Asn Ala Met Leu Glu Tyr Leu
   180 185 190
   Lys Ile Ala Gln Asp Leu Glu Met Tyr Gly Ile Asn Tyr Phe Glu Ile
   195 200 205
   Lys Asn Lys Lys Gly Thr Asp Leu Trp Leu Gly Val Asp Ala Leu Gly
   210 215 220
   Leu Asn Ile Tyr Glu Lys Asp Asp Lys Leu Thr Pro Lys Ile Gly Phe
   225 230 235 240
   Pro Trp Ser Glu Ile Arg Asn Ile Ser Phe Asn Asp Lys Lys Phe Val
   245 250 255
   Ile Lys Pro Ile Asp Lys Lys Ala Pro Asp Phe Val Phe Tyr Ala Pro
   260 265 270
   Arg Leu Arg Ile Asn Lys Arg Ile Leu Gln Leu Cys Met Gly Asn His
   275 280 285
   Glu Leu Tyr Met Arg Arg Arg Lys Pro Asp Thr Ile Glu Val Gln Gln
   290 295 300
   Met Lys Ala Gln Ala Arg Glu Glu Lys His Gln Lys Gln Leu Glu Arg
   305 310 315 320
   Glu Cys Asp Ser Glu Leu Glu Ile Lys Arg Tyr Lys Asn Arg Val Ala
   325 330 335
   Ser Arg Lys Cys Arg Ala Lys Phe Lys Gln Leu Leu Gln His Tyr Arg
   340 345 350
   Glu Val Ala Ala Ala Lys Ser Ser Glu Asn Asp Arg Leu Arg Leu Leu
   355 360 365
   Leu Lys Gln
   370
<210> 41
   <211> 251
   <212> PRT
   <213> Artificial sequence
<220>
   <223> dérivé de protéine humaine
<400> 41
   Glu Cys Asp Ser Glu Leu Glu Ile Lys Arg Tyr Lys Asn Arg Val Ala
   1               5                   10                  15
   Ser Arg Lys Cys Arg Ala Lys Phe Lys Gln Leu Leu Gln His Tyr Arg
               20                  25                  30
   Glu Val Ala Ala Ala Lys Ser Ser Glu Asn Asp Arg Leu Arg Leu Leu
           35                  40                  45
   Leu Lys Gln Met His His His His His His His His Lys Arg Tyr Lys
       50                  55                  60
   Asn Arg Val Ala Ser Arg Lys Cys Arg Ala Lys Phe Lys Gln Leu Leu
   65                  70                  75                  80
   Gln His Tyr Arg Glu Val Ala Ala Ala Lys Ser Ser Glu Asn Asp Arg
                   85                  90                  95
   Leu Arg Leu Leu Leu Lys Gln Met Cys Lys Leu Ala Lys Phe Val Ala
               100                 105                 110
   Ala Trp Thr Leu Lys Ala Ala Ala Gly Ser Asp Glu Val Ser Gly Leu
           115                 120                 125
   Glu Gln Leu Glu Ser Ile Ile Asn Phe Glu Lys Leu Thr Glu Trp Thr
       130                 135                 140
   Ser Ser Asn Val Met Glu Glu Arg Lys Ile Lys Val Tyr Leu Pro Arg
   145                 150                 155                 160
   Met Lys Met Glu Glu Lys Tyr Asn Leu Thr Ser Val Leu Met Ala Met
                   165                 170                 175
   Gly Ile Thr Asp Val Phe Ser Ser Ser Ala Asn Leu Ser Gly Ile Ser
               180                 185                 190
   Ser Ala Glu Ser Leu Lys Ile Ser Gln Ala Val His Ala Ala His Ala
           195                 200                 205
   Glu Ile Asn Glu Ala Gly Arg Glu Val Val Gly Ser Ala Glu Ala Gly
       210                 215                 220
   Val Asp Ala Ala Ser Val Ser Glu Glu Phe Arg Ala Asp His Pro Phe
   225 230 235 240
   Leu Phe Cys Ile Lys His Ile Ala Thr Asn Ala
   245 250
<210> 42
   <211> 349
   <212> PRT
   <213> Artificial sequence
<220>
   <223> dérivé de protéine humaine
<400> 42
   Met Arg His Met Gln Ser Ala Thr Arg Ala Thr Leu Val Cys Gly Ser
   1               5                   10                  15
   Gly Val Lys Gln Ile Ile Ala Lys Gly His Pro Asn Thr Arg Val Phe
               20                  25                  30
   Gly Ala Arg Lys Ala Arg Ile Pro Glu Arg Glu Val Leu Ala Ala Lys
           35                  40                  45
   Pro Lys Ile Thr Arg Ile Thr His Leu Asn Leu Gln Pro Gln Glu Arg
       50                  55                  60
   Gln Ala Phe Tyr Arg Leu Leu Glu Asn Glu Leu Ile Gln Glu Phe Leu
   65                  70                  75                  80
   Ser Met Asp Ser Cys Leu Lys Ile Ser Asp Lys Tyr Leu Ile Ala Met
                   85                  90                  95
   Val Leu Ala Tyr Phe Lys Arg Ala Gly Leu Tyr Thr Gly Glu Tyr Thr
               100                 105                 110
   Thr Met Asn Phe Phe Val Ala Leu Tyr Leu Ala Asn Asp Met Glu Glu
           115                 120                 125
   Asp Glu Glu Asp Tyr Lys Tyr Glu Ile Phe Pro Trp Ala Leu Gly Asp
       130                 135                 140
   Ser Trp Arg Glu Phe Phe Pro Gln Phe Leu Arg Leu Arg Asp Asn Phe
   145                 150                 155                 160
   Trp Ala Lys Met Asn Tyr Arg Ala Val Val Ser Arg Arg Cys Cys Asp
                   165                 170                 175
   Glu Val Met Ala Lys Asp Pro Thr His Trp Ala Trp Leu Arg Asp Arg
               180                 185                 190
   Pro Ile His His Ser Gly Ala Leu Arg Gly Tyr Leu Arg Asn Glu Asp
   195 200 205
   Asp Phe Phe Pro Arg Gly Pro Gly Leu Thr Pro Ala Ser Cys Ala Leu
   210 215 220
   Cys His Lys Ala Ser Val Cys Asp Ser Gly Gly Val Ala His Asp Asn
   225 230 235 240
   Ser Ser Pro Glu Gln Glu Ile Phe His Tyr Thr Asn Arg Glu Trp Ser
   245 250 255
   Gln Glu Leu Leu Ile Leu Pro Pro Glu Leu Leu Leu Asp Pro Glu Ser
   260 265 270
   Thr Tyr Asp Ile His Ile Phe Gln Glu Pro Leu Val Gly Leu Glu Pro
   275 280 285
   Asp Gly Ala Ala Leu Glu Trp His His Leu Glu Cys Asp Ser Glu Leu
   290 295 300
   Glu Ile Lys Arg Tyr Lys Asn Arg Val Ala Ser Arg Lys Cys Arg Ala
   305 310 315 320
   Lys Phe Lys Gln Leu Leu Gln His Tyr Arg Glu Val Ala Ala Ala Lys
   325 330 335
   Ser Ser Glu Asn Asp Arg Leu Arg Leu Leu Leu Lys Gln
   340 345
<210> 43
   <211> 182
   <212> PRT
   <213> Artificial sequence
<220>
   <223> dérivé de protéine humaine
<400> 43
   Gln Glu Val Gln Ala Phe Leu Ser Leu Leu Glu Asp Ser Phe Val Gln
   1               5                   10                  15
   Glu Phe Leu Ser Lys Asp Pro Cys Phe Gln Ile Ser Asp Lys Tyr Leu
               20                  25                  30
   Leu Ala Met Val Leu Val Tyr Phe Gln Arg Ala His Leu Lys Leu Ser
           35                  40                  45
   Glu Tyr Thr His Ser Ser Leu Phe Leu Ala Leu Tyr Leu Ala Asn Asp
       50                  55                  60
   Met Glu Glu Asp Leu Glu Gly Pro Lys Cys Glu Ile Phe Pro Trp Ala
   65                  70                  75                  80
   Leu Gly Lys Asp Trp Cys Leu Arg Val Gly Lys Phe Leu His Gln Arg
                   85                  90                  95
   Asp Lys Leu Trp Ala Arg Met Gly Phe Arg Ala Val Val Ser Arg Gln
               100                 105                 110
   Cys Cys Glu Glu Val Met Ala Lys Glu Pro Phe His Trp Ala Trp Thr
           115                 120                 125
   Arg Asp Arg Glu Cys Asp Ser Glu Leu Glu Ile Lys Arg Tyr Lys Asn
       130                 135                 140
   Arg Val Ala Ser Arg Lys Cys Arg Ala Lys Phe Lys Gln Leu Leu Gln
   145                 150                 155                 160
   His Tyr Arg Glu Val Ala Ala Ala Lys Ser Ser Glu Asn Asp Arg Leu
                   165                 170                 175
   Arg Leu Leu Leu Lys Gln
               180
<210> 44
   <211> 462
   <212> PRT
   <213> Artificial sequence
<220>
   <223> dérivé de protéine humaine <400> 44
<210> 45
   <211> 71
   <212> PRT
   <213> Artificial sequence
<220>
   <223> dérivé de protéine humaine
<400> 45
   Glu Leu Met Ile Met Lys Ala Leu Lys Trp Arg Leu Ser Pro Leu Thr
   1 5 10 15
   Ile Val Ser Trp Glu Cys Asp Ser Glu Leu Glu Ile Lys Arg Tyr Lys
   20 25 30
   Asn Arg Val Ala Ser Arg Lys Cys Arg Ala Lys Phe Lys Gln Leu Leu
   35 40 45
   Gln His Tyr Arg Glu Val Ala Ala Ala Lys Ser Ser Glu Asn Asp Arg
   50 55 60
   Leu Arg Leu Leu Leu Lys Gln
   65 70
<210> 46
   <211> 377
   <212> PRT
   <213> Artificial sequence
<220>
   <223> dérivé de protéine humaine
<400> 46
   Met Asp Leu Thr Ala Ile Tyr Glu Ser Leu Leu Ser Leu Ser Pro Asp
   1               5                   10                  15
   Val Pro Val Pro Ser Asp His Gly Gly Thr Glu Ser Ser Pro Gly Trp
               20                  25                  30
   Gly Ser Ser Gly Pro Trp Ser Leu Ser Pro Ser Asp Ser Ser Pro Ser
           35                  40                  45
   Gly Val Thr Ser Arg Leu Pro Gly Arg Ser Thr Ser Leu Val Glu Gly
       50                  55                  60
   Arg Ser Cys Gly Trp Val Pro Pro Pro Pro Gly Phe Ala Pro Leu Ala
   65                  70                  75                  80
   Pro Arg Leu Gly Pro Glu Leu Ser Pro Ser Pro Thr Ser Pro Thr Ala
                   85                  90                  95
   Thr Ser Thr Thr Pro Ser Arg Tyr Lys Thr Glu Leu Cys Arg Thr Phe
               100                 105                 110
   Ser Glu Ser Gly Arg Cys Arg Tyr Gly Ala Lys Cys Gln Phe Ala His
           115                 120                 125
   Gly Leu Gly Glu Leu Arg Gln Ala Asn Arg His Pro Lys Tyr Lys Thr
       130                 135                 140
   Glu Leu Cys His Lys Phe Tyr Leu Gln Gly Arg Cys Pro Tyr Gly Ser
   145                 150                 155                 160
   Arg Cys His Phe Ile His Asn Pro Ser Glu Asp Leu Ala Ala Pro Gly
                   165                 170                 175
   His Pro Pro Val Leu Arg Gln Ser Ile Ser Phe Ser Gly Leu Pro Ser
               180                 185                 190
   Gly Arg Arg Thr Ser Pro Pro Pro Pro Gly Leu Ala Gly Pro Ser Leu
           195                 200                 205
   Ser Ser Ser Ser Phe Ser Pro Ser Ser Ser Pro Pro Pro Pro Gly Asp
       210                 215                 220
   Leu Pro Leu Ser Pro Ser Ala Phe Ser Ala Ala Pro Gly Thr Pro Leu
   225                 230                 235                 240
   Ala Arg Arg Asp Pro Thr Pro Val Cys Cys Pro Ser Cys Arg Arg Ala
   245 250 255
   Thr Pro Ile Ser Val Trp Gly Pro Leu Gly Gly Leu Val Arg Thr Pro
   260 265 270
   Ser Val Gln Ser Leu Gly Ser Asp Pro Asp Glu Tyr Ala Ser Ser Gly
   275 280 285
   Ser Ser Leu Gly Gly Ser Asp Ser Pro Val Phe Glu Ala Gly Val Phe
   290 295 300
   Ala Pro Pro Gln Pro Val Ala Ala Pro Arg Arg Leu Pro Ile Phe Asn
   305 310 315 320
   Arg Ile Ser Val Ser Glu Glu Cys Asp Ser Glu Leu Glu Ile Lys Arg
   325 330 335
   Tyr Lys Asn Arg Val Ala Ser Arg Lys Cys Arg Ala Lys Phe Lys Gln
   340 345 350
   Leu Leu Gln His Tyr Arg Glu Val Ala Ala Ala Lys Ser Ser Glu Asn
   355 360 365
   Asp Arg Leu Arg Leu Leu Leu Lys Gln
   370 375
<210> 47
   <211> 406
   <212> PRT
   <213> Artificial sequence
<220>
   <223> dérivé de protéine humaine
<400> 47
   Met Pro Phe Leu Gly Gln Asp Trp Arg Ser Pro Gly Gln Asn Trp Val
   1               5                   10                  15
   Lys Thr Ala Asp Gly Trp Lys Arg Phe Leu Asp Glu Lys Ser Gly Ser
               20                  25                  30
   Phe Val Ser Asp Leu Ser Ser Tyr Cys Asn Lys Glu Val Tyr Asn Lys
           35                  40                  45
   Glu Asn Leu Phe Asn Ser Leu Asn Tyr Asp Val Ala Ala Lys Lys Arg
       50                  55                  60
   Lys Lys Asp Met Leu Asn Ser Lys Thr Lys Thr Gln Tyr Phe His Gln
   65                  70                  75                  80
   Glu Lys Trp Ile Tyr Val His Lys Gly Ser Thr Lys Glu Arg His Gly
                   85                  90                  95
   Tyr Cys Thr Leu Gly Glu Ala Phe Asn Arg Leu Asp Phe Ser Thr Ala
               100                 105                 110
   Ile Leu Asp Ser Arg Arg Phe Asn Tyr Val Val Arg Leu Leu Glu Leu
           115                 120                 125
   Ile Ala Lys Ser Gln Leu Thr Ser Leu Ser Gly Ile Ala Gln Lys Asn
       130                 135                 140
   Phe Met Asn Ile Leu Glu Lys Val Val Leu Lys Val Leu Glu Asp Gln
   145                 150                 155                 160
   Gln Asn Ile Arg Leu Ile Arg Glu Leu Leu Gln Thr Leu Tyr Thr Ser
                   165                 170                 175
   Leu Cys Thr Leu Val Gln Arg Val Gly Lys Ser Val Leu Val Gly Asn
               180                 185                 190
   Ile Asn Met Trp Val Tyr Arg Met Glu Thr Ile Leu His Trp Gln Gln
           195                 200                 205
   Gln Leu Asn Asn Ile Gln Ile Thr Arg Pro Ala Phe Lys Gly Leu Thr
       210                 215                 220
   Phe Thr Asp Leu Pro Leu Cys Leu Gln Leu Asn Ile Met Gln Arg Leu
   225                 230                 235                 240
   Ser Asp Gly Arg Asp Leu Val Ser Leu Gly Gln Ala Ala Pro Asp Leu
                   245                 250                 255
   His Val Leu Ser Glu Asp Arg Leu Leu Trp Lys Lys Leu Cys Gln Tyr
               260                 265                 270
   His Phe Ser Glu Arg Gln Ile Arg Lys Arg Leu Ile Leu Ser Asp Lys
           275                 280                 285
   Gly Gln Leu Asp Trp Lys Lys Met Tyr Phe Lys Leu Val Arg Cys Tyr
       290                 295                 300
   Pro Arg Lys Glu Gln Tyr Gly Asp Thr Leu Gln Leu Cys Lys His Cys
   305                 310                 315                 320
   His Ile Leu Ser Trp Lys Gly Thr Asp His Pro Cys Thr Ala Asn Asn
                   325                 330                 335
   Pro Glu Ser Cys Ser Val Ser Leu Ser Pro Gln Asp Phe Ile Asn Leu
   340 345 350
   Phe Lys Phe Glu Cys Asp Ser Glu Leu Glu Ile Lys Arg Tyr Lys Asn
   355 360 365
   Arg Val Ala Ser Arg Lys Cys Arg Ala Lys Phe Lys Gln Leu Leu Gln
   370 375 380
   His Tyr Arg Glu Val Ala Ala Ala Lys Ser Ser Glu Asn Asp Arg Leu
   385 390 395 400
   Arg Leu Leu Leu Lys Gln
   405
<210> 48
   <211> 349
   <212> PRT
   <213> Artificial sequence
<220>
   <223> dérivé de protéine humaine
<400> 48
   Gly Gln Thr Pro Ala Ser Ala Gln Ala Pro Ala Gln Thr Pro Ala Pro
   1               5                   10                  15
   Ala Leu Pro Gly Pro Ala Leu Pro Gly Pro Phe Pro Gly Gly Arg Val
               20                  25                  30
   Val Arg Leu His Pro Val Ile Leu Ala Ser Ile Val Asp Ser Tyr Glu
           35                  40                  45
   Arg Arg Asn Glu Gly Ala Ala Arg Val Ile Gly Thr Leu Leu Gly Thr
       50                  55                  60
   Val Asp Lys His Ser Val Glu Val Thr Asn Cys Phe Ser Val Pro His
   65                  70                  75                  80
   Asn Glu Ser Glu Asp Glu Val Ala Val Asp Met Glu Phe Ala Lys Asn
                   85                  90                  95
   Met Tyr Glu Leu His Lys Lys Val Ser Pro Asn Glu Leu Ile Leu Gly
               100                 105                 110
   Trp Tyr Ala Thr Gly His Asp Ile Thr Glu His Ser Val Leu Ile His
           115                 120                 125
   Glu Tyr Tyr Ser Arg Glu Ala Pro Asn Pro Ile His Leu Thr Val Asp
       130                 135                 140
   Thr Ser Leu Gln Asn Gly Arg Met Ser Ile Lys Ala Tyr Val Ser Thr
   145 150 155 160
   Leu Met Gly Val Pro Gly Arg Thr Met Gly Val Met Phe Thr Pro Leu
   165 170 175
   Thr Val Lys Tyr Ala Tyr Tyr Asp Thr Glu Arg Ile Gly Val Asp Leu
   180 185 190
   Ile Met Lys Thr Cys Phe Ser Pro Asn Arg Val Ile Gly Leu Ser Ser
   195 200 205
   Asp Leu Gln Gln Val Gly Gly Ala Ser Ala Arg Ile Gln Asp Ala Leu
   210 215 220
   Ser Thr Val Leu Gln Tyr Ala Glu Asp Val Leu Ser Gly Lys Val Ser
   225 230 235 240
   Ala Asp Asn Thr Val Gly Arg Phe Leu Met Ser Leu Val Asn Gln Val
   245 250 255
   Pro Lys Ile Val Pro Asp Asp Phe Glu Thr Met Leu Asn Ser Asn Ile
   260 265 270
   Asn Asp Leu Leu Met Val Thr Tyr Leu Ala Asn Leu Thr Gln Ser Gln
   275 280 285
   Ile Ala Leu Asn Glu Lys Leu Val Asn Leu Glu Cys Asp Ser Glu Leu
   290 295 300
   Glu Ile Lys Arg Tyr Lys Asn Arg Val Ala Ser Arg Lys Cys Arg Ala
   305 310 315 320
   Lys Phe Lys Gln Leu Leu Gln His Tyr Arg Glu Val Ala Ala Ala Lys
   325 330 335
   Ser Ser Glu Asn Asp Arg Leu Arg Leu Leu Leu Lys Gln
   340 345
<210> 49
   <211> 257
   <212> PRT
   <213> Artificial sequence
<220>
   <223> dérivé de protéine humaine
<400> 49
   Met Asn Phe Gln Gln Arg Leu Gln Ser Leu Trp Thr Leu Ala Arg Pro
   1               5                   10                  15
   Phe Cys Pro Pro Leu Leu Ala Thr Ala Ser Gln Met Gln Met Val Val
   20 25 30
   Leu Pro Cys Leu Gly Phe Thr Leu Leu Leu Trp Ser Gln Val Ser Gly
   35 40 45
   Ala Gln Gly Gln Glu Phe His Phe Gly Pro Cys Gln Val Lys Gly Val
   50 55 60
   Val Pro Gln Lys Leu Trp Glu Ala Phe Trp Ala Val Lys Asp Thr Met
   65 70 75 80
   Gln Ala Gln Asp Asn Ile Thr Ser Ala Arg Leu Leu Gln Gln Glu Val
   85 90 95
   Leu Gln Asn Val Ser Asp Ala Glu Ser Cys Tyr Leu Val His Thr Leu
   100 105 110
   Leu Glu Phe Tyr Leu Lys Thr Val Phe Lys Asn Tyr His Asn Arg Thr
   115 120 125
   Val Glu Val Arg Thr Leu Lys Ser Phe Ser Thr Leu Ala Asn Asn Phe
   130 135 140
   Val Leu Ile Val Ser Gln Leu Gln Pro Ser Gln Glu Asn Glu Met Phe
   145 150 155 160
   Ser Ile Arg Asp Ser Ala His Arg Arg Phe Leu Leu Phe Arg Arg Ala
   165 170 175
   Phe Lys Gln Leu Asp Val Glu Ala Ala Leu Thr Lys Ala Leu Gly Glu
   180 185 190
   Val Asp Ile Leu Leu Thr Trp Met Gln Lys Phe Tyr Lys Leu Glu Cys
   195 200 205
   Asp Ser Glu Leu Glu Ile Lys Arg Tyr Lys Asn Arg Val Ala Ser Arg
   210 215 220
   Lys Cys Arg Ala Lys Phe Lys Gln Leu Leu Gln His Tyr Arg Glu Val
   225 230 235 240
   Ala Ala Ala Lys Ser Ser Glu Asn Asp Arg Leu Arg Leu Leu Leu Lys
   245 250 255
   Gln
<210> 50
   <211> 835
   <212> PRT
   <213> Artificial sequence
<220>
   <223> dérivé de protéine humaine
<400> 50
   Met Gln Arg Leu Met Met Leu Leu Ala Thr Ser Gly Ala Cys Leu Gly
   1               5                   10                  15
   Leu Leu Ala Val Ala Ala Val Ala Ala Ala Gly Ala Asn Pro Ala Gln
               20                  25                  30
   Arg Asp Thr His Ser Leu Leu Pro Thr His Arg Arg Gln Lys Arg Asp
           35                  40                  45
   Trp Ile Trp Asn Gln Met His Ile Asp Glu Glu Lys Asn Thr Ser Leu
       50                  55                  60
   Pro His His Val Gly Lys Ile Lys Ser Ser Val Ser Arg Lys Asn Ala
   65                  70                  75                  80
   Lys Tyr Leu Leu Lys Gly Glu Tyr Val Gly Lys Val Phe Arg Val Asp
                   85                  90                  95
   Ala Glu Thr Gly Asp Val Phe Ala Ile Glu Arg Leu Asp Arg Glu Asn
               100                 105                 110
   Ile Ser Glu Tyr His Leu Thr Ala Val Ile Val Asp Lys Asp Thr Gly
           115                 120                 125
   Glu Asn Leu Glu Thr Pro Ser Ser Phe Thr Ile Lys Val His Asp Val
       130                 135                 140
   Asn Asp Asn Trp Pro Val Phe Thr His Arg Leu Phe Asn Ala Ser Val
   145                 150                 155                 160
   Pro Glu Ser Ser Ala Val Gly Thr Ser Val Ile Ser Val Thr Ala Val
                   165                 170                 175
   Asp Ala Asp Asp Pro Thr Val Gly Asp His Ala Ser Val Met Tyr Gln
               180                 185                 190
   Ile Leu Lys Gly Lys Glu Tyr Phe Ala Ile Asp Asn Ser Gly Arg Ile
           195                 200                 205
   Ile Thr Ile Thr Lys Ser Leu Asp Arg Glu Lys Gln Ala Arg Tyr Glu
       210                 215                 220
   Ile Val Val Glu Ala Arg Asp Ala Gln Gly Leu Arg Gly Asp Ser Gly
   225 230 235 240
   Thr Ala Thr Val Leu Val Thr Leu Gln Asp Ile Asn Asp Asn Phe Pro
   245 250 255
   Phe Phe Thr Gln Thr Lys Tyr Thr Phe Val Val Pro Glu Asp Thr Arg
   260 265 270
   Val Gly Thr Ser Val Gly Ser Leu Phe Val Glu Asp Pro Asp Glu Pro
   275 280 285
   Gln Asn Arg Met Thr Lys Tyr Ser Ile Leu Arg Gly Asp Tyr Gln Asp
   290 295 300
   Ala Phe Thr Ile Glu Thr Asn Pro Ala His Asn Glu Gly Ile Ile Lys
   305 310 315 320
   Pro Met Lys Pro Leu Asp Tyr Glu Tyr Ile Gln Gln Tyr Ser Phe Ile
   325 330 335
   Val Glu Ala Thr Asp Pro Thr Ile Asp Leu Arg Tyr Met Ser Pro Pro
   340 345 350
   Ala Gly Asn Arg Ala Gln Val Ile Ile Asn Ile Thr Asp Val Asp Glu
   355 360 365
   Pro Pro Ile Phe Gln Gln Pro Phe Tyr His Phe Gln Leu Lys Glu Asn
   370 375 380
   Gln Lys Lys Pro Leu Ile Gly Thr Val Leu Ala Met Asp Pro Asp Ala
   385 390 395 400
   Ala Arg His Ser Ile Gly Tyr Ser Ile Arg Arg Thr Ser Asp Lys Gly
   405 410 415
   Gln Phe Phe Arg Val Thr Lys Lys Gly Asp Ile Tyr Asn Glu Lys Glu
   420 425 430
   Leu Asp Arg Glu Val Tyr Pro Trp Tyr Asn Leu Thr Val Glu Ala Lys
   435 440 445
   Glu Leu Asp Ser Thr Gly Thr Pro Thr Gly Lys Glu Ser Ile Val Gln
   450 455 460
   Val His Ile Glu Val Leu Asp Glu Asn Asp Asn Ala Pro Glu Phe Ala
   465 470 475 480
   Lys Pro Tyr Gln Pro Lys Val Cys Glu Asn Ala Val His Gly Gln Leu
   485 490 495
   Val Leu Gln Ile Ser Ala Ile Asp Lys Asp Ile Thr Pro Arg Asn Val
   500 505 510
   Lys Phe Lys Phe Thr Leu Asn Thr Glu Asn Asn Phe Thr Leu Thr Asp
   515 520 525
   Asn His Asp Asn Thr Ala Asn Ile Thr Val Lys Tyr Gly Gln Phe Asp
   530 535 540
   Arg Glu His Thr Lys Val His Phe Leu Pro Val Val Ile Ser Asp Asn
   545 550 555 560
   Gly Met Pro Ser Arg Thr Gly Thr Ser Thr Leu Thr Val Ala Val Cys
   565 570 575
   Lys Cys Asn Glu Gln Gly Glu Phe Thr Phe Cys Glu Asp Met Ala Ala
   580 585 590
   Gln Val Gly Val Ser Ile Gln Ala Val Val Ala Ile Leu Leu Cys Ile
   595 600 605
   Leu Thr Ile Thr Val Ile Thr Leu Leu Ile Phe Leu Arg Arg Arg Leu
   610 615 620
   Arg Lys Gln Ala Arg Ala His Gly Lys Ser Val Pro Glu Ile His Glu
   625 630 635 640
   Gln Leu Val Thr Tyr Asp Glu Glu Gly Gly Gly Glu Met Asp Thr Thr
   645 650 655
   Ser Tyr Asp Val Ser Val Leu Asn Ser Val Arg Arg Gly Gly Ala Lys
   660 665 670
   Pro Pro Arg Pro Ala Leu Asp Ala Arg Pro Ser Leu Tyr Ala Gln Val
   675 680 685
   Gln Lys Pro Pro Arg His Ala Pro Gly Ala His Gly Gly Pro Gly Glu
   690 695 700
   Met Ala Ala Met Ile Glu Val Lys Lys Asp Glu Ala Asp His Asp Gly
   705 710 715 720
   Asp Gly Pro Pro Tyr Asp Thr Leu His Ile Tyr Gly Tyr Glu Gly Ser
   725 730 735
   Glu Ser Ile Ala Glu Ser Leu Ser Ser Leu Gly Thr Asp Ser Ser Asp
   740 745 750
   Ser Asp Val Asp Tyr Asp Phe Leu Asn Asp Trp Gly Pro Arg Phe Lys
   755 760 765
   Met Leu Ala Glu Leu Tyr Gly Ser Asp Pro Arg Glu Glu Leu Leu Tyr
   770 775 780
   Glu Cys Asp Ser Glu Leu Glu Ile Lys Arg Tyr Lys Asn Arg Val Ala
   785 790 795 800
   Ser Arg Lys Cys Arg Ala Lys Phe Lys Gln Leu Leu Gln His Tyr Arg
   805 810 815
   Glu Val Ala Ala Ala Lys Ser Ser Glu Asn Asp Arg Leu Arg Leu Leu
   820 825 830
   Leu Lys Gln
   835
<210> 51
   <211> 60
   <212> PRT
   <213> Artificial sequence
<220>
   <223> dérivé de protéine humaine
<220>
   <221> SITE
   <222> (5)..(5)
   <223> la tyrosine Y5 est phosphorylée par la src kinase
<400> 51
   Arg Pro Ser Leu Tyr Ala Gln Val Gln Glu Cys Asp Ser Glu Leu Glu
   1 5 10 15
   Ile Lys Arg Tyr Lys Asn Arg Val Ala Ser Arg Lys Cys Arg Ala Lys
   20 25 30
   Phe Lys Gln Leu Leu Gln His Tyr Arg Glu Val Ala Ala Ala Lys Ser
   35 40 45
   Ser Glu Asn Asp Arg Leu Arg Leu Leu Leu Lys Gln
   50 55 60
<210> 52
   <211> 444
   <212> PRT
   <213> Artificial sequence
<220>
   <223> dérivé de protéine humaine
<400> 52
   Met Glu Glu Pro Gln Ser Asp Pro Ser Val Glu Pro Pro Leu Ser Gln
   1               5                   10                  15
   Glu Thr Phe Ser Asp Leu Trp Lys Leu Leu Pro Glu Asn Asn Val Leu
               20                  25                  30
   Ser Pro Leu Pro Ser Gln Ala Met Asp Asp Leu Met Leu Ser Pro Asp
           35                  40                  45
   Asp Ile Glu Gln Trp Phe Thr Glu Asp Pro Gly Pro Asp Glu Ala Pro
       50                  55                  60
   Arg Met Pro Glu Ala Ala Pro Pro Val Ala Pro Ala Pro Ala Ala Pro
   65                  70                  75                  80
   Thr Pro Ala Ala Pro Ala Pro Ala Pro Ser Trp Pro Leu Ser Ser Ser
                   85                  90                  95
   Val Pro Ser Gln Lys Thr Tyr Gln Gly Ser Tyr Gly Phe Arg Leu Gly
               100                 105                 110
   Phe Leu His Ser Gly Thr Ala Lys Ser Val Thr Cys Thr Tyr Ser Pro
           115                 120                 125
   Ala Leu Asn Lys Met Phe Cys Gln Leu Ala Lys Thr Cys Pro Val Gln
       130                 135                 140
   Leu Trp Val Asp Ser Thr Pro Pro Pro Gly Thr Arg Val Arg Ala Met
   145                 150                 155                 160
   Ala Ile Tyr Lys Gln Ser Gln His Met Thr Glu Val Val Arg Arg Cys
                   165                 170                 175
   Pro His His Glu Arg Cys Ser Asp Ser Asp Gly Leu Ala Pro Pro Gln
               180                 185                 190
   His Leu Ile Arg Val Glu Gly Asn Leu Arg Val Glu Tyr Leu Asp Asp
           195                 200                 205
   Arg Asn Thr Phe Arg His Ser Val Val Val Pro Tyr Glu Pro Pro Glu
       210                 215                 220
   Val Gly Ser Asp Cys Thr Thr Ile His Tyr Asn Tyr Met Cys Asn Ser
   225                 230                 235                 240
   Ser Cys Met Gly Gly Met Asn Arg Arg Pro Ile Leu Thr Ile Ile Thr
   245 250 255
   Leu Glu Asp Ser Ser Gly Asn Leu Leu Gly Arg Asn Ser Phe Glu Val
   260 265 270
   Arg Val Cys Ala Cys Pro Gly Arg Asp Arg Arg Thr Glu Glu Glu Asn
   275 280 285
   Leu Arg Lys Lys Gly Glu Pro His His Glu Leu Pro Pro Gly Ser Thr
   290 295 300
   Lys Arg Ala Leu Pro Asn Asn Thr Ser Ser Ser Pro Gln Pro Lys Lys
   305 310 315 320
   Lys Pro Leu Asp Gly Glu Tyr Phe Thr Leu Gln Ile Arg Gly Arg Glu
   325 330 335
   Arg Phe Glu Met Phe Arg Glu Leu Asn Glu Ala Leu Glu Leu Lys Asp
   340 345 350
   Ala Gln Ala Gly Lys Glu Pro Gly Gly Ser Arg Ala His Ser Ser His
   355 360 365
   Leu Lys Ser Lys Lys Gly Gln Ser Thr Ser Arg His Lys Lys Leu Met
   370 375 380
   Phe Lys Thr Glu Gly Pro Asp Ser Asp Glu Cys Asp Ser Glu Leu Glu
   385 390 395 400
   Ile Lys Arg Tyr Lys Asn Arg Val Ala Ser Arg Lys Cys Arg Ala Lys
   405 410 415
   Phe Lys Gln Leu Leu Gln His Tyr Arg Glu Val Ala Ala Ala Lys Ser
   420 425 430
   Ser Glu Asn Asp Arg Leu Arg Leu Leu Leu Lys Gln
   435 440
<210> 53
   <211> 371
   <212> PRT
   <213> séquence artificielle
<400> 53
   Glu Cys Asp Ser Glu Leu Glu Ile Lys Arg Tyr Lys Asn Arg Val Ala
   1               5                   10                  15
   Ser Arg Lys Cys Arg Ala Lys Phe Lys Gln Leu Leu Gln His Tyr Arg
               20                  25                  30
   Glu Val Ala Ala Ala Lys Ser Ser Glu Asn Asp Arg Leu Arg Leu Leu
           35                  40                  45
   Leu Lys Gln Met Pro Lys Pro Ile Asn Val Arg Val Thr Thr Met Asp
       50                  55                  60
   Ala Glu Leu Glu Phe Ala Ile Gln Pro Asn Thr Thr Gly Lys Gln Leu
   65                  70                  75                  80
   Phe Asp Gln Val Val Lys Thr Ile Gly Leu Arg Glu Val Trp Tyr Phe
                   85                  90                  95
   Gly Leu His Tyr Val Asp Asn Lys Gly Phe Pro Thr Trp Leu Lys Leu
               100                 105                 110
   Asp Lys Lys Val Ser Ala Gln Glu Val Arg Lys Glu Asn Pro Leu Gln
           115                 120                 125
   Phe Lys Phe Arg Ala Lys Phe Tyr Pro Glu Asp Val Ala Glu Glu Leu
       130                 135                 140
   Ile Gln Asp Ile Thr Gln Lys Leu Phe Phe Leu Gln Val Lys Glu Gly
   145                 150                 155                 160
   Ile Leu Ser Asp Glu Ile Tyr Cys Pro Pro Glu Thr Ala Val Leu Leu
                   165                 170                 175
   Gly Ser Tyr Ala Val Gln Ala Lys Phe Gly Asp Tyr Asn Lys Glu Val
               180                 185                 190
   His Lys Ser Gly Tyr Leu Ser Ser Glu Arg Leu Ile Pro Gln Arg Val
           195                 200                 205
   Met Asp Gln His Lys Leu Thr Arg Asp Gln Trp Glu Asp Arg Ile Gln
       210                 215                 220
   Val Trp His Ala Glu His Arg Gly Met Leu Lys Asp Asn Ala Met Leu
   225                 230                 235                 240
   Glu Tyr Leu Lys Ile Ala Gln Asp Leu Glu Met Tyr Gly Ile Asn Tyr
                   245                 250                 255
   Phe Glu Ile Lys Asn Lys Lys Gly Thr Asp Leu Trp Leu Gly Val Asp
               260                 265                 270
   Ala Leu Gly Leu Asn Ile Tyr Glu Lys Asp Asp Lys Leu Thr Pro Lys
           275                 280                 285
   Ile Gly Phe Pro Trp Ser Glu Ile Arg Asn Ile Ser Phe Asn Asp Lys
       290                 295                 300
   Lys Phe Val Ile Lys Pro Ile Asp Lys Lys Ala Pro Asp Phe Val Phe
   305                 310                 315                 320
   Tyr Ala Pro Arg Leu Arg Ile Asn Lys Arg Ile Leu Gln Leu Cys Met
                   325                 330                 335
   Gly Asn His Glu Leu Tyr Met Arg Arg Arg Lys Pro Asp Thr Ile Glu
               340                 345                 350
   Val Gln Gln Met Lys Ala Gln Ala Arg Glu Glu Lys His Gln Lys Gln
           355                 360                 365
   Leu Glu Arg
       370
<210> 54
   <211> 251
   <212> PRT
   <213> Artificial sequence
<220>
   <223> dérivé de protéine humaine
<400> 54
   Met His His His His His His His His Lys Arg Tyr Lys Asn Arg Val
   1               5                   10                  15
   Ala Ser Arg Lys Cys Arg Ala Lys Phe Lys Gln Leu Leu Gln His Tyr
               20                  25                  30
   Arg Glu Val Ala Ala Ala Lys Ser Ser Glu Asn Asp Arg Leu Arg Leu
           35                  40                  45
   Leu Leu Lys Gln Met Cys Lys Leu Ala Lys Phe Val Ala Ala Trp Thr
       50                  55                  60
   Leu Lys Ala Ala Ala Gly Ser Asp Glu Val Ser Gly Leu Glu Gln Leu
   65                  70                  75                  80
   Glu Ser Ile Ile Asn Phe Glu Lys Leu Thr Glu Trp Thr Ser Ser Asn
                   85                  90                  95
   Val Met Glu Glu Arg Lys Ile Lys Val Tyr Leu Pro Arg Met Lys Met
               100                 105                 110
   Glu Glu Lys Tyr Asn Leu Thr Ser Val Leu Met Ala Met Gly Ile Thr
           115                 120                 125
   Asp Val Phe Ser Ser Ser Ala Asn Leu Ser Gly Ile Ser Ser Ala Glu
   130 135 140
   Ser Leu Lys Ile Ser Gln Ala Val His Ala Ala His Ala Glu Ile Asn
   145 150 155 160
   Glu Ala Gly Arg Glu Val Val Gly Ser Ala Glu Ala Gly Val Asp Ala
   165 170 175
   Ala Ser Val Ser Glu Glu Phe Arg Ala Asp His Pro Phe Leu Phe Cys
   180 185 190
   Ile Lys His Ile Ala Thr Asn Ala Glu Cys Asp Ser Glu Leu Glu Ile
   195 200 205
   Lys Arg Tyr Lys Asn Arg Val Ala Ser Arg Lys Cys Arg Ala Lys Phe
   210 215 220
   Lys Gln Leu Leu Gln His Tyr Arg Glu Val Ala Ala Ala Lys Ser Ser
   225 230 235 240
   Glu Asn Asp Arg Leu Arg Leu Leu Leu Lys Gln
   245 250
<210> 55
   <211> 105
   <212> PRT
   <213> Séquence artificielle
<400> 55
   Leu Ala Asp Ile Gly Val Pro Gln Pro Ala Pro Val Ala Ala Pro Ala
   1 5 10 15
   Arg Arg Thr Arg Lys Pro Gln Gln Pro Glu Ser Leu Glu Glu Cys Asp
   20 25 30
   Ser Glu Leu Glu Ile Lys Arg Tyr Lys Asn Arg Val Ala Ser Arg Lys
   35 40 45
   Cys Arg Ala Lys Phe Lys Gln Leu Leu Gln His Tyr Arg Glu Val Ala
   50 55 60
   Ala Ala Lys Ser Ser Glu Asn Asp Arg Leu Arg Leu Leu Leu Lys Gln
   65 70 75 80
   Met Cys Pro Ser Leu Asp Val Asp Ser Ile Ile Pro Arg Thr Pro Asp
   85 90 95
   Val Leu His Glu Asp Leu Leu Asn Phe
   100 105
<210> 56
   <211> 75
   <212> PRT
   <213> Séquence artificelle
<400> 56
   Cys Asp Ser Glu Leu Glu Ile Lys Arg Tyr Lys Asn Arg Val Ala Ser
   1 5 10 15
   Arg Lys Cys Arg Ala Lys Phe Lys Gln Leu Leu Gln His Tyr Arg Glu
   20 25 30
   Val Ala Ala Ala Lys Ser Ser Glu Asn Asp Arg Leu Arg Leu Leu Leu
   35 40 45
   Lys Gln Met Cys Pro Ser Leu Asp Val Asp Ser Ile Ile Pro Arg Thr
   50 55 60
   Pro Asp Val Leu His Glu Asp Leu Leu Asn Phe
   65 70 75

## Description

La présente invention concerne l'utilisation de peptides comme transporteurs destinés à l'internalisation de molécules d'intérêt dans des cellules cibles.

Les biomédicaments, c'est-à-dire les médicaments issus des biotechnologies, représentent une part de plus en plus importante dans le traitement des pathologies humaines. Ces biomédicaments sont représentés par les protéines thérapeutiques (enzymes, hormones de croissance, anticorps monoclonaux, facteurs de croissance, vaccins protéiques), les acides nucléiques (siARN, ADN, oligonucléotides), les peptides et les dérivés (PNA). Ils nécessitent, dans certains cas, des transporteurs pour être internalisés dans les cellules cibles. L'internalisation des molécules thérapeutiques a fait l'objet ces dernières années, de nombreuses recherches et développements afin d'augmenter l'efficacité d'internalisation de transporteurs, leur ciblage au niveau des cellules et des organes, mais aussi de diminuer leurs effets secondaires potentiels.

Ainsi, des familles de transporteurs ont été mises en évidence tout d'abord à partir des propriétés de transfert intracellulaire de la protéine TAT du virus VIH (Fawell, S., Seery, J., Daikh, Y., Moore, C., Chen, L. L., Pepinsky, B., and Barsoum, J. (1994) Proc Natl Acad Sci USA 91(2), 664-668 ; Vives, E., Brodin, P., and Lebleu, B. (1997) J Biol Chem 272(25), 16010-16017), mais aussi de la pénétratine issue de la troisième hélice de la protéine Antennapedia de drosophile (Derossi, D., Joliot, A. H., Chassaing, G., and Prochiantz, A. (1994) J Biol Chem 269(14), 10444-10450), de la protéine VP22 du virus de l'herpès simplex (Elliott, G., and O'Hare, P. (1997) Cell 88(2), 223-233 ; Nishi, K., and Saigo, K. (2007) J Biol Chem 282(37), 27503-27517) et de peptides synthétiques composés de répétitions d'acides aminés basiques tels que arginine ou lysine (Matsui H, Tomizawa K, Lu YF, Matsushita M. Curr Protein Pept Sci. (2003) Apr;4(2):151-7). Ces peptides naturels ou synthétiques dénommés PTD (pour Protein Transduction Domain) ou CPP (pour Cell-Penetratin Peptides) possèdent la capacité de transporter et de transférer des molécules telles que des peptides, ou des acides nucléiques par un mécanisme cellulaire dénommé endocytose. Néanmoins, l'internalisation par endocytose des molécules thérapeutiques peut avoir des conséquences sur l'activité et le devenir intracellulaire de ces molécules. En effet, il est nécessaire que les vésicules d'endocytose soient rompues pour pouvoir délivrer la molécule thérapeutique dans la cellule. Cette rupture de la membrane des vésicules d'endocytose s'effectue souvent à pH acide entraînant potentiellement une modification de la structure et de l'activité de la molécule thérapeutique associée au transporteur. D'autre part, seulement une petite partie des molécules thérapeutiques associées aux transporteurs va donc pouvoir s'échapper des endosomes pour se retrouver dans le cytoplasme diminuant l'effet des molécules.

Un autre mécanisme d'internalisation des molécules dans les cellules consiste en la formation de pores cellulaires. En effet, un petit nombre de PTD ou CPP constitués d'acides aminés hydrophobes (MPG, Pep-1, Pep-2, Pep-3, SSHR [Séquence Signal Hydrophobic Region dérivées du FGF4 et de l'intégrine β3 humaines]) sont capables de pénétrer à travers la membrane plasmique en formant des pores cellulaires. Ces pores, en fonction de leur taille, peuvent ainsi permettre la diffusion directe de la molécule thérapeutique dans le cytoplasme sans passer par les vésicules d'endocytoses (Langel, Ü. (2006) Handbook of Cell-Penetrating Péptides, 2 Ed.; Hawiger J. Curr Opin Chem Biol. 1999 Feb;3(1):89-94; Yan Liu X, Robinson D, Veach RA, Liu D, Timmons S, Collins RD, Hawiger J., J Biol Chem. 2000 Jun 2;275(22):16774-8). La formation des pores peut s'avérer dans certains cas, délétères pour la cellule qui, si ceux-ci sont trop importants en nombre ou en taille, peut entraîner une fuite du cytosol vers le milieu extracellulaire résultant en une mort cellulaire.

Rothe et Lenormand (Curr.t protoc. in Protein Sci., 54 : 18.11.1-18.11.29, 2008) décrivent une méthode de production de protéines de fusion comprenant un segment de la protéine ZEBRA (allant de l'acide aminé en position 170 à l'acide aminé en position 222) et la protéine EGFP ou la β-galactosidase. Lesdites protéines de fusion sont capables d'être internalisées dans les cellules HeLa à une concentration de 0,01 µM à 0,3 µM.

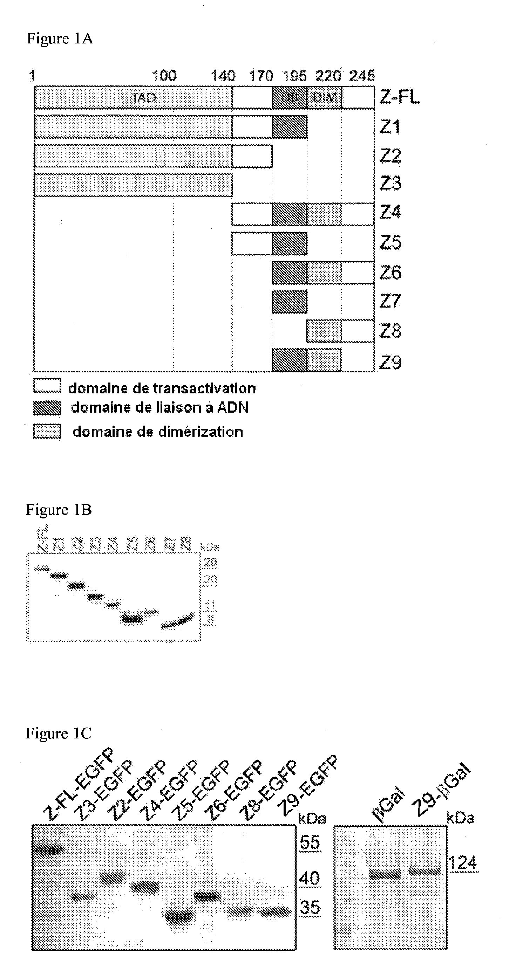

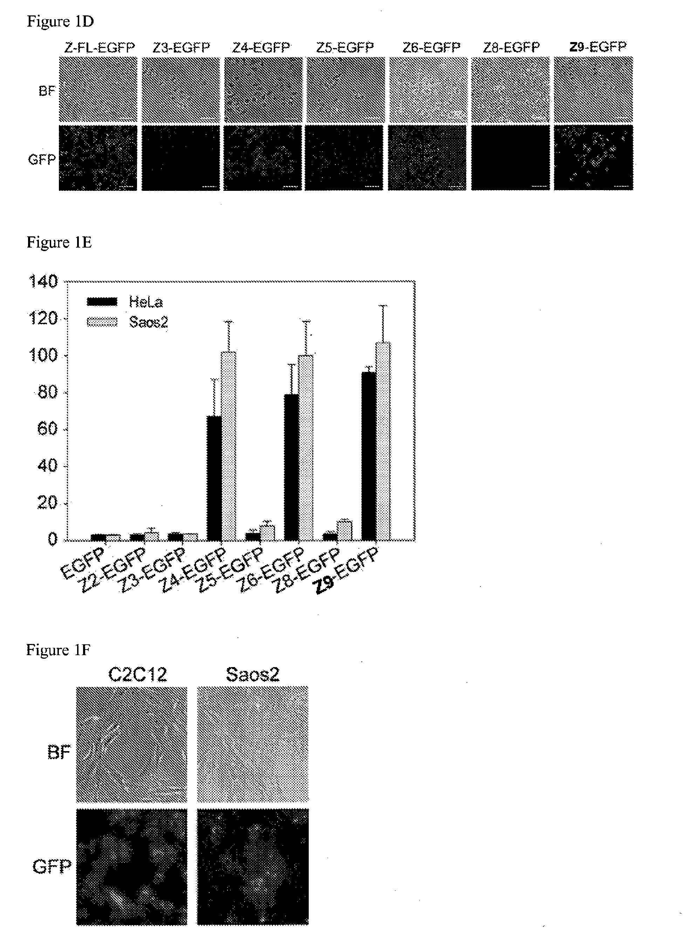

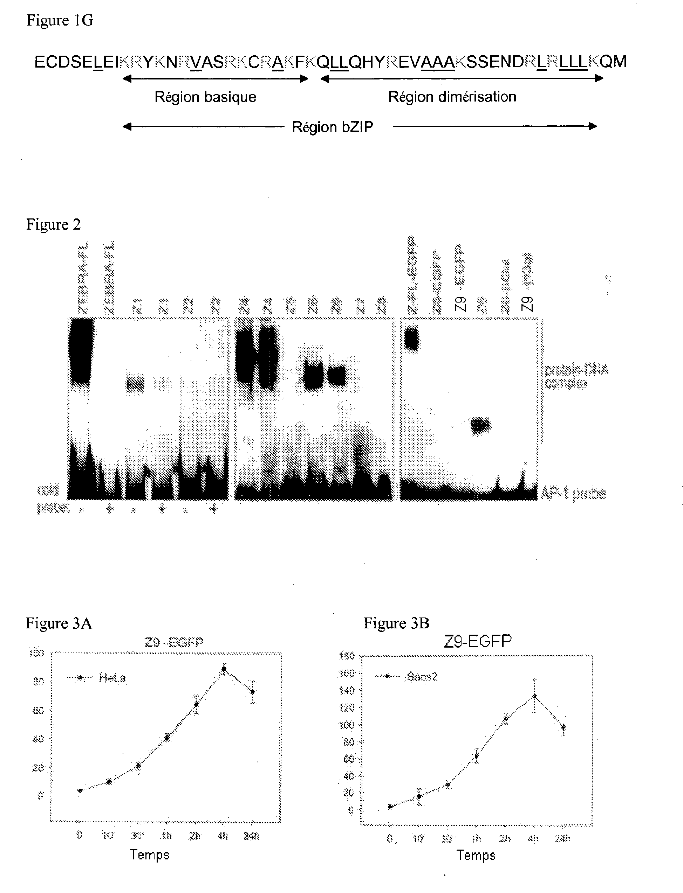

La protéine ZEBRA, représentée par la séquence SEQ ID NO : 42, est un activateur transcriptionnel issu du virus Epstein-Barr. Elle est une protéine de 245 acides aminés comprenant une région de trans-activation en N-terminal (TAD), un domaine de liaison à l'ADN (DB) et une région de dimérisation (DIM) de type leucine zipper (Figure 1). Le domaine C-terminal de ladite protéine interagit avec le domaine leucine zipper conduisant à la formation d'une poche hydrophobe qui stabilise le complexe protéine ZEBRA/ADN.

Jusqu'à présent, les voies d'internalisation empruntées par les peptides de transport, connus de l'homme du métier, telles que l'endocytose et la macropinocytose, nécessitent une dépense d'énergie importante afin de réaliser ce mécanisme de pénétration intracellulaire. De plus, cette internalisation par endocytose résulte souvent dans la dégradation du polypeptide transporté. Seule, une petite fraction des peptides de transport sont relargués dans le cytosol après rupture de la membrane des endosomes permettant aux polypeptides transportés d'exercer leur action au niveau cellulaire. Par conséquent, à l'échelle de production industrielle, afin d'assurer l'efficacité de la transduction de polypeptides d'intérêt, il est nécessaire de produire une grande quantité de transporteur et de polypeptides d'intérêt, ce qui exige parfois une procédure de production ou de purification lourde, et n'est pas réalisable pour tous les types de polypeptides d'intérêt.

Par conséquent, il existe un grand besoin de mettre à disposition un transporteur destiné à l'internalisation de molécules d'intérêt dans les cellules cibles, qui, d'une part, permet de transporter, à une concentration faible, avec une haute efficacité, des molécules d'intérêt dans les cellules cibles, en préservant la dégradation partielle ou totale des molécules d'intérêt à l'intérieur des cellules cibles, et d'autre part, présente une faible cytotoxicité vis-à-vis des cellules cibles.

Un des buts de la présente invention est de fournir de nouveaux peptides comme transporteurs destinés à l'internalisation de molécules d'intérêt dans des cellules cibles.

Un autre but de la présente invention est de fournir de nouvelles combinaisons comprenant une molécule d'intérêt et un transporteur de ladite molécule.

Un autre but de la présente invention est de fournir de nouveaux peptides de fusion comprenant une molécule d'intérêt et un transporteur de ladite molécule.

Egalement, un des buts de la présente invention est de fournir de nouvelles compositions pharmaceutiques comprenant une molécule d'intérêt et un transporteur de ladite molécule.

Par conséquent, la présente invention concerne l'utilisation :
(i) - d'un peptide comprenant la séquence d'acides aminés SEQ ID NO : 1, ou
   - d'un peptide comprenant une séquence d'acides aminés ayant 93%, notamment 95%, particulièrement 98% d'homologie d'identité de séquence avec la séquence SEQ ID NO : 1, ou
(ii) d'un acide nucléique codant
   - un peptide comprenant la séquence d'acides aminés SEQ ID NO : 1, ou
   - un peptide comprenant une séquence d'acides aminés ayant 93%, notamment 95%, particulièrement 98% d'homologie d'identité de séquence avec la séquence SEQ ID NO : 1, pour l'obtention d'un transporteur destiné à l'internalisation d'une molécule d'intérêt diagnostique ou thérapeutique dans les cellules cibles, ledit peptide étant utilisé à une concentration inférieure à 5 nM, avantageusement inférieure à 1 nM, plus avantageusement inférieure à 0,3 nM, encore plus avantageusement inférieure à 0,2nM, notamment inférieure à 0,1nM, particulièrement inférieure à 0,05nM, plus particulièrement inférieure à 0,03nM, notamment une concentration de 0,01 à 5 nM, avantageusement de 0,01 à 1 nM, plus avantageusement de 0,01 à 0,3 nM, encore plus avantageusement de 0,01 à 0,2 nM, notamment de 0,01 à 0,1 nM, particulièrement de 0,01 à 0,05 nM, plus particulièrement de 0,01 à 0,03 nM.

Par « molécule d'intérêt », on entend les polypeptides, les analogues de nucléosides, les acides nucléiques ou toute autre molécule chimique ou biologique produisant un effet utile pour le diagnostic ou le traitement d'une maladie.

Plus particulièrement, la présente invention concerne l'utilisation :
(i) - d'un peptide comprenant la séquence d'acides aminés SEQ ID NO : 1, ou
   - d'un peptide comprenant une séquence d'acides aminés ayant 93%, notamment 95%, particulièrement 98% d'homologie d'identité de séquence avec la séquence SEQ ID NO : 1, ou
(ii) d'un acide nucléique codant
   - un peptide comprenant la séquence d'acides aminés SEQ ID NO : 1, ou
   - un peptide comprenant une séquence d'acides aminés ayant 93%, notamment 95%, particulièrement 98% d'homologie d'identité de séquence avec la séquence SEQ ID NO : 1, pour l'obtention d'un transporteur destiné à l'internalisation d'un polypeptide d'intérêt diagnostique ou thérapeutique dans les cellules cibles, ledit peptide étant utilisé à une concentration inférieure à 5 nM, avantageusement inférieure à 1 nM, plus avantageusement inférieure à 0,3 nM, encore plus avantageusement inférieure à 0,2nM, notamment inférieure à 0,1nM, particulièrement inférieure à 0,05nM, plus particulièrement inférieure à 0,03nM, ledit polypeptide d'intérêt étant choisi parmi :
      1) la protéine eIF3-f, telle la protéine eIF3-f de souris représentée par la séquence SEQ ID NO : 19, ou la protéine eIF3-f humaine représentée par la séquence SEQ ID NO : 20, ou un polypeptide ayant 80%, notamment 90%, particulièrement 95% d'identité de séquence avec la séquence d'une protéine eIF3-f, ou
      2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

La présente invention repose sur la mise en évidence d'un nouveau mécanisme d'internalisation de protéines, différent de l'endocytose, et qui consiste en la pénétration directe dudit peptide dans la membrane plasmique par formation de pores. Ces pores sont de plus faible diamètre que ceux formés par des autres CPP tels que Pep-1 et ses dérivés, MPG et ses dérivés, CADY et ses dérivés et par conséquent ne sont pas délétères pour les cellules.

Compte tenu de ce mécanisme inconnu à ce jour, certains fragments peptidiques issus de la protéine ZEBRA sont capables de transporter des molécules d'intérêt, dans un très grand nombre de lignées de cellules, avec une très haute efficacité, à des concentrations faibles, car l'internalisation est majoritairement indépendante des voies traditionnelles (endocytose et macropinocytose) empruntées par les autres peptides de transport tels que les PTD (par exemple : TAT, VP22, Penetratin) et CPP (par exemple : MPG, Pep1, Pep2).

Etant donné que l'internalisation effectuée par la protéine ZEBRA est dispensée de l'internalisation préalable d'endosomes, il devient possible d'effectuer une internalisation de polypeptides d'intérêt à des concentrations faibles et d'éviter la dégradation des polypeptides d'intérêt lors de la rupture des membranes des endosomes.

L'expression « transporteur » désigne une molécule capable de transférer une autre molécule différente à travers la membrane cellulaire pour lui permettre de pénétrer dans la cellule.

L'expression « transporteur » peut être remplacée, dans la présente invention, par les expressions telles que « cargo » ou « carrier ».

L'expression « l'internalisation d'un polypeptide d'intérêt dans les cellules cibles » désigne le transfert d'un polypeptide d'intérêt de l'extérieur d'une cellule cible à l'intérieur de celle-ci.

Le peptide représenté par la séquence SEQ ID NO : 1 consiste en un fragment peptidique issu de la protéine ZEBRA (allant de l'acide aminé en position 170 à l'acide aminé en position 220). Ledit peptide comprend entre-autre, le domaine de liaison à l'ADN (allant de l'acide aminé en position 178 à l'acide aminé en position 194), qui est une région basique, et le domaine de dimérisation (allant de l'acide aminé en position 195 à l'acide aminé en position 219). La région basique contient entre autre, 5 acides aminés lysine (K) et 4 acides aminés arginine (R). Le domaine de dimérisation contient 6 acides aminés leucine (L), 3 acides aminés alanine (A), 3 acides aminés arginine (R) et 2 acides aminés lysine (K). La région basique est plutôt chargée positivement tandis que le domaine de dimérisation est plutôt constitué d'acides aminés hydrophobes.

Les séquences d'acides nucléiques codant le peptide représenté par la séquence SEQ ID NO : 1 ou un peptide homologue tel que décrit ci-dessus peuvent être déduites à partir des séquences d'acides aminés des peptides selon le principe de la dégénérescence du code génétique connu de l'homme du métier.

Le pourcentage d'identité de séquences de peptides est déterminé par comparaison directe de deux séquences de molécules polypeptidiques, en déterminant le nombre de résidus d'acides aminés identique dans les deux séquences, puis en le divisant par le nombre de résidus d'acides aminées de la séquence la plus longue des deux, et en multipliant le résultat par 100.

On entend par l'efficacité de l'internalisation de polypeptides d'intérêt, le pourcentage de polypeptides d'intérêt internalisés dans des cellules de cible. Cette efficacité d'internalisation repose sur la détection d'un grand nombre de polypeptides d'intérêt détectés dans les cellules transduites au moyen de la microscopie à fluorescence ou par de l'analyse par la cytométrie en flux (FACS) ou par de l'analyse par western blotting après lyse des cellules.

L'efficacité de l'internalisation de polypeptides d'intérêt peut être mesurée par la cytométrie en flux ou la microscopie à fluorescence selon le protocole décrit par Rothe et Lenormand (2008).

L'utilisation d'un transporteur de molécules d'intérêt selon l'invention, représenté par la séquence SEQ ID NO : 1 (aa170-220), à la concentration inférieure à 5nM, permet d'augmenter l'efficacité de l'internalisation de polypeptides d'intérêt d'un facteur 20 par rapport à l'utilisation de la protéine ZEBRA complète, et d'un facteur 2 par rapport à l'utilisation du fragment de ZEBRA décrit par Rothe et Lenormand (2008).

Dans un mode de réalisation particulier, l'invention concerne l'utilisation :
(i) - d'un peptide représenté par la séquence d'acides aminés SEQ ID NO : 1, ou
   - d'un peptide homologue ayant 93%, notamment 95%, particulièrement 98% d'identité de séquence avec la séquence SEQ ID NO : 1, ou
(ii) d'un acide nucléique codant
   - un peptide représenté par la séquence d'acides aminés SEQ ID NO : 1, ou
   - un peptide homologue séquence ayant 93%, notamment 95%, particulièrement 98% d'identité de séquence avec la séquence SEQ ID NO : 1,
      pour l'obtention d'un transporteur destiné à l'internalisation d'un polypeptide d'intérêt diagnostique ou thérapeutique dans les cellules cibles, ledit peptide étant utilisé à une concentration de 0,01 à 5 nM, avantageusement de 0,01 à 1 nM, plus avantageusement de 0,01 à 0,3 nM, encore plus avantageusement de 0,01 à 0,2 nM, notamment de 0,01 à 0,1 nM, particulièrement de 0,01 à 0,05 nM, plus particulièrement de 0,01 à 0,03 nM, ledit polypeptide d'intérêt étant choisi parmi :
      1) la protéine eIF3-f, telle la protéine eIF3-f de souris représentée par la séquence SEQ ID NO : 19, ou la protéine eIF3-f humaine représentée par la séquence SEQ ID NO : 20, ou un polypeptide ayant 80%, notamment 90%, particulièrement 95% d'identité de séquence avec la séquence d'une protéine eIf-f, ou
      2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

Dans un autre mode de réalisation particulier, l'invention concerne l'utilisation :
(i) - d'un peptide représenté par la séquence d'acides aminés SEQ ID NO : 55, ou
   - d'un peptide homologue ayant 93%, notamment 95%, particulièrement 98% d'identité de séquence avec la séquence SEQ ID NO : 55, ou
(ii) d'un acide nucléique codant
   - un peptide représenté par la séquence d'acides aminés SEQ ID NO : 55, ou
   - un peptide homologue séquence ayant 93%, notamment 95%, particulièrement 98% d'identité de séquence avec la séquence SEQ ID NO : 55,
      pour l'obtention d'un transporteur destiné à l'internalisation d'un polypeptide d'intérêt diagnostique ou thérapeutique dans les cellules cibles, ledit peptide étant utilisé à une concentration de 0,01 à 5 nM, avantageusement de 0,01 à 1 nM, plus avantageusement de 0,01 à 0,3 nM, encore plus avantageusement de 0,01 à 0,2 nM, notamment de 0,01 à 0,1 nM, particulièrement de 0,01 à 0,05 nM, plus particulièrement de 0,01 à 0,03 nM, ledit polypeptide d'intérêt étant choisi parmi :
      1) la protéine eIF3-f, telle la protéine eIF3-f de souris représentée par la séquence SEQ ID NO : 19, ou la protéine eIf3f humaine représentée par la séquence SEQ ID NO : 20, ou un polypeptide ayant 80%, notamment 90%, particulièrement 95% d'identité de séquence avec la séquence d'une protéine eIF3-f, ou
      2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

La séquence SEQ ID NO : 55 correspond au segment de la protéine ZEBRA allant de l'acide aminé en position 140 à l'acide aminé en position 245.

Dans un autre mode de réalisation particulier, l'invention concerne l'utilisation :
(i) - d'un peptide représenté par la séquence d'acides aminés SEQ ID NO : 56, ou
   - d'un peptide homologue ayant 93%, notamment 95%, particulièrement 98% d'identité de séquence avec la séquence SEQ ID NO : 56, ou
(ii) d'un acide nucléique codant
   - un peptide représenté par la séquence d'acides aminés SEQ ID NO : 56, ou
   - un peptide homologue séquence ayant 93%, notamment 95%, particulièrement 98% d'identité de séquence avec la séquence SEQ ID NO : 56,
      pour l'obtention d'un transporteur destiné à l'internalisation d'un polypeptide d'intérêt diagnostique ou thérapeutique dans les cellules cibles, ledit peptide étant utilisé à une concentration de 0,01 à 5 nM, avantageusement de 0,01 à 1 nM, plus avantageusement de 0,01 à 0,3 nM, encore plus avantageusement de 0,01 à 0,2 nM, notamment de 0,01 à 0,1 nM, particulièrement de 0,01 à 0,05 nM, plus particulièrement de 0,01 à 0,03 nM, ledit polypeptide d'intérêt étant choisi parmi :
      1) la protéine eIF3-f, telle la protéine eIF3-f de souris représentée par la séquence SEQ ID NO : 19, ou la protéine eIf3f humaine représentée par la séquence SEQ ID NO : 20, ou un polypeptide ayant 80%, notamment 90%, particulièrement 95% d'identité de séquence avec la séquence d'une protéine eIF3-f, ou
      2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

La séquence SEQ ID NO : 56 correspond au segment de la protéine ZEBRA allant de l'acide aminé en position 170 à l'acide aminé en position 245.

Un polypeptide d'intérêt peut être lié à un transporteur selon l'invention par une liaison covalente ou non covalente, telle qu'une liaison ionique, une liaison hydrogène, ou une liaison hydrophobique.

En particulier, le polypeptide d'intérêt peut être aussi lié à un transporteur par un linker biologique conventionnel, tel que GSGG, ou un agent de réticulation conventionnel, tel que SMCC (succinimidyl 4-(N-maleimidomethyl)cyclohexane-1-carboxylate), SPDP (N-succinimidyl 3-(2-pyridyldithio)propionate).

Selon l'invention, un polypeptide d'intérêt peut être lié à l'extrémité N-terminale ou C-terminale d'un transporteur, sous réserve que les propriétés biologiques dudit polypeptide d'intérêt ne soient pas modifiées.

Dans un mode de réalisation avantageux de l'invention, ledit polypeptide d'intérêt peut être choisi parmi :
(1) la protéine eIF3-f, telle la protéine eIF3-f de souris représentée par la séquence SEQ ID NO : 19, ou la protéine eIF3-f humaine représentée par la séquence SEQ ID NO : 20, ou un polypeptide ayant 80%, notamment 90%, particulièrement 95% d'identité de séquence avec la séquence d'une protéine eIF3-f, ou
(2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

Les polypeptides d'intérêt susceptibles d'être internalisés par le transporteur selon l'invention peuvent aussi être :
(1) la protéine SPEEDY, telle que la protéine SPEEDY de Xenopus représentée par la séquence SEQ ID NO : 2, ou
(2) le domaine de liaison avec cdk (cyclines dépendante des kinases) d'une protéine SPEEDY, tel que le domaine de liaison avec cdk de la protéine SPEEDY humaine, représenté par la séquence SEQ ID NO : 3, le domaine de liaison avec cdk de la protéine SPEEDY de souris, représenté par la séquence SEQ ID NO : 4, le domaine de liaison avec cdk d'une protéine SPEEDY de Xenopus, représenté par la séquence SEQ ID NO : 5, le domaine de liaison avec cdk d'une protéine SPEEDY de Xenopus, représenté par la séquence SEQ ID NO : 6, le domaine de liaison avec cdk d'une protéine SPEEDY de la drosophile, représenté par la séquence SEQ ID NO : 7, ou
(3) un peptide ayant 80%, notamment 90%, particulièrement 95% d'identité de séquence avec la séquence du domaine de liaison cdk d'une protéine SPEEDY, et conservant la séquence consensus d'une protéine SPEEDY, représentée par la séquence SEQ ID NO : 8.
(4) la protéine Cyclin E1, telle que la protéine Cyclin E1 de rat, représentée par la séquence SEQ ID NO : 9, ou
(5) le domaine de liaison avec CLS (Centrosomal Localization signal) d'une protéine Cyclin E1, tel que le domaine de liaison avec CLS de la protéine Cyclin E1 de rat, représenté par la séquence SEQ ID NO : 10, le domaine de liaison avec CLS d'une protéine Cyclin E1 de souris, représenté par la séquence SEQ ID NO : 11 ou SEQ ID NO : 12, le domaine de liaison avec CLS d'une protéine Cyclin E1 humaine, représenté par la séquence SEQ ID NO : 13 ou SEQ ID NO :14, ou
(6) un peptide ayant 80%, notamment 90%, particulièrement 95% d'identité de séquence avec la séquence du domaine de liaison avec CLS d'une protéine Cyclin E1.
(7) la protéine tristetraprolin (TTp), telle la protéine TTp de souris représentée par la séquence SEQ ID NO : 15, ou la protéine TTp humaine représentée par la séquence SEQ ID NO : 16, ou
(8) un polypeptide ayant 80%, notamment 90%, particulièrement 95% d'identité de séquence avec la séquence d'une protéine TTp.
(9) la protéine Atrogin ou MAFbx, telle la protéine Atrogin humaine représentée par la séquence SEQ ID NO : 17, ou la protéine F-box 32 de souris, représentée par la séquence SEQ ID NO : 18, ou
(10) un polypeptide ayant 80%, notamment 90%, particulièrement 95% d'identité de séquence avec la séquence d'une protéine Atrogin ou MAFbx.
(11) la protéine Mad-7 ou IL-24, telle la protéine Mad-7 humaine représentée par la séquence SEQ ID NO : 21, ou la protéine Mad-7 de souris représentée par la séquence SEQ ID NO : 22, ou une variante de la protéine Mad-7 humaine (IL-24), représentée par la séquence SEQ ID NO : 23, ou
(12) un polypeptide ayant 80%, notamment 90%, particulièrement 95% d'identité de séquence avec la séquence d'une protéine Mad-7.
(13) la protéine Vascular endothelial-cadherin, telle que la protéine Vascular endothelial-cadherin humaine représentée par la séquence SEQ ID NO : 24, ou
(14) le peptide issu de Vascular endothelial-cadherin, représenté par la séquence SEQ ID NO : 25, dans lequel la tyrosine Y685 est phosphorylée par la src kinase,
(15) la protéine TP53, telle que la protéine TP 53 humaine, représentée par la séquence SEQ ID NO : 26,
(16) la protéine PADRE-OVA, représentée par la séquence SEQ ID NO : 28.

La protéine PADRE (Pan-DR-epitope) est un épitope CD4(+) T helper, qui permet une réponse immunitaire de l'antigène associé (içi OVA) spécifique CD8(+) T-cellule chez les souris vaccinées.

Un susdit polypeptide d'intérêt peut être conjugué au transporteur selon l'invention par une liaison peptidique directe, ou par un linker biologique.

Un susdit polypeptide d'intérêt peut être aussi conjugué au transporteur selon l'invention par un agent de réticulation sous réserve que les propriétés biologiques du transporteur et celles du polypeptide d'intérêt ne soient pas modifiées.

Dans un mode de réalisation avantageux de l'invention, le polypeptide d'intérêt est lié au transporteur selon l'invention par une liaison peptidique directe.

Le transporteur selon l'invention peut être aussi lié à d'autres types de molécules d'intérêt, telles que ADN, ARN, oligonucléotides, siARN, miARN, ARN antisens, ou des acides nucléiques peptidiques (ANP).

Les acides nucléiques conjugués au transporteur destiné à l'internalisation peuvent être utilisés comme sonde diagnostique, ou comme un agent thérapeutique. Par exemple, un siARN, un miARN ou un ARN antisens peut hybrider à un gène cible dont l'expression chez un patient est à modifier.

Par ailleurs, une molécule d'intérêt susceptible d'être internalisée par le transporteur selon l'invention peut être un analogue de nucléoside, par exemple, la Didanosine, la Vidarabine, la Cytarabine, l'Entricitabine, la Lamivudine, la Zalcitabine, l'Abacavir, la Stavudine, la Zidovudine.

Les cellules susceptibles d'être les cellules cibles d'un processus d'internalisation mis en oeuvre par un transporteur selon l'invention sont choisies parmi des cellules eucaryotes, notamment des cellules humaines. Ces cellules humaines peuvent être des cellules tumorales, telles que des cellules de mélanomes, des cellules de cancer du sein, des cellules de glioblastomes, des cellules de cancer du colon, des cellules de lymphomes. Ces cellules humaines peuvent être également des cellules normales incluant des fibroblastes, cellules épithéliales, lymphocytes, cellules dendritiques, cellules différenciées (cellules musculaires comme les myotubes par exemple). Pour cibler certaines lignées de cellules, des séquences peptidiques comme des homing peptides, des NLS (nuclear localisation signal), peuvent être greffées sur le transporteur selon l'invention.

Dans un mode de réalisation avantageux, l'invention porte sur l'utilisation du peptide représenté par la séquence SEQ ID NO : 1, pour l'obtention d'un transporteur destiné à l'internalisation d'un polypeptide d'intérêt diagnostique ou thérapeutique, telle que décrit ci-dessus, dans les cellules cibles, ledit peptide étant utilisé à une concentration inférieure à 5 nM, ledit polypeptide d'intérêt étant choisi parmi :
1) la protéine eIF3-f, telle la protéine eIF3-f de souris représentée par la séquence SEQ ID NO : 19, ou la protéine eIf3f humaine représentée par la séquence SEQ ID NO : 20, ou un polypeptide ayant 80%, notamment 90%, particulièrement 95% d'identité de séquence avec la séquence d'une protéine eIF3-f, ou
2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

Plus particulièrement, l'invention porte sur l'utilisation du peptide représenté par la séquence SEQ ID NO : 1, ou la séquence SEQ ID NO : 55, ou la séquence SEQ ID NO : 56, pour l'obtention d'un transporteur destiné à l'internalisation d'un polypeptide d'intérêt dans les cellules cibles, ledit transporteur étant utilisé à une concentration de 0,01 à 5 nM, avantageusement de 0,01 à 1 nM, plus avantageusement de 0,01 à 0,3 nM, encore plus avantageusement de 0,01 à 0,2 nM, notamment de 0,01 à 0,1 nM, particulièrement de 0,41 à 0,05 nM, plus particulièrement de 0,01 à 0,03 nM, et ledit polypeptide d'intérêt étant choisi parmi :
1) la protéine eIF3-f, telle la protéine eIF3-f de souris représentée par la séquence SEQ ID NO : 19, ou la protéine eIF3-f humaine représentée par la séquence SEQ ID NO : 20, ou un polypeptide ayant 80%, notamment 90%, particulièrement 95% d'identité de séquence avec la séquence d'une protéine eIF3-f, ou
2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

La présente invention concerne aussi une combinaison comprenant :
- un polypeptide d'intérêt diagnostic ou thérapeutique, et
- un transporteur, utilisé à une concentration inférieure à 5 nM, avantageusement inférieure à 1 nM, plus avantageusement inférieure à 0,3 nM, encore plus avantageusement inférieure à 0,2nM, notamment inférieure à 0,1nM, particulièrement inférieure à 0,05nM, plus particulièrement inférieure à 0,03nM, notamment une concentration de 0,01 à 5 nM, avantageusement de 0,01 à 1 nM, plus avantageusement de 0,01 à 0,3 nM, encore plus avantageusement de 0,01 à 0,2 nM, notamment de 0,01 à 0,1 nM, particulièrement de 0,01 à 0,05 nM, plus particulièrement de 0,01 à 0,03 nM, destiné à l'internalisation dudit polypeptide d'intérêt à des cellules cibles,
   ledit transporteur étant un peptide comprenant la séquence d'acides aminés SEQ ID NO : 1, ou un peptide comprenant une séquence d'acides aminés ayant 93%, notamment 95%, particulièrement 98% d'homologie d'identité de séquence avec la séquence SEQ ID NO : 1, ledit polypeptide d'intérêt étant choisi parmi :
   1) la protéine eIF3-f, telle la protéine eIF3-f de souris représentée par la séquence SEQ ID NO : 19, ou la protéine eIF3-f humaine représentée par la séquence SEQ ID NO : 20, ou un polypeptide ayant 80%, notamment 90%, particulièrement 95% d'identité de séquence avec la séquence d'une protéine eIF3-f, ou
   2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

Dans un autre mode de réalisation particulier de l'invention, ledit transporteur est un peptide comprenant la séquence d'acides aminés SEQ ID NO : 55 , ou un peptide comprenant une séquence d'acides aminés ayant 93%, notamment 95%, particulièrement 98% d'homologie d'identité de séquence avec la séquence SEQ ID NO : 55.

Dans un autre mode de réalisation particulier de l'invention, ledit transporteur est un peptide comprenant la séquence d'acides aminés SEQ ID NO : 56, ou un peptide comprenant une séquence d'acides aminés ayant 93%, notamment 95%, particulièrement 98% d'homologie d'identité de séquence avec la séquence SEQ ID NO : 56.

Par l'expression « combinaison », on entend qu'au moins une molécule d'intérêt est liée à un transporteur tel que décrit ci-dessus, par tout moyen permettant une interaction physique entre le transporteur et le polypeptide d'intérêt. Ce moyen d'interaction peut être une liaison covalente ou non covalente, telle qu'une liaison ionique, une liaison hydrogène, ou une liaison hydrophobique.

La molécule d'intérêt susceptible d'être combinée avec le transporteur selon l'invention peut également être les polypeptides, les analogues de nucléosides, les acides nucléiques, telle que décrite ci-dessus.

Un polypeptide susceptible d'être combiné avec le transporteur selon l'invention peut également être une enzyme, un anticorps, un antigène, une toxine, un immunomodulateur, ou un fragment fonctionnel desdits polypeptides, par exemple
1) la protéine SPEEDY, telle que la protéine SPEEDY de Xenopus représentée par la séquence SEQ ID NO : 2, ou
2) le domaine de liaison avec cdk (cyclines dépendante des kinases) d'une protéine SPEEDY, tel que le domaine de liaison avec cdk de la protéine SPEEDY humaine, représenté par la séquence SEQ ID NO : 3, le domaine de liaison avec cdk de la protéine SPEEDY de souris, représenté par la séquence SEQ ID NO : 4, le domaine de liaison avec cdk d'une protéine SPEEDY de Xenopus, représenté par la séquence SEQ ID NO : 5, le domaine de liaison avec cdk d'une protéine SPEEDY de Xenopus, représenté par la séquence SEQ ID NO : 6, le domaine de liaison avec cdk d'une protéine SPEEDY de la drosophile, représenté par la séquence SEQ ID NO : 7, ou
3) un peptide ayant 80%, notamment 90%, particulièrement 95% d'identité de séquence avec la séquence du domaine de liaison cdk d'une protéine SPEEDY, et conservant la séquence consensus d'une protéine SPEEDY, représentée par la séquence SEQ ID NO : 8,
4) la protéine Cyclin E1, telle que la protéine Cyclin E1 de rat, représentée par la séquence SEQ ID NO : 9, ou
5) le domaine de liaison avec CLS (Centrosomal Localization signal) d'une protéine Cyclin E1, tel que le domaine de liaison avec CLS de la protéine Cyclin E1de rat, représenté par la séquence SEQ ID NO : 10, le domaine de liaison avec CLS d'une protéine Cyclin E1 de souris, représenté par la séquence SEQ ID NO : 11 ou SEQ ID NO : 12, le domaine de liaison avec CLS d'une protéine Cyclin E1 humaine, représenté par la séquence SEQ ID NO : 13 ou SEQ ID NO :14, ou
6) un peptide ayant 80%, notamment 90%, particulièrement 95% d'identité de séquence avec la séquence du domaine de liaison avec CLS d'une protéine Cyclin E1,
7) la protéine tristetraprolin (TTp), telle la protéine TTp de souris représentée par la séquence SEQ ID NO : 15, ou la protéine TTp humaine représentée par la séquence SEQ ID NO : 16, ou
8) un polypeptide ayant 80%, notamment 90%, particulièrement 95% d'identité de séquence avec la séquence d'une protéine TTp,
9) la protéine Atrogin ou MAFbx, telle la protéine Atrogin humaine représentée par la séquence SEQ ID NO : 17, ou la protéine F-box 32 de souris, représentée par la séquence SEQ ID NO : 18, ou
10) un polypeptide ayant 80%, notamment 90%, particulièrement 95% d'identité de séquence avec la séquence d'une protéine Atrogin ou MAFbx,
11) la protéine Mad-7 ou IL-24, telle la protéine Mad-7 humaine représentée par la séquence SEQ ID NO : 21, ou la protéine Mad-7 de souris représentée par la séquence SEQ ID NO : 22, ou une variante de la protéine Mad-7 humaine (IL-24), représentée par la séquence SEQ ID NO : 23, ou
12) un polypeptide ayant 80%, notamment 90%, particulièrement 95% d'identité de séquence avec la séquence d'une protéine Mad-7,
13) la protéine Vascular endothelial-cadherin, telle que la protéine Vascular endothelial-cadherin humaine représentée par la séquence SEQ ID NO : 24, ou
14) le peptide issu de Vascular endothelial-cadherin, représenté par la séquence SEQ ID NO : 25, dans lequel la tyrosine Y685 est phosphorylée par la src kinase,
15) la protéine TP53, telle que la protéine TP 53 humaine, représentée par la séquence SEQ ID NO : 26,
16) la protéine PADRE-OVA, représentée par la séquence SEQ ID NO : 28.

La présente invention concerne également un peptide de fusion comprenant un polypeptide d'intérêt diagnostique ou thérapeutique et un transporteur destiné à l'internalisation dudit polypeptide d'intérêt dans les cellules cibles, ledit transporteur étant
- un peptide comprenant la séquence d'acides aminés SEQ ID NO : 1, ou
- un peptide comprenant une séquence d'acides aminés ayant 93%, notamment 95%, particulièrement 98% d'homologie d'identité de séquence avec la séquence SEQ ID NO : 1, ledit polypeptide d'intérêt étant choisi parmi :
   1) la protéine eIF3-f, telle la protéine eIF3-f de souris représentée par la séquence SEQ ID NO : 19, ou la protéine eIF3-f humaine représentée par la séquence SEQ ID NO : 20, ou un polypeptide ayant 80%, notamment 90%, particulièrement 95% d'identité de séquence avec la séquence d'une protéine eIF3-f, ou
   2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

Dans un autre mode de réalisation particulier de l'invention, ledit transporteur est un peptide comprenant la séquence d'acides aminés SEQ ID NO : 55, ou un peptide comprenant une séquence d'acides aminés ayant 93%, notamment 95%, particulièrement 98% d'homologie d'identité de séquence avec la séquence SEQ ID NO : 55.

Dans un autre mode de réalisation particulier de l'invention, ledit transporteur est un peptide comprenant la séquence d'acides aminés SEQ ID NO : 56, ou un peptide comprenant une séquence d'acides aminés ayant 93%, notamment 95%, particulièrement 98% d'homologie d'identité de séquence avec la séquence SEQ ID NO : 56.

Par « peptide de fusion », on entend un peptide recombinant ou synthétique contenant au moins deux peptides, issus de deux protéines différentes, l'un lié à l'autre directement par liaison peptidique, ou par un linker peptidique, tel que GSGG.

Le polypeptide d'intérêt peut être lié à l'extrémité N-terminale ou C-terminale du transporteur, sous réserve que la propriété biologique du polypeptide d'intérêt ne soit pas modifiée.

Dans un mode de réalisation particulier, dans le peptide de fusion selon l'invention, le polypeptide d'intérêt est fusionné à l'extrémité N-terminale du transporteur destiné à l'internalisation dudit polypeptide.

Dans un autre mode de réalisation particulier, dans le peptide de fusion selon l'invention, le polypeptide d'intérêt est fusionné à l'extrémité C-terminale du transporteur destiné à l'internalisation dudit polypeptide.

Un polypeptide d'intérêt susceptible d'être compris dans un peptide de fusion selon l'invention peut également être:
1) la protéine SPEEDY, telle que la protéine SPEEDY de Xenopus représentée par la séquence SEQ ID NO : 2, ou
2) le domaine de liaison avec cdk (cyclines dépendante des kinases) d'une protéine SPEEDY, tel que le domaine de liaison avec cdk de la protéine SPEEDY humaine, représenté par la séquence SEQ ID NO : 3, le domaine de liaison avec cdk de la protéine SPEEDY de souris, représenté par la séquence SEQ ID NO : 4, le domaine de liaison avec cdk d'une protéine SPEEDY de Xenopus, représenté par la séquence SEQ ID NO : 5, le domaine de liaison avec cdk d'une protéine SPEEDY de Xenopus, représenté par la séquence SEQ ID NO : 6, le domaine de liaison avec cdk d'une protéine SPEEDY de la drosophile, représenté par la séquence SEQ ID NO : 7, ou
3) un peptide ayant 80%, notamment 90%, particulièrement 95% d'identité de séquence avec la séquence du domaine de liaison cdk d'une protéine SPEEDY, et conservant la séquence consensus d'une protéine SPEEDY, représentée par la séquence SEQ ID NO : 8,
4) la protéine Cyclin E1, telle que la protéine Cyclin E1 de rat, représentée par la séquence SEQ ID NO : 9, ou
5) le domaine de liaison avec CLS (Centrosomal Localization signal) d'une protéine Cyclin E1, tel que le domaine de liaison avec CLS de la protéine Cyclin E1 de rat, représenté par la séquence SEQ ID NO : 10, le domaine de liaison avec CLS d'une protéine Cyclin E1 de souris, représenté par la séquence SEQ ID NO : 11 ou SEQ ID NO : 12, le domaine de liaison avec CLS d'une protéine Cyclin E1 humaine, représenté par la séquence SEQ ID NO : 13 ou SEQ ID NO :14, ou
6) un peptide ayant 80%, notamment 90%, particulièrement 95% d'identité de séquence avec la séquence du domaine de liaison avec CLS d'une protéine Cyclin E1,
7) la protéine tristetraprolin (TTp), telle la protéine TTp de souris représentée par la séquence SEQ ID NO : 15, ou la protéine TTp humaine représentée par la séquence SEQ ID NO : 16, ou
8) un polypeptide ayant 80%, notamment 90%, particulièrement 95% d'identité de séquence avec la séquence d'une protéine TTp,
9) la protéine Atrogin ou MAFbx, telle la protéine Atrogin humaine représentée par la séquence SEQ ID NO : 17, ou la protéine F-box 32 de souris, représentée par la séquence SEQ ID NO: 18, ou
10) un polypeptide ayant 80%, notamment 90%, particulièrement 95% d'identité de séquence avec la séquence d'une protéine Atrogin ou MAFbx,
11) la protéine Mad-7 ou IL-24, telle la protéine Mad-7 humaine représentée par la séquence SEQ ID NO : 21, ou la protéine Mad-7 de souris représentée par la séquence SEQ ID NO : 22, ou une variante de la protéine Mad-7 humaine (IL-24), représentée par la séquence SEQ ID NO : 23, ou
12) un polypeptide ayant 80%, notamment 90%, particulièrement 95% d'identité de séquence avec la séquence d'une protéine Mad-7,
13) la protéine Vascular endothelial-cadherin, telle que la protéine Vascular endothelial-cadherin humaine représentée par la séquence SEQ ID NO : 24, ou
14) le peptide issu de Vascular endothelial-cadherin, représenté par la séquence SEQ ID NO : 25, dans lequel la tyrosine Y685 est phosphorylée par la src kinase,
15) la protéine TP53, telle que la protéine TP 53 humaine, représentée par la séquence SEQ ID NO : 26,
16) la protéine PADRE-OVA, représentée par la séquence SEQ ID NO : 28.

Dans un mode de réalisation plus avantageux de l'invention, un peptide de fusion comprend le transporteur représenté par la séquence SEQ ID NO : 1.

Dans un autre mode de réalisation plus avantageux de l'invention, un peptide de fusion comprend le transporteur représenté par la séquence SEQ ID NO : 55.

Dans un autre mode de réalisation plus avantageux de l'invention, un peptide de fusion comprend le transporteur représenté par la séquence SEQ ID NO : 56.

Dans un mode de réalisation plus avantageux de l'invention, l'un des susdits polypeptides d'intérêt est liés directement par liaison peptidique à l'extrémité N-terminale du transporteur selon l'invention, représenté par la séquence SEQ ID NO : 1, ou la séquence SEQ ID NO : 55, ou la séquence SEQ ID NO : 56.

Dans un mode de réalisation particulièrement avantageux, le peptide de fusion selon l'invention est choisi parmi :
1) le peptide de fusion représenté par la séquence SEQ ID NO : 48, dans lequel le polypeptide d'intérêt, à savoir la protéine eIF-3f humaine représentée par la séquence SEQ ID NO : 20, est lié directement à l'extrémité N-terminale du transporteur représenté par la séquence SEQ ID NO : 1, ou
2) le peptide de fusion représenté par la séquence SEQ ID NO : 40, dans lequel le polypeptide d'intérêt, à savoir la protéine FERM humaine représentée par la séquence SEQ ID NO : 27, est lié directement à l'extrémité N-terminale du transporteur représenté par la séquence SEQ ID NO : 1.

Un peptide de fusion, dans lequel un polypeptide d'intérêt est lié directement par liaison peptidique à l'extrémité N-terminale du transporteur représenté par la séquence SEQ ID NO : 1, peut également être :
1) le peptide de fusion représenté par la séquence SEQ ID NO : 42, dans lequel le polypeptide d'intérêt est la protéine SPEEDY de Xenopus représentée par la séquence SEQ ID NO : 2, ou
2) le peptide de fusion représenté par la séquence SEQ ID NO : 43, dans lequel le polypeptide d'intérêt est le domaine de liaison avec cdk (cyclines dépendante des kinases) de la protéine SPEEDY humaine, représenté par la séquence SEQ ID NO : 3, ou
3) le peptide de fusion représenté par la séquence SEQ ID NO : 44, dans lequel le polypeptide d'intérêt est la protéine Cyclin E1 de rat, représentée par la séquence SEQ ID NO : 9, ou
4) le peptide de fusion représenté par la séquence SEQ ID NO : 45, dans lequel le polypeptide d'intérêt est le domaine de liaison avec CLS (Centrosomal Localization signal) d'une protéine Cyclin E1 humaine, représenté par la séquence SEQ ID NO : 13, ou
5) le peptide de fusion représenté par la séquence SEQ ID NO : 46, dans lequel le polypeptide d'intérêt est la protéine tristetraprolin (TTP) humaine représentée par la séquence SEQ ID NO : 16, ou
6) le peptide de fusion représenté par la séquence SEQ ID NO : 47, dans lequel le polypeptide d'intérêt est la protéine Atrogin humaine représentée par la séquence SEQ ID NO : 17, ou
7) le peptide de fusion représenté par la séquence SEQ ID NO : 49, dans lequel le polypeptide d'intérêt est la protéine Mad-7 (IL24) humaine représentée par la séquence SEQ ID NO : 21, ou
8) le peptide de fusion représenté par la séquence SEQ ID NO : 50, dans lequel le polypeptide d'intérêt est la protéine Vascular endothelial-cadherin humaine représentée par la séquence SEQ ID NO : 24, ou
9) le peptide de fusion représenté par la séquence SEQ ID NO : 51, dans lequel le polypeptide d'intérêt est le peptide issu de Vascular endothelial-cadherin, représenté par la séquence SEQ ID NO : 25, dans lequel la tyrosine Y685 est phosphorylée par la src kinase, ou
10) le peptide de fusion représenté par la séquence SEQ ID NO : 52, dans lequel le polypeptide d'intérêt est la protéine TP53 humaine, représentée par la séquence SEQ ID NO : 26,
11) le peptide de fusion représenté par la séquence SEQ ID NO : 54, dans lequel le polypeptide d'intérêt est la protéine PADRE-OVA représentée par la séquence SEQ ID NO : 28.

Dans un autre mode de réalisation plus avantageux de l'invention, l'un des susdits polypeptides d'intérêt est lié directement à l'extrémité C-terminale du transporteur selon l'invention, représenté par la séquence SEQ ID NO : 1, ou la séquence SEQ ID NO : 55, ou la séquence SEQ ID NO : 56.

Dans un mode de réalisation particulièrement avantageux, le peptide de fusion selon l'invention est choisi parmi :
1) le peptide de fusion représenté par la séquence SEQ ID NO : 35, dans lequel le polypeptide d'intérêt, à savoir la protéine eIF-3f humaine représentée par la séquence SEQ ID NO : 20, est lié directement à l'extrémité C-terminale du transporteur représenté par la séquence SEQ ID NO : 1, ou
2) le peptide de fusion représenté par la séquence SEQ ID NO : 53, dans lequel le polypeptide d'intérêt, à savoir la protéine FERM humaine représentée par la séquence SEQ ID NO : 27, est lié directement à l'extrémité C-terminale du transporteur représenté par la séquence SEQ ID NO : 1.

Un peptide de fusion, dans lequel un polypeptide d'intérêt est lié directement par liaison peptidique à l'extrémité C-terminale du transporteur représenté par la séquence SEQ ID NO : 1, peut également être :
1) le peptide de fusion représenté par la séquence SEQ ID NO : 29, dans lequel le polypeptide d'intérêt est la protéine SPEEDY de Xenopus représentée par la séquence SEQ ID NO : 2, ou
2) le peptide de fusion représenté par la séquence SEQ ID NO : 30, dans lequel le polypeptide d'intérêt est le domaine de liaison avec cdk (cyclines dépendante des kinases) de la protéine SPEEDY humaine, représenté par la séquence SEQ ID NO : 3, ou
3) le peptide de fusion représenté par la séquence SEQ ID NO : 31, dans lequel le polypeptide d'intérêt est la protéine Cyclin E1 de rat, représentée par la séquence SEQ ID NO : 9, ou
4) le peptide de fusion représenté par la séquence SEQ ID NO : 32, dans lequel le polypeptide d'intérêt est le domaine de liaison avec CLS (Centrosoma1 Localization signal) d'une protéine Cyclin E1 humaine, représenté par la séquence SEQ ID NO : 13, ou
5) le peptide de fusion représenté par la séquence SEQ ID NO : 33, dans lequel le polypeptide d'intérêt est la protéine tristetraprolin (TTP) humaine représentée par la séquence SEQ ID NO : 16, ou
6) le peptide de fusion représenté par la séquence SEQ ID NO : 34, dans lequel le polypeptide d'intérêt est la protéine Atrogin humaine représentée par la séquence SEQ ID NO : 17, ou
7) le peptide de fusion représenté par la séquence SEQ ID NO : 36, dans lequel le polypeptide d'intérêt est la protéine Mad-7 (IL24) humaine représentée par la séquence SEQ ID NO : 21, ou
8) le peptide de fusion représenté par la séquence SEQ ID NO : 37, dans lequel le polypeptide d'intérêt est la protéine Vascular endothelial-cadherin humaine représentée par la séquence SEQ ID NO : 24, ou
9) le peptide de fusion représenté par la séquence SEQ ID NO : 38, dans lequel le polypeptide d'intérêt est le peptide issu de Vascular endothelial-cadherin, représenté par la séquence SEQ ID NO : 25, dans lequel la tyrosine Y685 est phosphorylée par la src kinase, ou
10) le peptide de fusion représenté par la séquence SEQ ID NO : 39, dans lequel le polypeptide d'intérêt est la protéine TP53 humaine, représentée par la séquence SEQ ID NO : 26,
11) le peptide de fusion représenté par la séquence SEQ ID NO : 41, dans lequel le polypeptide d'intérêt est la protéine PADRE-OVA représentée par la séquence SEQ ID NO : 28.

La présente invention concerne aussi les acides nucléiques codant un peptide de fusion tel que décrit ci-dessus.

La présente invention concerne aussi des vecteurs comprenant un acide nucléique codant un peptide de fusion tel que décrit ci-dessus.

Dans un mode de réalisation particulier, le vecteur selon l'invention comprend en outre les moyens génétiques, en particulier les origines de réplication, les promoteurs, permettant de contrôler l'expression des susdites protéines de fusion.

La présente invention a également pour objet des cellules hôtes comprenant un vecteur d'expression. Lesdites cellules hôtes peuvent être des cellules procaryotes, telles que *E.coli,* les *basillus,* notamment *basillus brevis,* ou des cellules eucaryotes, telles que les levures, les champignons filamenteux, notamment *Trichoderma reesei et Aspergillus niger,* les cellules d'insecte en utilisant les Baculovirus, ou des lignées cellulaire, telles que CHO, HEK 293, ou Cos.

La présente invention a aussi pour objet une composition pharmaceutique comprenant un polypeptide d'intérêt et un transporteur de la molécule d'intérêt, ledit transporteur étant
- un peptide comprenant la séquence d'acides aminés SEQ ID NO : 1, ou
- un peptide comprenant une séquence d'acides aminés ayant 93%, notamment 95%, particulièrement 98% d'homologie d'identité de séquence avec la séquence SEQ ID NO : 1.
   en association avec un excipient et/ou un véhicule pharmaceutiquement acceptable, ledit polypeptide d'intérêt étant choisi parmi :
   1) la protéine eIF3-f, telle la protéine eIF3-f de souris représentée par la séquence SEQ ID NO : 19, ou la protéine eIF3-f humaine représentée par la séquence SEQ ID NO : 20, ou un polypeptide ayant 80%, notamment 90%, particulièrement 95% d'identité de séquence avec la séquence d'une protéine eIF3-f, ou
   2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

Dans un autre mode de réalisation particulier de l'invention, ledit transporteur est un peptide comprenant la séquence d'acides aminés SEQ ID NO : 55, ou un peptide comprenant une séquence d'acides aminés ayant 93%, notamment 95%, particulièrement 98% d'homologie d'identité de séquence avec la séquence SEQ ID NO : 55.

Dans un autre mode de réalisation particulier de l'invention, ledit transporteur est un peptide comprenant la séquence d'acides aminés SEQ ID NO : 56, ou un peptide comprenant une séquence d'acides aminés ayant 93%, notamment 95%, particulièrement 98% d'homologie d'identité de séquence avec la séquence SEQ ID NO : 56.

Le choix d'un véhicule pharmaceutiquement acceptable est connu de l'homme du métier.

Dans un mode de réalisation avantageux d'une composition pharmaceutique, la molécule d'intérêt peut être choisie parmi les peptides, les analogues de nucléosides, ou les acides nucléiques.

Un transporteur selon l'invention peut être également compris dans une composition pharmaceutique, qui comprend en outre un polypeptide d'intérêt, tel que :
1) la protéine SPEEDY, telle que la protéine SPEEDY de Xenopus représentée par la séquence SEQ ID NO : 2, ou
2) le domaine de liaison avec cdk (cyclines dépendante des kinases) d'une protéine SPEEDY, tel que le domaine de liaison avec cdk de la protéine SPEEDY humaine, représenté par la séquence SEQ ID NO : 3, le domaine de liaison avec cdk de la protéine SPEEDY de souris, représenté par la séquence SEQ ID NO : 4, le domaine de liaison avec cdk d'une protéine SPEEDY de Xenopus, représenté par la séquence SEQ ID NO : 5, le domaine de liaison avec cdk d'une protéine SPEEDY de Xenopus, représenté par la séquence SEQ ID NO : 6, le domaine de liaison avec cdk d'une protéine SPEEDY de la drosophile, représenté par la séquence SEQ ID NO : 7, ou
3) un peptide ayant 80%, notamment 90%, particulièrement 95% d'identité de séquence avec la séquence du domaine de liaison cdk d'une protéine SPEEDY, et conservant la séquence consensus d'une protéine SPEEDY, représentée par la séquence SEQ ID NO : 8,
4) la protéine Cyclin E1, telle que la protéine Cyclin E1 de rat, représentée par la séquence SEQ ID NO : 9, ou
5) le domaine de liaison avec CLS (Centrosomal Localization signal) d'une protéine Cyclin E1, tel que le domaine de liaison avec CLS de la protéine Cyclin E1 de rat, représenté par la séquence SEQ ID NO : 10, le domaine de liaison avec CLS d'une protéine Cyclin E1 de souris, représenté par la séquence SEQ ID NO : 11 ou SEQ ID NO : 12, le domaine de liaison avec CLS d'une protéine Cyclin E1 humaine, représenté par la séquence SEQ ID NO : 13 ou SEQ ID NO :14, ou
6) un peptide ayant 80%, notamment 90%, particulièrement 95% d'identité de séquence avec la séquence du domaine de liaison avec CLS d'une protéine Cyclin E1,
7) la protéine tristetraprolin (TTp), telle la protéine TTp de souris représentée par la séquence SEQ ID NO : 15, ou la protéine TTp humaine représentée par la séquence SEQ ID NO : 16, ou
8) un polypeptide ayant 80%, notamment 90%, particulièrement 95% d'identité de séquence avec la séquence d'une protéine TTp,
9) la protéine Atrogin ou MAFbx, telle la protéine Atrogin humaine représentée par la séquence SEQ ID NO : 17, ou la protéine F-box 32 de souris, représentée par la séquence SEQ ID NO : 18, ou
10) un polypeptide ayant 80%, notamment 90%, particulièrement 95% d'identité de séquence avec la séquence d'une protéine Atrogin ou MAFbx,
11) la protéine Mad-7 ou IL-24, telle la protéine Mad-7 humaine représentée par la séquence SEQ ID NO : 21, ou la protéine Mad-7 de souris représentée par la séquence SEQ ID NO : 22, ou une variante de la protéine Mad-7 humaine (IL-24), représentée par la séquence SEQ ID NO : 23, ou
12) un polypeptide ayant 80%, notamment 90%, particulièrement 95% d'identité de séquence avec la séquence d'une protéine Mad-7,
13) la protéine Vascular endothelial-cadherin, telle que la protéine Vascular endothelial-cadherin humaine représentée par la séquence SEQ ID NO : 24, ou
14) le peptide issu de Vascular endothelial-cadherin, représenté par la séquence SEQ ID NO : 25, dans lequel la tyrosine Y685 est phosphorylée par la src kinase,
15) la protéine TP53, telle que la protéine TP 53 humaine, représentée par la séquence SEQ ID NO : 26,
16) la protéine PADRE-OVA, représentée par la séquence SEQ ID NO : 28.

Dans un mode de réalisation avantageux, la composition pharmaceutique selon l'invention comprend un peptide de fusion tel que décrit ci-dessus, en particulier, un peptide choisi parmi un peptide représenté par la séquence SEQ ID NO : 35, ou un peptide représenté par la séquence SEQ ID NO : 40,.

Dans un mode de réalisation particulier de l'invention, ladite composition pharmaceutique est formulée pour une administration journalière de 1 mg/m² à 1000 mg/m².

L'administration d'une telle composition pharmaceutique peut être effectuée par voie orale, par voie intraveineuse, par voie parentérale, par voie nasale, par voie pulmonaire.

La présente invention a également pour objet l'utilisation d'une combinaison comprenant :
- une molécule d'intérêt diagnostic ou thérapeutique, et
- un transporteur destiné à l'internalisation de ladite molécule d'intérêt à des cellules cibles,
   pour la préparation d'un médicament destiné au traitement ou à la prévention des cancers tels que mélanomes, cancer du sein, glioblastomes (tumeurs du cerveau), cancer du colon, lymphomes, ledit transporteur étant :
- un peptide comprenant la séquence d'acides aminés SEQ ID NO : 1, ou
- un peptide comprenant une séquence d'acides aminés ayant 93%, notamment 95%, particulièrement 98% d'homologie d'identité de séquence avec la séquence SEQ ID NO : 1.

Plus particulièrement, la présente invention concerne l'utilisation d'un peptide de fusion choisi parmi un peptide représenté par la séquence SEQ ID NO : 35, ou un peptide représenté par la séquence SEQ ID NO : 40, pour la préparation d'un médicament destiné au traitement ou à la prévention des cancers tels que mélanomes, cancer du sein, glioblastomes (tumeurs du cerveau), cancer du colon, lymphomes.

La présente invention est illustrée par les figures et les exemples suivants. Les exemples ci-après visent à éclaircir l'objet de l'invention et illustrer des modes de réalisation avantageux, mais en aucun cas ne vise à restreindre la portée de l'invention.
Figure 1A : La figure 1A présente la structure de la protéine ZEBRA complète (Z-FL), ainsi que celle de neuf fragments tronqués de la protéine ZEBRA, à savoir Z1 (acides aminés 1 à 195 de la protéine ZEBRA), Z2 (acides aminés 1 à 170 de la protéine ZEBRA), Z3 (acides aminés 1 à 140 de la protéine ZEBRA), Z4 (acides aminés 140 à 245 de la protéine ZEBRA), Z5 (acides aminés 140 à 195 de la protéine ZEBRA), Z6 (acides aminés 170 à 245 de la protéine ZEBRA), Z7 (acides aminés 170 à 195 de la protéine ZEBRA), Z8 (acides aminés 195 à 245 de la protéine ZEBRA) et Z9 (acides aminés 170 à 220 de la protéine ZEBRA).
Figure 1B : la figure 1B montre l'expression de la protéine ZEBRA complète (Z-FL) et de ses fragments Z1, Z2, Z3, Z4, Z5, Z6, Z7 et Z8 dans *E.coli* BL21 (DE3). Les protéines sont purifiées par chromatographie d'affinité à nickel, et séparées sur un SDS-PAGE, ensuite détectées par Western blot utilisant l'anticorps anti-His.
Figure 1C : La figure 1C montre l'expression des protéines de fusion Z-FL-EGFP, Z2-EGFP, Z3-EGFP, Z4- EGFP, Z5- EGFP, Z6- EGFP, Z8- EGFP, Z9- EGFP et 29-βGal dans *E.coli* BL21 (DE3). Les protéines sont séparées sur un SDS-PAGE de 13% et détectées par la coloration au bleu de Coomassie.
Figure 1D : La figure 1D montre la fluorescence intracellulaire émise par les cellules HeLa vivantes après 15 heures d'incubation respectivement avec 0,2µM de Z-FL-EGFP, Z2-EGFP, Z3-EGFP, Z4- EGFP, Z5- EGFP, Z6- EGFP, Z8- EGFP et Z9- EGFP. La fluorescence émise par des cellules est observée par microscopie à fluorescence. Les photos de la ligne supérieure représentent, pour chacune des protéines de fusion, la visualisation de cellules vivantes avant la transduction effectuée par la protéine ZEBRA complète ou avant celle par différents fragments tronqués de ZEBRA. Les photos de la ligne inférieure représentent, pour chacune des protéines de fusion, la visualisation de cellules après l'internalisation des différentes protéines de fusion indiquées.
Figure 1E : La figure 1E montre l'analyse des cellules HeLa et Saos2 par cytométrie en flux. Les cellules sont incubées respectivement avec 0,2µM de Z-FL-EGFP, Z2-EGFP, Z3-EGFP, Z4- EGFP, Z5- EGFP, Z6- EGFP, Z8- EGFP et Z9-EGFP, et ensuite traitées par trypsine avant l'analyse FACS. L'intensité de fluorescence moyenne est obtenue à partir de trois expériences indépendantes.
Figure 1F : La figure 1F montre l'accumulation de Z9-EGFP dans les cellules C2C12 et Saos2. 0,2µM de Z9-EGFP sont ajoutées dans le milieu de culture sans sérum. La fluorescence intracellulaire est visualisée par microscopie à florescence. Les photos de la ligne supérieure représentent pour chacune des lignées cellulaires la visualisation de cellules vivantes avant la transduction effectuée par le fragment Z9-EGFP. Les photos de la ligne inférieure représentent respectivement la visualisation de cellules après l'internalisation des différentes protéines de fusion indiquées.
Figure 1G : La figure 1G représente la séquence d'acides aminés du domaine minimal de la protéine ZEBRA. Les résidus basiques sont marqués en gris, et les résidus hydrophobes sont soulignés.
Figure 2 : La figure 2 présente le résultat de l'expérience du gel retard avec l'ADN. Les différents fragments tronqués de la protéine ZEBRA et les protéines de fusion, à savoir Z-FL-EGFP, Z6-EGFP, Z9-EGFP, Z6-βGal, Z9- βGal, sont analysés pour leur capacité de se lier à la sonde d'ADN AP-1. Les signaux sont détectés par Chemiluminescent Assay (Pierce).
Figure 3A: La figure 3A présente l'internalisation intracellulaire temps-dépendante de Z9-EGFP dans les cellules HeLa. Les cellules sont incubées pendant respectivement 10 minutes, 30 minutes, 1 heure, 2 heures, 4 heures et 24 heures, avec 0,2µM de Z9-EGFP à 37°C. Les cellules sont ensuite traitées par trypsine pendant 10 minutes à 37°C. La fluorescence cellulaire moyenne est analysée par cytométrie en flux. L'axe des abscisses représente le temps d'incubation de cellules avec Z9-EGFP. L'axe des ordonnées représente la fluorescence cellulaire moyenne.
Figure 3B : La figure 3B présente l'internalisation intracellulaire temps-dépendante de Z9-EGFP dans les cellules Saos2. Les cellules sont incubées pendant respectivement 10 minutes, 30 minutes, 1 heure, 2 heures, 4 heures et 24 heures, avec 0,2µM de Z9-EGFP à 37°C. Les cellules sont ensuite traitées par trypsine pendant 10 minutes à 37°C. La fluorescence cellulaire moyenne est analysée par cytométrie en flux. L'axe des abscisses représente le temps d'incubation de cellules avec Z9-EGFP. L'axe des ordonnées représente la fluorescence cellulaire moyenne.

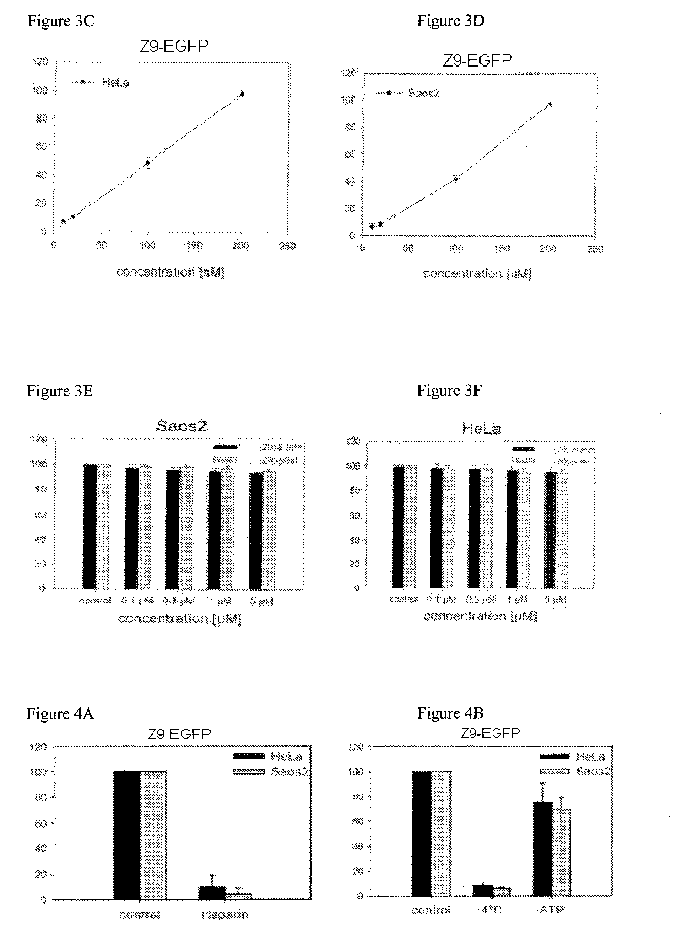

Figure 3C : La figure 3C présente l'internalisation intracellulaire dose-dépendante de Z9-EGFP dans les cellules HeLa. Z9-EGFP de différentes concentrations (10, 20, 100, 200nM) sont ajoutés respectivement dans le milieu de culture sans sérum. Après 4 heures d'incubation, les cellules sont rincées par PBS, et ensuite traitées par trypsine pendant 10 minutes à 37°C. La fluorescence cellulaire est mesurée par la cytométrie en flux. Toutes les expériences de transduction sont effectuées en triple. L'axe des abscisses représente la concentration cellulaire (nM). L'axe des ordonnées représente le pourcentage de cellules EGFP-positives.
Figure 3D : La figure 3D présente l'internalisation intracellulaire dose-dépendante de Z9-EGFP dans les cellules Saos2. Z9-EGFP à différentes concentrations (10, 20, 100, 200nM) sont ajoutées respectivement dans le milieu de culture sans sérum. Après 4 heures d'incubation, les cellules sont rincées par PBS, et ensuite traitées par trypsine pendant 10 minutes à 37°C. La fluorescence cellulaire est mesurée par la cytométrie en flux. Toutes les expériences de transduction sont effectuées en triple dans deux expériences indépendantes. L'axe des abscisses représente la concentration cellulaire (nM). L'axe des ordonnées représente le pourcentage de cellules EGFP-positives.
Figure 3E: La figure 3E présente la viabilité des cellules Saos2 après l'internalisation de Z9-EGFP à différentes concentrations. Les cellules Saos2 sont incubées pendant 24 heures avec respectivement 0,1µM, 0,3µM, 1µM ou 3µM de Z9-EGFP dans le milieu de culture à 37°C. La cytotoxicité après l'internalisation de Z9-EGFP est déterminée par la présence de la déshydrogénase lactique (LDH) dans le milieu de culture. Chaque colonne représente la viabilité moyenne de deux expériences indépendantes répétées en triples. L'axe des abscisses représente la concentration cellulaire (µM). L'axe des ordonnées représente la viabilité cellulaire.
Figure 3F : La figure 3F présente la viabilité des cellules HeLa après l'internalisation de Z9-EGFP de différentes concentrations. Les cellules HeLa sont incubées pendant 24 heures avec respectivement 0,1µM, 0,3µM, 1µM ou 3µM de Z9-EGFP dans le milieu de culture à 37°C. La cytotoxicité après l'internalisation de Z9-EGFP est déterminée par la présence de la déshydrogénase lactique (LDH) dans le milieu de culture. Chaque colonne représente la viabilité moyenne de deux expériences indépendantes répétées en triples. L'axe des abscisses représente la concentration cellulaire (µM). L'axe des ordonnées représente la viabilité cellulaire.
Figure 4A : La figure 4A présente l'effet d'héparine sur l'internalisation de fragment Z9-EGFP dans les cellules HeLa représentées par la barre noire, et les cellules Saos2 représentées par la barre grise. Les cellules sont incubées avec pendant 30 minutes à 37°C 20µg/ml d'héparine et ensuite exposées à 0,2 µM de Z9-EGFP pendant 3 heures à 37°C. Les cellules sont incubées avec la trypsine pendant 10 minutes à 37°C et rincées avant d'être analysées par cytométrie en flux. L'axe des ordonnées représente le taux d'internalisation de Z9-EGFP dans les cellules.
Figure 4B : La figure 4B présente l'effet de la basse température et l'épuisement de réserve d'ATP cellulaire sur l'internalisation de fragment Z9-EGFP. Les cellules HeLa représentées par la barre noire, et les cellules Saos2 représentées par la barre grise, sont incubées avec 0,2 µM de Z9-EGFP pendant 1 heure à 4°C. Afin d'épuiser la réserve d'ATP cellulaire, les cellules sont incubées pendant 1 heure avec 6mM de 2-deoxy-D-glucose et 10mM d'azoture de sodium, ensuite exposées à 0,2 µM de Z9-EGFP pendant 1 heure. Après le traitement par trypsine, les cellules sont analysées par cytométrie en flux. L'axe des ordonnées représente le taux d'internalisation de Z9-EGFP dans les cellules.

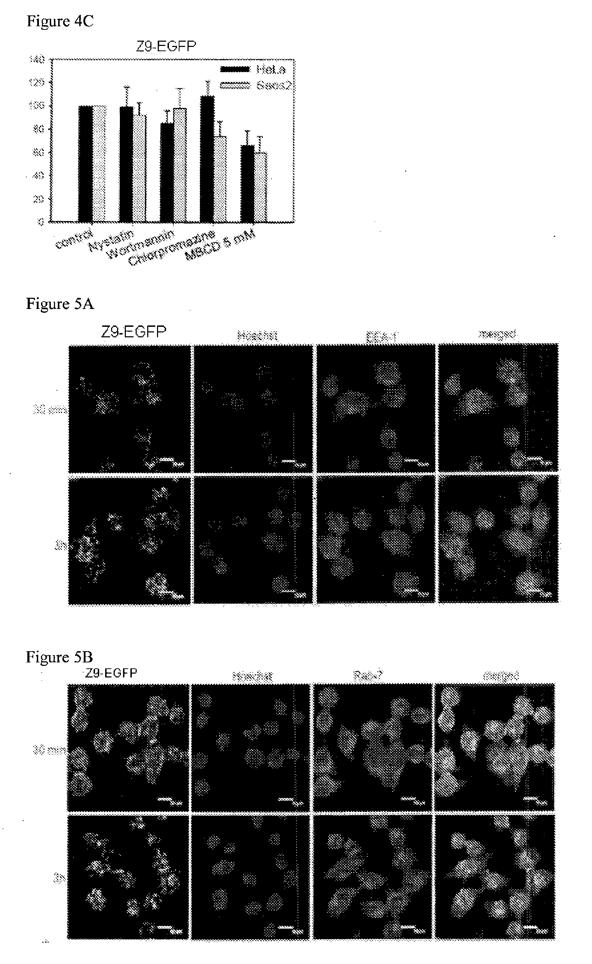

Figure 4C : La figure 4C présente l'effet de différents inhibiteurs endocytotiques sur l'internalisation de fragment Z9-EGFP dans les cellules HeLa représentées par la barre noire, et les cellules Saos2 représentées par la barre grise. Les cellules sont traitées respectivement par 20 µg/ml d'héparine, 100 nM de wortmannine, 50 µg/ml de nystatine, 30 µM d'hydrochlorure de chlorpromazine and 5-10 mM de méthyl-β-cyclodextrine (MβCD), pendant 30 minutes, avant l'addition de 0,2 µM de Z9-EGFP. La fluorescence cellulaire moyenne mesurée dans les cellules traitées est normalisée par rapport à celle mesurée dans les cellules non traitées, en tant que témoin. Chaque expérience est répétée indépendamment en triple. L'axe des ordonnées représente le taux d'internalisation de Z9-EGFP dans les cellules.
Figure 5A : La figure 5A présente la localisation intracellulaire de Z9-EGFP dans les cellules HeLa. Les cellules sont incubées respectivement pendant 30 minutes ou 3 heures avec 0,2 µM de Z9-EGFP. Les cellules sont visualisées par microscopie confocale. Les images sont acquises séquentiellement. Les flèches blanches dans les photos de la première colonne de gauche indiquent la présence Z9-EGFP dans les cellules. Les flèches blanches dans les photos de la deuxième colonne de gauche indiquent les nucleus, qui sont révélés par le colorant fluorescent Hoechst 33258. Les flèches blanches dans les photos de la troisième colonne de gauche indiquent les marqueurs d'endosomes EEA-1, qui sont révélés par le colorant fluorescent Alexa Fluor®647. Les flèches blanches dans les photos de la première colonne de droite indiquent la superposition des signaux fluorescents émis par les nucleus, par Z9-EGFP dans les cellules, et par les marqueurs EEA-1. La barre blanche représente 10 µm.
Figure 5B : La figure 5B présente la localisation intracellulaire de Z9-EGFP dans les cellules HeLa. Les cellules sont incubées respectivement pendant 30 minutes ou 3 heures avec 0,2 µM de Z9-EGFP. Les cellules sont visualisées par microscopie confocale. Les images sont acquises séquentiellement. Les flèches blanches dans les photos de la première colonne de gauche indiquent la présence Z9-EGFP dans les cellules. Les flèches blanches dans les photos de la deuxième colonne de gauche indiquent les nuclei, qui sont révélés par le colorant fluorescent Hoechst 33258. Les flèches blanches dans les photos de la troisième colonne de gauche indiquent les marqueurs d'endosomes Rab-7, qui sont révélés par le colorant fluorescent Alexa Fluor®647. Les flèches blanches dans les photos de la première colonne de droite indiquent la superposition des signaux fluorescents émis par les nucleus, par Z9-EGFP dans les cellules, et par les marqueurs Rab-7. La barre blanche représente 10 µm.

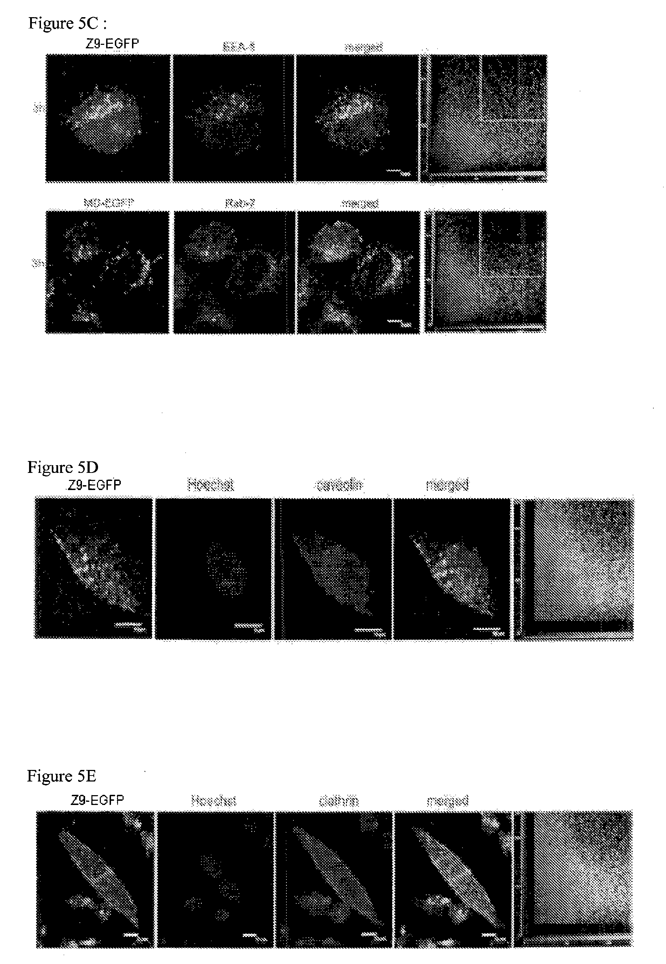

Figure 5C : La figure 5C présente la localisation intracellulaire de Z9-EGFP dans les cellules HeLa. Les cellules sont incubées respectivement pendant 3 heures avec 0,2 µM de Z9-EGFP. Les flèches blanches dans les photos de la deuxième colonne de gauche indiquent les marqueurs d'endosomes Rab-7 ou EEA-1, qui sont révélés par le colorant fluorescent Alexa Fluor®647. Les flèches blanches dans les photos de la troixième colonne de droite indiquent la superposition des signaux fluorescents émis par Z9-EGFP dans les cellules et par les marqueurs Rab-7 ou EEA-1. La barre blanche représente 5 µm. Le diagramme de droite représente l'intensité relative de pixels.
Figure 5D : La figure 5D présente la localisation intracellulaire de Z9-EGFP dans les cellules HeLa. Les cellules sont incubées respectivement pendant 3 heures avec 0,2 µM de Z9-EGFP. Les flèches blanches dans les photos de la première colonne de gauche indiquent la présence Z9-EGFP dans les cellules. Les flèches blanches dans les photos de la deuxième colonne de gauche indiquent les nucleus, qui sont révélés par le colorant fluorescent Hoechst 33258. Les flèches blanches dans les photos de la troisième colonne de gauche indiquent les marqueurs d'endosomes caveolin-1, qui sont révélés par Alexa Fluor®647. Les flèches blanches dans les photos de la deuxième colonne de droite indiquent la superposition des signaux fluorescents émis par les nucleus, par Z9-EGFP dans les cellules, et par les marqueurs caveolin-1. Le diagramme de droite représente l'intensité relative de pixels.
Figure 5E : La figure 5E présente la localisation intracellulaire de Z9-EGFP dans les cellules HeLa. Les cellules sont incubées respectivement pendant 3 heures avec 0,2 µM de Z9-EGFP. Les flèches blanches dans les photos de la première colonne de gauche indiquent la présence Z9-EGFP dans les cellules. Les flèches blanches dans les photos de la deuxième colonne de gauche indiquent les nucleus, qui sont révélés par le colorant fluorescent Hoechst 33258. Les flèches blanches dans les photos de la troisième colonne de gauche indiquent les marqueurs d'endosomes clathrine, qui sont révélés par Alexa Fluor®647. Les flèches blanches dans les photos de la deuxième colonne de droite indiquent la superposition des signaux fluorescents émis par les nucleus, par Z9-EGFP dans les cellules, et par les marqueurs clathrine. Le diagramme de droite représente l'intensité relative de pixels.

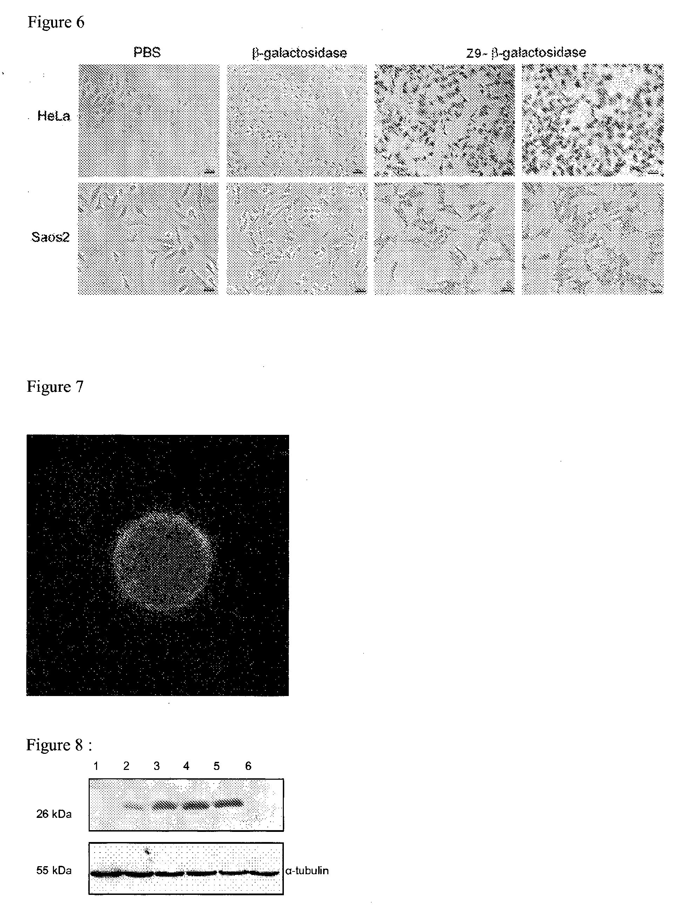

Figure 6 : La figure 6 présente l'internalisation de la β-galactosidase fonctionnelle dans les cellules HeLa et Saos2 par l'intermédiaire de fragment Z9. Les cellules sont fixées et détectées par le kit de détection de X-Gal (Sigma), après 16 heures d'incubation avec 0,2 µM de Z9-β-galactosidase à 37°C. La présence de la β-galactosidase est visualisée par microscope à contraste de phase. Les photos de la première colonne de gauche représentent les cellules incubées seulement dans un tampon de PBS. Les photos de la deuxième colonne de gauche représentent les cellules incubées avec la β-galactosidase. Les photos de deux colonnes de droite représentent les cellules incubées avec la protéine de fusion Z9-β-galactosidase. La barre noire représente 10 µm.
Figure 7 : La figure 7 montre l'entrée de Z9-EGFP dans des liposomes synthétiques.
Figure 8 : La figure 8 montre l'analyse de l'activation de la voie des caspases dans des cellules de glioblastomes de souris (GL26) après traitement et internalisation de la protéine de fusion Z9-eIF3-f, **représentée par la séquence SEQ ID NO : 35**. Piste 1 : contrôle sans protéine ; pistes 2 à 5 : 0.07nM, 0.13nM ; 0.25nM ; 0.31nM respectivement ; piste 6 : contrôle avec etoposide. L'activation de la voie des caspases se fait par détection de la caspase 9 activée et clivée pour donner une bande à 26kDa.

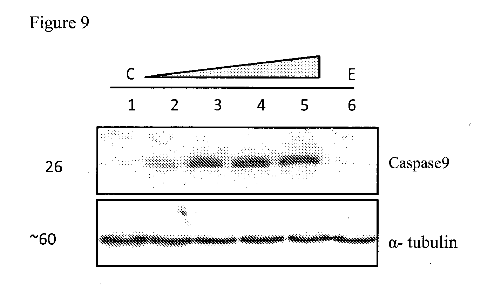

Figure 9 : La figure 9 montre l'analyse de l'activation de la voie des caspases dans des cellules de glioblastomes de souris (GL26) après traitement et internalisation de la protéine de fusion Z9-eIF3-f, représentée par la séquence SEQ ID NO : 35. Piste 1 : contrôle sans protéine ; pistes 2 à 5 : 0.07nM, 0.13nM ; 0.25nM ; 0.31nM respectivement ; piste 6 : contrôle avec etoposide. L'activation de la voie des caspases se fait par détection de la caspase 9 activée et clivée pour donner une bande à 26kDa.

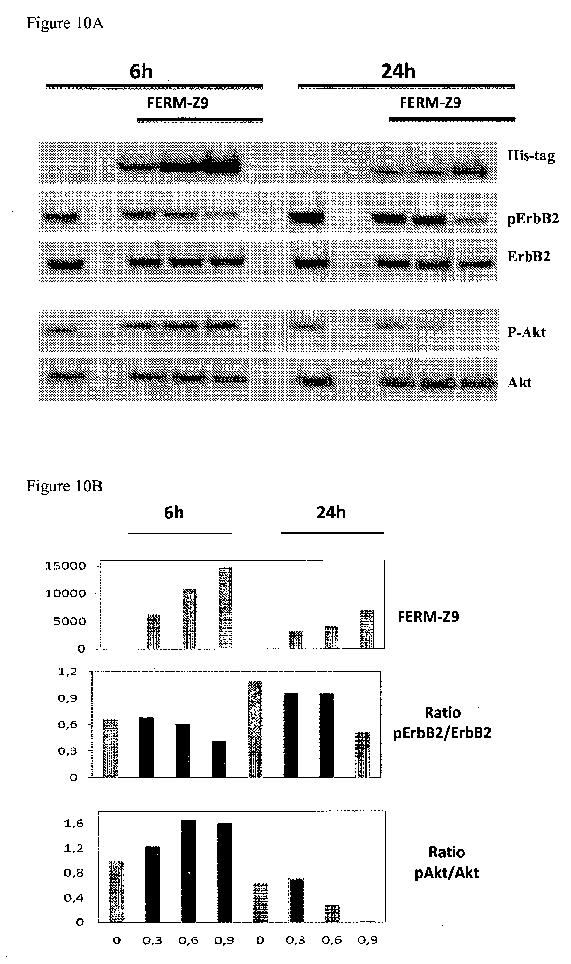

Figure 10A : La figure 10A montre l'analyse des effets cellulaires dans des cellules de carcinomes mammaires humains (SKBR3) après traitement et internalisation de la protéine de fusion FERM-Z9, représentée par la séquence 40. Piste 1 : T=0, contrôle sans protéine ; pistes 2 à 4 : 0.3 µM, 0.6 µM ; 0.9 µM respectivement après 6 heures de traitement en présence de la protéine de fusion FERM-Z9; piste 5 : T=0, contrôle sans protéine ; pistes 6 à 8 : 0.3 µM, 0.6 µM ; 0.9 µM respectivement après 24 heures de traitement en présence de la protéine de fusion FERM-Z9. L'internalisation de la protéine de fusion FERM-Z9 est révélée par un anticorps anti-histidine (His-tag) ; l'activité des protéines ErbB2 et Akt par des anticorps anti-phospho ErbB2 (pErbB2) et Akt (P-Akt) respectivement. La présence des protéines ErbB2 et Akt comme contrôle est révélée par des anticorps anti-ErbB2 (ErbB2) et Akt (Akt) respectivement. Une diminution de l'activité et de la phosphorylation du récepteur ErbB2 et de là protéine Akt est détectée au bout de 24 heures de traitement par la protéine de fusion FERM-Z9 pour des concentrations allant de 0.3 µM à 0.9 µM.
Figure 10B : La figure 10B montre une représentation schématique des effets cellulaires dans des cellules de carcinomes mammaires humains (SKBR3) après 6 ou 24 heures de traitement et internalisation de la protéine de fusion FERM-Z9. Schéma supérieur ; analyse quantitative de la protéine de fusion FERM-Z9 détectée dans les cellules de carcinomes mammaires humains (SKBR3) après traitement et internalisation. L'axe des ordonnées indique le nombre de protéine de fusion FERM-Z9 révélée par un anticorps anti-histidine (unités arbitraires). L'axe des abscisses indique la concentration de protéine de fusion FERM-Z9 utilisée dans le traitement des cellules SKBR3 à 6 ou 24 heures. Les quantités de protéines ErbB2 et Akt phosphorylées après traitement sont quantifiées par rapport à la quantité totale de protéine ErbB2 et Akt respectivement indiquant une diminution de la phosphorylation et par voie de conséquence de l'activité de ces protéines en fonction des quantités croissantes de protéine de fusion FERM-Z9 utilisée dans ces expériences.

### Exemple 1

### Matériels et méthodes

### 1.1 Clonage de la protéine ZEBRA et ses fragments

Les fragments d'ADN codant la protéine ZEBRA complète (Z-FL) ou ses fragments (Z1, Z2, Z3, Z4, Z5, Z6, Z7, Z8 et Z9) sont obtenus par PCR et insérés dans le vecteur d'expression pET15b (Novagen) de *E.coli,* qui permet d'exprimer les peptides dont l'extrémité N-terminale est liée à une étiquette de 6-histidine. La protéine complète de ZEBRA, ainsi que les fragments tronqués, à savoir Z2, Z4, Z5, Z6, Z7, Z8 et Z9, sont fusionnées respectivement avec EGFP (Enhanced Green Fluorescent Proteins), donnant les protéines de fusion Z-FL-EGFP, Z2-EGFP, Z3-EGFP, Z4-EGFP, Z5-EGFP, Z6-EGFP, Z8-EGFP et Z9-EGFP. Les fragments Z6 et Z9 sont également fusionnées respectivement avec la β-galactosidase, donnant les protéines de fusion Z6- βGal, Z9- βGal.

Les séquences d'amorces utilisées pour la construction de ces fragments sont listées dans le tableau 1 ci-dessous.

**Tableau 1**

| **Nom** | **Séquence 5' → 3'** | **Site de restriction** |
|---|---|---|
| | **Construction de ZEBRA complet** | |
| ZebraXhoIfor | CCGCTCGAGATGATGGACCCAAACTCGAC | *Xho*I |
| ZebraBamHIrev | CGCGGATCCGAAATTTAAGAGATCCTCGTG | *Bam*HI |
| ZebraBamHIStoprev | CGCGGATCCTTAGAAATTTAAGAGATCCTCGTG | *Bam*HI |

| | **Construction de fragments ZEBRA tronqués** | |
|---|---|---|
| ZebraAS1NdeIfor | GGAATTCCATATGGACCAAACTCGAC | *Nde*I |
| ZebraAS195BamHIrev | CGCGGATCCTTATTGCTTAAACTTGGCCCGGC | *Bam*HI |
| ZebraAS170BamHIrev | CGCGGATCCTTATTCCTCCAGCGATTCTGGC | *Bam*HI |
| ZebraAS140BamHIrev | CGCGGATCCTTACTGTTGTCCTTGGTTAGCCC | *Bam*HI |
| ZebraAS170NdeIfor | GGAATTCCATATGCAGCTAGCAGACATTGGTGTTCC | *Nde*I |
| ZebraAS195NdeIfor | GGAATTCCATATGCAACTGCTGCAGCACTACC | *Nde*I |
| ZebraBamHIStoprev | CGCGGATCCTTAGAAATTTAAGAGATCCTCGTG | *Bam*HI |
| ZebraAS50NdeIfor | GGAATTCCATATGCCGGTGCTGCCAGAGCC | *Nde*I |
| ZebraAS100NdeIfor | GGAATTCCATATGGACATAACCCAGAATCAACAG | *Nde*I |
| ZebraAS50XhoIrev | CCGCTCGAGCGGCCACAGCACACAAGG | *XHo*I |
| ZebraAS100XhoIrev | CCGCTCGAGTATGTCGGAGACTGGGAACAG | *XHo*I |
| ZebraAS140XhoIrev | CCCTCTCGAGCTGTTGTCCTTGGTTAGCCC | *Xho*I |
| ZebraAS245XhoIrev | CCGCTCGAGGAAATITAAGAGATCCTCGTG | *Xho*I |
| ZebraAS195XhoIrev | CCCTCTCGAGTTGCTTAAACTTGGCCCGGC | *Xho*I |
| ZebraAS140XhoIrev | CCCTCTCGAGCTGTTGTCCTTGGTTAGCCC | *Xho*I |
| ZebraAS140NdeIfor | GGAATTCCATATGCAGCTAGCAGACATTGGTGTTCC | *Nde*I |
| ZebraAS195NdeIfor | GGAATTCCATATGCAACTGCTGCAGCACTACC | *Nde*I |
| ZebraAS170NdeIfor | GGAATTCCATATGGAGGAATGCGATTCTGAACTAG | *Nde*I |
| ZebraAS220XhoIrev | CCGCTCGAGGCACATCTGCTTCAACAGG | *Xho*I |
| ZebraAS100XhoIrev | CCGCTCGAGTATGTCGGAGACTGGGAACAG | *Xho*I |
| ZebraAS175NdeIfor | GGAATTCCATATGAAGCGATACAAGAATCGGGTGGC | *Nde*I |

| | **Protéine de fusion avec GFP** | |
|---|---|---|
| GFPNdeIfor | GGA ATTCCATATGGTGAGCAAGGGCGAGGA | *Nde*I |
| GFPXhoIrev | CCCTCTCGAGCTTGTACAGCTCG | *Xho*I |
| GFPXhoIfor | CCGCTCGAGATGGTGAGCAAGGGCGAG | *Xho*I |
| GFPBamHIrev | CGCGGATCCCTTGTACAGCTCGTCCATGC | *Bam*HI |

| | **Protéine de fusion avec LacZ** | |
|---|---|---|
| lacZXhoIfor | CCCTCTCGAGATGATTACGGATTCACTGGCCGTC GTTTTACAACGTCG | *Xho*I |
| LacZBamHIrev | CGCGGATCCTTTTTGACACCAGACCAACTGG | *Bam*HI |

| | **Protéine de fusion avec MDA7** | |
|---|---|---|
| Mda7 N (D1) | TAACGCCTCGAGATTTTCAACAGAGGCTGCAAAG | *Xho*I |
| Mda7 N (R1 | ATTCTTATGGATCTTTAGAGCTTGTAGATTTT?TGCATCC | *Bam*HI |
| MDA7AS51XhoIfor | CCGCTCGAGGGCCAAGAATTCCACTTTGG | *Xho*I |

| | **Protéine de fusion avec eIF3** | |
|---|---|---|
| eIF3-N (D1) | TAACGCCTCGAGGCCACACCGGCGGTACCAGTAAGTGCT CCTCCG | *Xho*I |
| eIF3-N (R1) | ATTCTTATGGATCCTTACAGGTTTACAAGTTTTTCATTG | *Bam*HI |

### 1.2 Expression et purification de protéines recombinantes

Les protéines de fusion recombinantes sont exprimées dans la lignée BL21 (DE3) de *E.coli,* après l'induction par 0,5 mM d'IPTG pendant 15h à 16°C. Les cellules sont lysées par sonication dans 20mMde tampon de Tris, à pH 6,8 à 8, contenant 250mM de NaCl et 10% de glycérol. Les cellules lysées sont ensuite traitées par DNase I (Roche) pour éliminer des acides nucléiques. La purification des protéines possédant une étiquette de His6 est effectuée par la chromatographie d'affinité nickel. Les protéines sont rincées par un gradient de NaCl de 0,5 à 1,5 M et éluées avec un tampon contenant 500mM d'imidazole, 20mM de Tris, 75 mM de KCl, 0,5 M de NaCl et 10% de glycérol. Toutes les étapes de purification sont effectuées à 4°C et à la présence des inhibiteurs de protéase (Pepstatine, E-64, Aprotinin, Pefablock et le mix complet des inhibiteurs de protéase, Roche). Avant les expériences de transduction, les protéines purifiées sont dialysées contre PBS (tampon phosphate salin).

### 1.3 Milieu de culture et expériences de transduction

Les lignées cellulaires HeLa, Saos-2, C2C12 sont mises en oeuvre dans les expériences de transduction.

Les cellules HeLa sont maintenues dans le milieu de culture DMEM (Dulbecco's Modified Eagle Medium) (Gibco), et les cellules Saos-2 sont maintenues dans le milieu de culture « MaCoy's 5A » (Gibco) complété par 50U/ml de pénicilline, 50 µg/ml de streptomycine et 2 mM de L-glutamine (Gibco), et 10-20% du sérum de veau foetal inactivé à haute température (Gibco). 7,5x10⁵ cellules par puits sont semées sur une plaque de 12 puits à 24h avant les expériences de transduction. Pour l'analyse par microscopie, les cellules (2,5x10⁵) sont étalées dans une chambre de culture de 4 puits au moins 24 h avant la manipulation.

Les expériences d'internalisation sont effectuées à 60-80% de confluence. Les cellules sont rincées deux fois par le tampon phosphate salin avant l'addition du milieu de culture frais et dépourvu de sérum. Le milieu de culture contient la quantité indiquée de protéines. 4 heures après, le milieu de culture est complété par du sérum de veau foetal inactivé à haute température (Gibco) pour une incubation de long terme.

### 1.4 Immunocytochimie et microscopie à fluorescence

Lors de l'incubation de cellules avec les protéines fusionnées à EGFP, les cellules sont rincées par PBS, soumises ensuite à une trypsination modérée (0,5% de trypsine-EDTA) et plusieurs rinçages par héparine (20 µg/ml) dans PBS et fixées pendant 10 minutes dans 4% PFA à température ambiante. Les cellules sont perméabilisées et bloquées par 0,25% TritonX100 et 5% de BSA dans PBS pendant 1h à température ambiante, et ensuite incubées pendant 1h à température ambiante dans un tampon PBS contenant 0,1% TritonX100 et 5% BSA avec un anticorps primaire correspondant. Afin de détecter les protéines endosomales, un anticorps anti-EEA1 (4µg/ml, Calbiochem), un anticorps anti-Rab7 (5µg/ml, Cell Signaling), un anticorps anti-clathrin ou un anti-caveloelin-1 (5µg/ml, Santa Cruz Biotechnology) sont utilisés respectivement. Après 3 rinçages par PBS pendant 10 minutes, les cellules sont incubées avec un anticorps secondaire correspondant, à savoir anti-souris Alexa Fluor^{®}555 et anti-lapin Alexa Fluor^{®}647 (Molecular Probes), dans un tampon contenant 0,4% de TritonX100 et 5% de BSA. Les cellules sont rincées 5 fois pendant 10 minutes par PBS et les nucleus sont colorés par Hoechst 33258 (Molecular Probes). La fluorescence cellulaire sur les cellules non-fixées est visualisée à l'aide d'une microscopie à fluorescence (Nikon Eclipse TE2000-E) équipée d'un filtre de GFP (l'excitation à de 465 à 495 nm et l'émission de 515 à 555 nm).

Pour étudier la localisation des protéines de fusion dans des cellules, une microscopie confocale (TCS-SP2-Leica Manheim, Germany) est utilisée. Les images sont acquises séquentiellement, avec l'excitation à 488nm pour Z9-EGFP (collection de fluorescence entre 500 et 540nm, affiché en vert), l'excitation à 633nm pour Alexa Fluor® 647 couplé avec un anti-EEA1, un anti-Rab7, un anti-clathrin ou un anti-caveloelin-1 (collection de fluorescence entre 650 et 700nm, affiché en rouge), et l'excitation à 405nm pour Hoechst (collection de fluorescence entre 415 et 460nm, affiché en bleu).

### 1.5 Analyse par Western blot

Après les expériences de transduction, les cellules sont collectées et les protéines non-internalisées sont éliminées par trypsination. L'extrait cellulaire complet est préparé par lyse de cellules mammifères dans le tampon de lyse (Sigma) à 0°C. Les fractions cytosoles et nucléiques sont séparées par le kit de compartimentation de cellules (Pierce). Les anticorps primaires sont respectivement un anticorps monoclonal murin anti-ZEBRA Z125/Z130 et un anticorps monoclonal murin anti-GFP (Euromedex). Après l'incubation avec un anticorps secondaire murin (Amersham) marqué par peroxidase, la membrane est rincée et ensuite analysée par un système de détection de chimiluminescence avancé (Amersham).

### 1.6 Analyse par cytométrie en flux

Les cellules sont traitées par 0,5% de trypsine et 20µg/ml de héparine pendant 10 minutes afin d'éliminer des protéines liées à la surface de cellules avant l'analyse de fluorescence verte. Seules les cellules vivantes sont analysées et les cellules mortes sont éliminées par Amino-Actinomycin D (7-AAD). La cytométrie en flux est mise en oeuvre par la technique FACS (fluorescence-activated cell sorting) (Becton Dickinson).

### 1.7 Expérience du gel retard avec de l'ADN (Electrophoretic mobility shift assay (EMSA))

La technique EMSA est mise en oeuvre par la sonde AP-1, constituée de deux oligonucléotides hybridés (5'-AGCACTGACTCATGAAGT-3' et 5'-TACTTCATGAGTCAGTGCT-3'). La sonde froide est marquée par de la biotine et purifiée sur une mini-colonne (Microspin G-25, Active Motif). Jusqu'à 500 µg de la protéine ZEBRA complète ou de ses fragments sont préincubées pendant 15 minutes sur la glace avec 4x tampon de liaison B-1, 2x tampon de stabilisation (Generka) et 1 mM de DTT. La sonde marquée par la biotine est mélangée avec 4x tampon de liaison C-1, 2x tampon de stabilisation (Generka) et 50ng/µl de poly (dI-dC) et ajoutée à la solution contenant les protéines. Après une incubation du mélange de réaction pendant 15 minutes à 4°C, les échantillons sont séparés sur gel de polyacrylamide non dénaturant dans le tampon 0,5xTBE et ensuite transférés sur une membrane de nylon Hybond H+ (Amersham). La présence des bandes est détectée par le kit (LightShift Chemiluminescent EMSA Kit, Pierce).

### 1.8 Mesure de cytotoxicité

L'intégrité de la membrane est mesurée par le kit de détection de cytotoxicité (Roche Applied Science). 1x10⁴ HeLa ou Saos2 cellules sont semées dans les plaques de 96 puits 24 h avant le traitement par Z9, dans un milieu de culture sans sérum, avec les concentrations indiquées par le fabricant. Après 24h de traitement, la mesure est mise en oeuvre à l'aide de la déshydrogénase lactique selon le protocole fourni par le fabricant.

### 1.9 Traitement par des produits chimiques

L'héparine, ainsi que des inhibiteurs endocytotiques tels que, la wortmannine, la nystatine, l'hydrochlorure de chlorpromazine et la méthyl-β-cyclodextrine (MβCD), la 2-deoxy-D-glucose et l'azoture de sodium, sont achetés de Sigma. Avant l'addition de Z9-EGFP, les cellules sont d'abords incubées pendant 30 minutes dans un milieu de culture sans sérum contenant l'un des produits cités ci-dessus, dans une concentration indiquée (20 µg/ml d'héparine, 100 nM de wortmannine, 50 µg/ml de nystatine, 30 µM d'hydrochlorure de chlorpromazine and 5-10 mM de méthyl-β-cyclodextrine (MβCD)). Les cellules sont ensuite incubées pendant 3 heures, à 37°C ou 4°C, en présence d'inhibiteurs et de Z9-EGFP. Avant l'analyse de fluorescence par cytométrie en flux, les cellules sont incubées avec 0,5% de trypsine-EDTA, pour éliminer les protéines liées à la surface de cellules. Afin d'épuiser la réserve d'ATP, les cellules sont préincubées pendant 1 heure dans PBS contenant 6mM de 2-deoxy-D-glucose et 10mM d'azoture de sodium.

### 1.10 Détection de β-galactosidase

Après les expériences de transduction, les cellules sont rincées par 20µg/ml d'héparine dans PBS, et traitées par trypsine afin d'éliminer les protéines liées à la surface de cellules. La fixation et la détection sont effectuées selon le protocole du kit (β-galactosidase Reporter Gene Staining Kit (Sigma)). Les cellules sont incubées pendant 10 minutes à température ambiante avec 1x solution de fixation, contenant 2% de formaldéhyde et 0,2% de glutaraldéhyde. Après trois étapes de rinçage par PBS à température ambiante, les cellules sont révélées par une solution, contenant 20mM de MgCl₂, 40mM de ferricyanide de potassium et 2mg/mL de β-galactosidase, pendant 3 heures à 37°C. Les images sont prises par microscope à contraste de phase (Nikon Eclipse TE2000-E).

### 1.11 Internalisation de cellules par des complexes ADN/Z9

Les complexes formés entre les siRNA ou ADN plasmidiques ou peptide nucléique acid (PNA) sont réalisés par mélange de 1 à 500nmol/L de siRNA re-suspendus dans de l'eau ou d'ADN plasmidique ou de PNA avec des concentrations de Z9 correspondant de 1 à 40000 nmol/L pour obtenir des rapports siRNA/Z9 allant de 1/1 à 1/5, 1/10, 1/15, 1/20, 1/40, 1/60, 1/80. Le peptide Z9 est re-suspendu dans du PBS contenant 10% de glycérol et mélangé aux concentrations indiquées ci-dessus avec les siRNA. Les mélanges Z9/acides nucléiques sont incubés sur des cellules de mammifères pendant 1 à 6 heures, puis les cellules sont lavées pour retirer l'excès de Z9/acides nucléiques et du milieu frais est rajouté aux cellules.

### Exemple 2

### Résultats

### 2.1 Identification du domaine minimal de ZEBRA

La protéine ZEBRA comprend trois domaines principaux : un domaine de trans-activation en N-terminal (TAD, résidus 1-140), un domaine de liaison à l'ADN (DB, résidus 175-195), un domaine de dimérisation (DIM, résidus 195-220) de type leucine zipper. Afin d'identifier le domaine minimal de ZEBRA requis pour effectuer une internalisation dans une lignée cellulaire mammifère, neuf différents mutants de délétion de la protéine ZEBRA complète (Z-FL) sont construits par les Inventeurs. Ces neuf mutants sont le mutant de délétion Z1 comprenant les domaines TAD, le linker et DB (acides aminés 1 à 195), le mutant de délétion Z2 comprenant les domaines TAD et le linker (acides aminés 1 à 170), le mutant de délétion Z3 comprenant uniquement le domaine TAD (acides aminés 1 à 140), le mutant de délétion Z4 comprenant les domaines linker, DB, DIM et le C-terminal (acides aminés 140 à 245), le mutant de délétion Z5 comprenant les domaines linker et DB (acides aminés 140 à 195), le mutant de délétion Z6 comprenant les domaines DB, DIM et C-terminal (acides aminés 170 à 245), le mutant de délétion Z7 comprenant le domaine DB (acides aminés 170 à 195), le mutant de délétion Z8 comprenant les domaines DIM et C-terminal (acides aminés 195 à 245) et le mutant de délétion Z9 comprenant les domaines DB et DIM (acides aminés 170 à 220) (figure 1A).

La protéine ZEBRA complète (Z-FL) ainsi que ses fragments (Z2, Z3, Z4, Z5, Z6, Z7 et Z8) sont surexprimés dans *E.coli* (Figure 1B) et ensuite purifiés selon la méthode décrite dans la partie ci-dessus 1.2.

Afin de déterminer la capacité de transduction de ces fragments de ZEBRA, les fragments Z2, Z3, Z4, Z5, Z6, Z8 et Z9 sont fusionnés avec EGFP (Enhanced Green Fluorescent Proteins).

Les protéines de fusion avec EGFP sont également surexprimées (figure 1C) et ensuite purifiées selon la méthode décrite dans la partie ci-dessus 1.2.

Les protéines de fusion sont ajoutées dans le milieu culture contenant une lignée de cellules de cancer cervical (HeLa) ou d'osteosarcoma (Saos2). Après 24 h d'incubation, la fluorescence émise par les cellules vivantes non fixées est détectée par la cytométrie en flux ou par la microscopie à fluorescence. Seule les constructions Z-FL-EGFP, Z4-EGFP, Z6-EGFP et Z9-EGFP peuvent être internalisées à l'intérieur de cellules HeLa (figures 1D). Aucun signal fluorescent n'est détecté dans les cellules incubées avec le fragment Z3-EGFP, qui contient seulement l'extrémité N-terminale de ZEBRA nature, ou le fragment Z5-EGFP, qui ne contient pas le domaine de dimérisation, ou le fragment Z8-EGFP, qui ne contient pas le domaine basique. L'efficacité d'internalisation effectuée par les fragments Z2, Z3, Z4, Z5, Z6, Z8 et Z9 est également évaluée dans la lignée cellulaire HeLa et Saos2 par cytométrie en flux après 15 h d'incubation. Cette méthode confirme l'internalisation de Z4-EGFP, Z6-EGFP et Z9-EGFP dans les deux lignées cellulaires (figure 1E). De plus, l'internalisation de Z9-EGFP dans les myoblastes de souris (C2C12) et la lignée de cellules Saos2 est observée par microscopie à fluorescence (figure 1F).

Ces résultats signifient que la présence du domaine de liaison à l'ADN (BD) et du domaine de dimérisation (DIM) est indispensable et suffisante pour effectuer l'internalisation. Le domaine minimal pour effectuer une internalisation de peptide est le mutant Z9, comprenant les domaines DB et DIM, qui permet d'effectuer une internalisation de polypeptides dans les cellules cibles avec une efficacité presque 100%.

### 2.2 Capacité de liaison à l'ADN

Etant donné que la protéine ZEBRA est un facteur de transcription qui se lie à l'ADN par son domaine de liaison à l'ADN (résidus de 175 à 195 aa), la capacité de liaison à l'ADN de différents fragments de la protéine ZEBRA est analysée par la technique du gel retard avec de l'ADN (Electrophoretic mobility shift assay). Il est déjà connu que ZEBRA reconnaît le consensus heptamère TGA ^{G}/_{C} TCA, qui peut se lier à AP-1 (activator protein). Ce heptamère est utilisé dans l'invention comme sonde pour évaluer la capacité de liaison à l'ADN. La figure 3 montre que les fragments Z4 et Z6, qui comprennent à la fois le domaine de liaison à l'ADN et le domaine de dimérisation peuvent se lier à la sonde avec une efficacité presque autant que celle de la protéine ZEBRA totale. Le fragment Z8, qui ne contient que le domaine DIM, les fragments Z5 et Z7, qui ne contiennent que le domaine DB, ou les fragments Z2 et Z3, qui ne contiennent ni le domaine DIM, ni le domaine DB, ne peuvent pas se lier à la sonde (figure 2).

Ces résultats confirment que la présence du domaine de liaison à l'ADN (BD) et du domaine de dimérisation (DIM) est indispensable et suffisante pour effectuer une liaison avec l'ADN.

En revanche, aucune protéine de fusion analysée dans cette expérience (Z6-EGFP, Z9-EFGP, Z6-βGal, Z9-βGal) ne montre une capacité de se lier à l'ADN.

### 2.3 Cinétique de l'internalisation de Z9-EGFP

La translocation de Z9-EGFP est surveillée par la mesure de la fluorescence dans les cellules vivantes par cytométrie en flux. L'addition de faible concentration de Z9-EGFP (0,2µM) dans le milieu de culture sans sérum de cellules HeLa ou Saos2 conduit à une accumulation intracellulaire rapide de protéines de fusion (Figures 3A et 3B). Après l'élimination des protéines se liant à la surface de cellules par trypsine/héparine, ces signaux sont détectés, et restent stable pendant au moins 24 heures (Figures 3A et 3B). L'augmentation de l'intensité de fluorescence dans les cellules Saos2 après la transduction est plutôt due à la grande taille de ces cellules comparées à celle des cellules HeLa, mais pas à une meilleure efficacité de translocation dans ces premières.

L'internalisation intracellulaire dose-dépendante de Z9-EGFP dans les cellules est analysée dans les cellules HeLa et Saos2. Les cellules sont incubées pendant 4 heures avec les différentes concentrations de Z9-EGFP (10, 20 100 et 200nM) (Figures 3C et 3D).

L'internalisation cellulaire de Z9-EGFP est caractérisée par l'imagerie de cellules vivantes. 0,3µM de ZEBRA-EGFP est ajouté dans les cellules HeLa et visualisés directement par microscopie à fluorescence pendant 1 heure. L'accumulation rapide de signaux d'EGFP dans les membranes cellulaires peut être observée dès la première 15 minutes. Ensuite les signaux d'EGFP sont transportés rapidement à l'intérieur de cellules.

### 2.4 Cytotoxicité de Z9-EGFP

La toxicité de Z9-EGFP et celle de Z9-βGal sont mesurées à l'aide de la déshydrogénase lactique (LDH). L'enzyme LDH est cytosolique et peut être détectée dans le milieu de culture après la rupture des membranes cellulaires. Les cellules Saos2 et HeLa sont incubées avec une protéine de fusion (Z9-EGFP ou Z9-βGal) de différentes concentrations indiquées (0,1-0,3 µM). 24 heures après l'addition des protéines de fusion, aucune différence de viabilité de cellules n'est observée entre les cellules incubées avec la protéine de fusion Z9-EGFP ou Z9-βGal et celles incubées dans le milieu de culture sans protéine de fusion (figures 3E et 3F).

### 2.5 Mécanisme d'internalisation

Les protéoglycannes de type héparane sulfate (HSPGs) joue un rôle important dans l'internalisation cellulaire effectuée par CCps.

Afin d'évaluer le rôle de HSPGs impliqué dans l'internalisation effectuée par Z9-EGFP, les cellules HeLa et Saos2 sont incubées pendant 30 minutes avec 20 µg/ml d'héparine avant l'addition de Z9-EGFP. L'héparine est un homologue structural de HSPGs et peut concurrencer la liaison de ce dernier avec Z9-EGFP. L'internalisation de Z9-EGFP est significativement inhibée par la présence d'héparine (Figure 4A). Ces résultats montrent que l'internalisation cellulaire de Z9-EGFP nécessite l'interaction entre HSPGs négativement chargés et les acides d'aminés basiques situés dans la séquence de Z9 (Figure 1G).

L'effet de la basse température et l'effet de l'épuisement de la réserve d'ATP cellulaire sur l'internalisation de fragment Z9-EGFP est analysé. Après l'incubation de cellules HeLa et Saos2 à 4°C, le signal fluorescent intracellulaire dans ces cellules est considérablement réduit (Figure 4B). Après l'épuisement de la réserve cellulaire d'ATP, on observe seulement 20-30% de diminution de fluorescence cellulaire dans toutes les lignées cellulaires étudiées (Figure 4B).

Ces résultats signifient que l'internalisation de Z9-EGFP est généralement indépendante d'ATP.

Afin de clarifier la voie d'internalisation de ZEBRA, l'effet de plusieurs inhibiteurs d'endocytose est analysé. La nystatine est un inhibiteur connu de l'endocytose dépendante de caveolin. Les cellules HeLa et Saos2 sont traitées par 50µg/ml de nystatine avant l'addition de 0,2 µM de Z9-EGFP. Dans toutes les lignées cellulaires, le signal fluorescent pour Z9-EGFP en présence de nystatine est identique à celui dans les conditions de contrôle (Figure 4C). Ces résultats montrent que l'internalisation de Z9-EGFP n'emprunte pas la voie d'endocytose dépendante de caveolin.

La macropinocytose, la voie d'internalisation empruntée par CPPs, est un mécanisme d'internalisation rapide et non-spécifique. La macropinocytose dépend de l'activité de phosphatidylinosital-3-kinase (PI3K) et est inhibée par wortmannine. L'impact de wortmannine sur les cellules HeLa et Saos2 est analysé. L'efficacité d'internalisation de Z9-EGFP dans toutes les lignées cellulaires traitées par 100nM de wortmannine n'est pas modifiée par rapport à celle dans les lignées cellulaires non traitées (Figure 4C). Ces résultats indiquent que l'internalisation par l'intermédiaire de Z9 n'emprunte pas la voie de macropinocytose.

Afin d'analyser si l'internalisation de Z9-EGFP implique l'endocytose par l'intermédiaire de CCP, l'internalisation de Z9-EGFP est mesurée en présence de chlorpromazine. Après 30 minutes d'incubation de cellules HeLa et Saos2 avec 30 µM de chlorpromazine, Z9-EGFP est ajouté dans le milieu de culture. La fluorescence émise par EGFP est considérablement réduite dans les cellules Saos2, mais pas dans les cellules HeLa (Figure 4C). Ces résultats signifient que, selon le type de cellules, il peut avoir de différents mécanismes d'internalisation de Z9-EGFP.

Afin d'analyser si l'internalisation de Z9-EGFP implique l'endocytose par l'intermédiaire de lipide rafts, les cellules sont traitées par méthyl-β-cyclodextrin pour éliminer des cholestérols associés à la surface de cellules. Ce traitement résulte d'une perturbation de lipide rafts. La figure 4C montre que l'internalisation de Z9-EGFP dans les cellules traitées est affectée par rapport à celle dans les cellules de contrôle. Ces résultats montrent que l'endocytose par l'intermédiaire de lipide rafts intervient dans l'internalisation de Z9-EGFP. Cependant, 60% de l'internalisation de Z9-EGFP emprunte un autre mécanisme. Cette constatation est soutenue par les expériences de l'entrée de Z9-EGFP dans des liposomes synthétiques (figure 7). Le colorant lipophilique, rouge foncé (10µM), est ajouté dans la préparation de liposomes synthétiques. Immédiatement après la réaction de coloration, les liposomes sont incubés pendant 30 minutes avec Z9-EGFP et analysés par microscopie confocale. La fluorescence émise par GFP est détectée au tour et à l'intérieur de vésicules lipidiques, cela signifie une translocation directe à travers la membrane lipidique.

### 2.6 Localisation intracellulaire de Z9-EGFP

La microscopie à immunofluorescence est utilisée pour étudier la co-localisation subcellulaire du peptide Z9-EGFP internalisé avec des marqueurs d'endosomes, tels que EEA1, Rab7, caveolin-1, et calthrin. Les cellules HeLa et Saos2 sont incubées pendant 30 minutes jusqu'à 15 h avec Z9-EGFP à 37°C. L'internalisation de protéine est analysée par microscopie confocale. La présence de Z9-EGFP à l'intérieur de cellules est confirmée par la visualisation directe de la fluorescence intracellulaire émise par EGFP or par un anticorps marqué qui est dirigé contre EGFP.

La majorité de signal d'EGFP n'est pas co-localisé avec celui de EEA1 et Rab7 dans une même lignée cellulaire (Figures 5A, 5B, 5C). Néanmoins, une partie de signal d'EGFP est superposé avec celui du marqueur endosomal. La variation de temps d'incubation ne modifie pas ce résultat. Par ailleurs, le signal d'EGFP n'est pas co-localisé non plus avec celui de caveolin ou clathrin (Figure 5D, 5E).

### 2.7 Internalisation de β-galactosidase dans les cellules

Afin de tester la capacité du fragment Z9 comme transporteur destiné à l'internalisation d'une molécule d'intérêt dans les cellules cibles, le fragment Z9 est fusionné à la β-galactosidase, une protéine de 120 kDa. La protéine de fusion Z9- βGal est ajoutée dans les cellules HeLa ou Saos2 dans un milieu de culture sans sérum. Les cellules sont fixées et révélées par la méthode décrite dans la partie ci-dessus. La figure 6 montre l'internalisation de β-galactosidase dans les cellules HeLa ou Saos2. La présence de β-galactosidase fonctionnelle dans les cellules est révélée par la coloration de cellules en bleu. Comme l'internalisation de EGFP par le fragment Z9, Z9-β-galactosidase sont internalisés dans 100% de population de cellules. La protéine β-galactosidase est également utilisée seule comme un témoin négatif, et aucune activité de β-galactosidase est détectée dans les cellules de contrôle.

### 2.8 Activation de la voie des caspases

L'activation de la voie des caspases est analysée dans des cellules de glioblastomes de souris (GL26) après traitement et internalisation de la protéine de fusion Z9-eIF3-f. La protéine eIF3-f peut interagir avec un isoforme de CDK11(CDK11p46) traité par des caspases. Les cellules de glioblastomes de souris (GL26) sont internalisées par la protéine de fusion Z9-eIF3-f construite par les amorces eIF3-N (D1) et eIF3-N (R1). L'activation de la voie des caspases se fait par détection de la caspase 9 activée clivée pour donner une bande à 26kDa (figure 8).

### 2.9 Internalisation de la protéine de fusion FERM-MD (Z9) dans les cellules

La protéine de fusion FERM-MD (Z9) est internalisée dans des cellules de carcinomes mammaires humains (SKBR3). La présence de la protéine de fusion dans les cellules est révélée par un anticorps anti-histidine (His-tag) après 6 heures de traitement (les figures 10A et 10B). Les protéines ErbB2 et Akt, qui expriment dans les cellules SKBR3, sont utilisées comme contrôle, et sont révélées respectivement par des anticorps anti-ErbB2 (ErbB2) et Akt (Akt) respectivement. Une diminution de l'activité et de la phosphorylation du récepteur ErbB2 et de la protéine Akt est détectée au bout de 24 heures de traitement par la protéine de fusion FERM-MD (Z9) pour des concentrations allant de 0.3 µM à 0.9 µM.

## Revendications

1. Utilisation
(i) - d'un peptide comprenant la séquence d'acides aminés SEQ ID NO : 1, ou
- d'un peptide comprenant une séquence d'acides aminés ayant 93%, notamment 95%, particulièrement 98% d'homologie d'identité de séquence avec la séquence SEQ ID NO : 1, ou
(ii) d'un acide nucléique codant
- un peptide comprenant la séquence d'acides aminés SEQ ID NO : 1, ou
- un peptide comprenant une séquence d'acides aminés ayant 93%, notamment 95%, particulièrement 98% d'homologie d'identité de séquence avec la séquence SEQ ID NO : 1, pour l'obtention d'un transporteur destiné à l'internalisation d'un polypeptide d'intérêt diagnostique ou thérapeutique dans les cellules cibles, ledit peptide étant utilisé à une concentration inférieure à 5 nM, avantageusement inférieure à 1 nM, plus avantageusement inférieure à 0,3 nM, encore plus avantageusement inférieure à 0,2nM, notamment inférieure à 0,1nM, particulièrement inférieure à 0,05nM, plus particulièrement inférieure à 0,03nM,
ledit polypeptide d'intérêt étant choisi parmi :
1) la protéine eIF3-f, telle la protéine eIF3-f de souris représentée par la séquence SEQ ID NO : 19, ou la protéine eIF3-f humaine représentée par la séquence SEQ ID NO : 20, ou un polypeptide ayant 80%, notamment 90%, particulièrement 95% d'identité de séquence avec la séquence d'une protéine eIF3-f, ou
2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

2. Utilisation selon la revendication 1, dans laquelle le polypeptide d'intérêt est lié au transporteur destiné à l'internalisation d'une molécule d'intérêt par une liaison covalente ou non covalente, telle que une liaison ionique, une liaison hydrogène, ou une liaison hydrophobique.

3. Utilisation selon la revendication 1 ou 2, dans laquelle le polypeptide d'intérêt est fusionné par une liaison peptidique au transporteur destiné à l'internalisation d'une molécule d'intérêt.

4. Utilisation selon l'une des revendications 1 à 3, dans laquelle les cellules cibles sont des cellules eucaryotes, notamment des cellules humaines.

5. Combinaison comprenant
- un polypeptide d'intérêt diagnostic ou thérapeutique, et
- un transporteur, utilisé à une concentration inférieure à 5 nM, avantageusement inférieure à 1 nM, plus avantageusement inférieure à 0,3 nM, encore plus avantageusement inférieure à 0,2nM, notamment inférieure à 0,1nM, particulièrement inférieure à 0,05nM, plus particulièrement inférieure à 0,03nM, destiné à l'internalisation de ladite molécule d'intérêt à des cellules cibles, ledit transporteur étant
- un peptide comprenant la séquence d'acides aminés SEQ ID NO : 1, ou
- un peptide comprenant une séquence d'acides aminés ayant 93%, notamment 95%, particulièrement 98% d'homologie d'identité de séquence avec la séquence SEQ ID NO : 1, ledit polypeptide d'intérêt étant choisi parmi :
1) la protéine eIF3-f, telle la protéine eIF3-f de souris représentée par la séquence SEQ ID NO : 19, ou la protéine eIf3-f humaine représentée par la séquence SEQ ID NO : 20, ou un polypeptide ayant 80%, notamment 90%, particulièrement 95% d'identité de séquence avec la séquence d'une protéine eIF3-f, ou
2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

6. Combinaison selon la revendication 5, dans laquelle le polypeptide est lié au transporteur par une liaison covalente ou non covalente, telle que une liaison ionique, une liaison hydrogène, ou une liaison hydrophobique.

7. Combinaison selon la revendication 5 ou 6, pour une utilisation dans le traitement ou à la prévention des cancers tels que mélanomes, cancer du sein, tumeurs du cerveau, telles que glioblastomes, cancer du colon, lymphomes.

8. Peptide de fusion comprenant un polypeptide d'intérêt diagnostique ou thérapeutique et un transporteur destiné à l'internalisation dudit polypeptide d'intérêt dans les cellules cibles, ledit transporteur étant
- un peptide comprenant la séquence d'acides aminés SEQ ID NO : 1, ou
- un peptide comprenant une séquence d'acides aminés ayant 80%, notamment 90%, particulièrement 95% d'homologie d'identité de séquence avec la séquence SEQ ID NO : 1, ledit polypeptide d'intérêt étant choisi parmi :
1) la protéine eIf3-f, telle la protéine eIf3-f de souris représentée par la séquence SEQ ID NO : 19, ou la protéine eIf3-f humaine représentée par la séquence SEQ ID NO : 20, ou un polypeptide ayant 80%, notamment 90%, particulièrement 95% d'identité de séquence avec la séquence d'une protéine eIf3-f, ou
2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

9. Peptide de fusion selon la revendication 8, dans lequel le polypeptide d'intérêt est fusionné à l'extrémité N-terminale du transporteur destiné à l'internalisation dudit polypeptide.

10. Peptide de fusion selon la revendication 8, dans lequel le polypeptide d'intérêt est fusionné à l'extrémité C-terminale du transporteur destiné à l'internalisation dudit polypeptide.

11. Peptide de fusion selon la revendication 8, choisi parmi :
1) le peptide de fusion représenté par la séquence SEQ ID NO : 35,
2) le peptide de fusion représenté par la séquence SEQ ID NO : 48,
3) le peptide de fusion représenté par la séquence SEQ ID NO : 40, ou
4) le peptide de fusion représenté par la séquence SEQ ID NO : 53.

12. Acide nucléique codant un peptide de fusion selon l'une des revendications 8 à 11.

13. Vecteur d'expression comprenant des acides nucléiques codant un peptide de fusion selon l'une des revendications 8 à 11.

14. Cellule hôte comprenant un vecteur d'expression selon la revendication 13.

15. Composition pharmaceutique comprenant un polypeptide d'intérêt et un transporteur de la molécule d'intérêt, ledit transporteur étant
- un peptide comprenant la séquence d'acides aminés SEQ ID NO : 1, ou
- un peptide comprenant une séquence d'acides aminés ayant 80%, notamment 90%, particulièrement 95% d'homologie d'identité de séquence avec la séquence SEQ ID NO : 1, en association avec un excipient et/ou un véhicule pharmaceutiquement acceptable ;
ledit polypeptide d'intérêt étant choisi parmi :
1) la protéine eIf3-f, telle la protéine eIf3-f de souris représentée par la séquence SEQ ID NO : 19, ou la protéine eIf3-f humaine représentée par la séquence SEQ ID NO : 20, ou un polypeptide ayant 80%, notamment 90%, particulièrement 95% d'identité de séquence avec la séquence d'une protéine eIf3-f, ou
2) la protéine FERM, telle que la protéine FERM humaine, représentée par la séquence SEQ ID NO : 27, ou une protéine ayant 80%, notamment 90%, particulièrement 95% d'identité de séquence avec la séquence représentée par la séquence SEQ ID NO : 27.

16. Composition pharmaceutique selon la revendication 15, comprenant un peptide de fusion selon l'une des revendications 8 à 11.

17. Composition pharmaceutique selon la revendication 15 ou 16, ladite composition étant formulée pour une administration journalière de 1 mg/m² à 1000 mg/m².

## Patentansprüche

1. Verwendung
(i) - eines Peptids, umfassend die Aminosäuresequenz SEQ ID NO: 1 oder
- eines Peptids, umfassend eine Aminosäuresequenz, die 93 %, bevorzugt 95 %, stärker bevorzugt 98 % Sequenzidentität mit der Sequenz SEQ ID NO: 1 aufweist, oder
(ii) einer Nukleinsäure, die kodiert für
- ein Peptid, umfassend die Aminosäuresequenz SEQ ID NO: 1 oder
- ein Peptid, umfassend eine Aminosäuresequenz, die 93 %, bevorzugt 95 %, stärker bevorzugt 98 % Sequenzidentität mit der Sequenz SEQ ID NO: 1 aufweist, zum Erhalten eines Transporters, der zur Internalisierung eines interessierenden diagnostischen oder therapeutischen Polypeptids in die Zielzellen bestimmt ist, wobei das Peptid in einer Konzentration von weniger als 5 nM, vorteilhafterweise von weniger als 1 nM, stärker bevorzugt von weniger als 0,3 nM, noch stärker bevorzugt von weniger als 0,2 nM, insbesondere von weniger als 0,1 nM, spezifisch von weniger als 0,05 nM, noch spezifischer von weniger als 0,03 nM verwendet wird, wobei das interessierende Polypeptid ausgewählt ist aus:
1) dem Protein eIF3-f, wie etwa dem Protein eIF3-f der Maus, das durch die Sequenz SEQ ID NO: 19 dargestellt wird, oder dem humanen Protein eIF3-f, das durch die Sequenz SEQ ID NO: 20 dargestellt wird, oder einem Polypeptid, das 80 %, bevorzugt 90 %, stärker bevorzugt 95 % Sequenzidentität mit der Sequenz eines Proteins eIF3-f aufweist, oder
2) dem Protein FERM, wie etwa dem humanen Protein FERM, das durch die Sequenz SEQ ID NO: 27 dargestellt wird, oder einem Protein, das 80 %, bevorzugt 90 %, stärker bevorzugt 95 % Sequenzidentität mit der Sequenz aufweist, die durch die Sequenz SEQ ID NO: 27 dargestellt wird.

2. Verwendung nach Anspruch 1, wobei das interessierende Polypeptid an den Transporter, der zur Internalisierung eines interessierenden Moleküls bestimmt ist, durch eine kovalente oder nicht kovalente Bindung, wie etwa eine ionische Bindung, eine Wasserstoffbindung oder eine hydrophobe Bindung, gebunden ist.

3. Verwendung nach Anspruch 1 oder 2, wobei das interessierende Polypeptid über eine Peptidbindung mit dem Transporter, der zur Internalisierung eines interessierenden Moleküls bestimmt ist, fusioniert ist.

4. Verwendung nach einem der Ansprüche-1 bis 3, wobei die Zielzellen eukaryotische Zellen, insbesondere humane Zellen, sind.

5. Kombination, umfassend
- ein interessierendes diagnostisches oder therapeutisches Polypeptid und
- einen Transporter, der in einer Konzentration von weniger als 5 nM, vorteilhafterweise von weniger als 1 nM, stärker bevorzugt von weniger als 0,3 nM, noch stärker bevorzugt von weniger als 0,2 nM, insbesondere von weniger als 0,1 nM, spezifisch von weniger als 0,05 nM, noch spezifischer von weniger als 0,03 nM verwendet wird, der zur Internalisierung des interessierenden Moleküls in Zielzellen bestimmt ist, wobei der Transporter Folgendes ist:
- ein Peptid, umfassend die Aminosäuresequenz SEQ ID NO: 1 oder
- ein Peptid, umfassend eine Aminosäuresequenz, die 93 %, bevorzugt 95 %, stärker bevorzugt 98 % Sequenzidentität mit der Sequenz SEQ ID NO: 1 aufweist, wobei das interessierende Polypeptid ausgewählt ist aus:
1) dem Protein eIF3-f, wie etwa dem Protein eIF3-f der Maus, das durch die Sequenz SEQ ID NO: 19 dargestellt wird, oder dem humanen Protein eIf3-f, das durch die Sequenz SEQ ID NO: 20 dargestellt wird, oder einem Polypeptid, das 80 %, bevorzugt 90 %, stärker bevorzugte 95 % Sequenzidentität mit der Sequenz eines Proteins eIF3-f aufweist, oder
2) dem Protein FERM, wie etwa dem humanen Protein FERM, das durch die Sequenz SEQ ID NO: 27 dargestellt wird, oder einem Protein, das 80 %, bevorzugt 90 %, stärker bevorzugt 95 % Sequenzidentität mit der Sequenz aufweist, die durch die Sequenz SEQ ID NO: 27 dargestellt wird.

6. Kombination nach Anspruch 5, wobei das Polypeptid an den Transporter über eine kovalente oder nicht kovalente Bindung, wie etwa eine ionische Bindung, eine Wasserstoffbindung oder eine hydrophobe Bindung, gebunden ist.

7. Kombination nach Anspruch 5 oder 6 für eine Verwendung bei der Behandlung oder der Vorbeugung von Krebsformen, wie etwa Melanomen, Brustkrebs, Gehirntumoren, wie etwa Glioblastomen, Kolonkrebs, Lymphomen.

8. Fusionspeptid, umfassend ein interessierendes diagnostisches oder therapeutisches Polypeptid und einen Transporter, der zur Internalisierung des interessierenden Polypeptids in Zielzellen bestimmt ist, wobei der Transporter Folgendes ist:
- ein Peptid, umfassend die Aminosäuresequenz SEQ ID NO: 1 oder
- ein Peptid, umfassend eine Aminosäuresequenz, die 80 %, bevorzugt 90 %, stärker bevorzugt 95 % Sequenzidentität mit der Sequenz SEQ ID NO: 1 aufweist, wobei das interessierende Polypeptid ausgewählt ist aus:
1) dem Protein eIf3-f, wie etwa dem Protein eIf3-f der Maus, das durch die Sequenz SEQ ID NO: 19 dargestellt wird, oder dem humanen Protein eIf3-f, das durch die Sequenz SEQ ID NO: 20 dargestellt wird, oder einem Polypeptid, das 80 %, bevorzugt 90 %, stärker bevorzugt 95 % Sequenzidentität mit der Sequenz eines Proteins eIf3-f aufweist, oder
2) dem Protein FERM, wie etwa dem humanen Protein FERM, das durch die Sequenz SEQ ID NO: 27 dargestellt wird, oder einem Protein, das 80 %, bevorzugt 90 %, stärker bevorzugt 95 % Sequenzidentität mit der Sequenz aufweist, die durch die Sequenz SEQ ID NO: 27 dargestellt wird.

9. Fusionspeptid nach Anspruch 8, wobei das interessierende Polypeptid mit dem N-terminalen Ende des Transporters, der zur Internalisierung des Polypeptids bestimmt ist, fusioniert ist.

10. Fusionspeptid nach Anspruch 8, wobei das interessierende Polypeptid mit dem C-terminalen Ende des Transporters, der zur Internalisierung des Polypeptids bestimmt ist, fusioniert ist.

11. Fusionspeptid nach Anspruch 8, ausgewählt aus:
1) dem Fusionspeptid, das durch die Sequenz SEQ ID NO: 35 dargestellt wird,
2) dem Fusionspeptid, das durch die Sequenz SEQ ID NO: 48 dargestellt wird,
3) dem Fusionspeptid, das durch die Sequenz SEQ ID NO: 40 dargestellt wird, oder
4) dem Fusionspeptid, das durch die Sequenz SEQ ID NO: 53 dargestellt wird.

12. Nukleinsäure, die ein Fusionspeptid gemäß einem der Ansprüche 8 bis 11 kodiert.

13. Expressionsvektor, der Nukleinsäuren umfasst, die ein Fusionspeptid gemäß einem der Ansprüche 8 bis 11 kodieren.

14. Wirtszelle, umfassend einen Expressionsvektor nach Anspruch 13.

15. Pharmazeutische Zusammensetzung, umfassend ein interessierendes Polypeptid und einen Transporter des interessierenden Moleküls, wobei der Transporter Folgendes ist:
- ein Peptid, umfassend die Aminosäuresequenz SEQ ID NO: 1 oder
- ein Peptid, umfassend eine Aminosäuresequenz, die 80 %, bevorzugt 90 %, stärker bevorzugt 95 % Sequenzidentität mit der Sequenz SEQ ID NO: 1 aufweist, in Assoziation mit einem Exzipienten und/oder einem pharmazeutisch verträglichen Vehikel; wobei das interessierende Polypeptid ausgewählt ist aus:
1) dem Protein eIf3-f, wie etwa dem Protein eIf3-f der Maus, das durch die Sequenz SEQ ID NO: 19 dargestellt wird, oder dem humanen Protein eIf3-f, das durch die Sequenz SEQ ID NO: 20 dargestellt wird, oder einem Polypeptid, das 80 %, bevorzugt 90 %, stärker bevorzugt 95 % Sequenzidentität mit der Sequenz eines Proteins eIf3-f aufweist, oder
2) dem Protein FERM, wie etwa dem humanen Protein FERM, das durch die Sequenz SEQ ID NO: 27 dargestellt wird, oder einem Protein, das 80 %, bevorzugt 90 %, stärker bevorzugt 95 % Sequenzidentität mit der Sequenz aufweist, die durch die Sequenz SEQ ID NO: 27 dargestellt wird.

16. Pharmazeutische Zusammensetzung nach Anspruch 15, umfassend ein Fusionspeptid nach einem der Ansprüche 8 bis 11.

17. Pharmazeutische Zusammensetzung nach Anspruch 15 oder 16, wobei die Zusammensetzung für eine tägliche Verabreichung von 1 mg/m² bis 1.000 mg/m² formuliert ist.

## Claims

1. Use
(i) - of a peptide comprising the amino acid sequence SEQ ID NO: 1, or
- of a peptide comprising an amino acid sequence having 93%, in particular 95%, particularly 98% sequence identity with the sequence SEQ ID NO: 1, or
(ii) - of a nucleic acid encoding
- a peptide comprising the amino acid sequence SEQ ID NO: 1, or
- a peptide comprising an amino acid sequence having 93%, in particular 95%, particularly 98% sequence identity with the sequence SEQ ID NO: 1,
in order to obtain a transporter intended for the internalization of a polypeptide of diagnostic or therapeutic interest into the target cells, said peptide being used at a concentration less than 5 nM, advantageously less than 1 nM, more advantageously less than 0.3 nM, even more advantageously less than 0.2 nM, in particular less than 0.1 nM, particularly less than 0.05 nM, more particularly less than 0.03 nM,
said polypeptide of interest being chosen from:
1) the eIF3-f protein, such as the mouse eIF3-f protein represented by the sequence SEQ ID NO: 19, or the human eIF3-f protein represented by the sequence SEQ ID NO: 20, or a polypeptide having 80%, in particular 90%, particularly 95% sequence identity with the sequence of an eIF3-f protein, or
2) the FERM protein, such as the human FERM protein, represented by the sequence SEQ ID NO: 27, or a protein having 80%, in particular 90%, particularly 95% sequence identity with the sequence represented by the sequence SEQ ID NO: 27.

2. Use according to claim 1, in which the polypeptide of interest is linked to the transporter intended for the internalization of a molecule of interest by a covalent or non-covalent bond, such as an ionic bond, a hydrogen bond, or a hydrophobic bond.

3. Use according to claim 1 or 2, in which the polypeptide of interest is fused by a peptide bond to the transporter intended for the internalization of a molecule of interest.

4. Use according to one of claims 1 to 3, in which the target cells are eukaryotic cells, in particular human cells.

5. Combination comprising
- a polypeptide of diagnostic or therapeutic interest, and
- a transporter, used at a concentration less than 5 nM, advantageously less than 1 nM, more advantageously less than 0.3 nM, even more advantageously less than 0.2 nM, in particular less than 0.1 nM, particularly less than 0.05 nM, more particularly less than 0.03 nM, intended for the internalization of said molecule of interest to target cells, said transporter being
- a peptide comprising the amino acid sequence SEQ ID NO: 1, or
- a peptide comprising an amino acid sequence having 93%, in particular 95%, particularly 98% sequence identity with the sequence SEQ ID NO: 1,
said polypeptide of interest being chosen from:
1) the eIF3-f protein, such as the mouse eIF3-f protein represented by the sequence SEQ ID NO: 19, or the human eIf3-f protein represented by the sequence SEQ ID NO: 20, or a polypeptide having 80%, in particular 90%, particularly 95% sequence identity with the sequence of an eIF3-f protein, or
2) the FERM protein, such as the human FERM protein, represented by the sequence SEQ ID NO: 27, or a protein having 80%, in particular 90%, particularly 95% sequence identity with the sequence represented by the sequence SEQ ID NO: 27.

6. Combination according to claim 5, in which the polypeptide is linked to the transporter by a covalent or non-covalent bond, such as an ionic bond, a hydrogen bond, or a hydrophobic bond.

7. Combination according to claim 5 or 6, for use in the treatment or prevention of cancers such as melanomas, breast cancer, brain tumours, such as glioblastomas, colon cancer, lymphomas.

8. Fusion peptide comprising a polypeptide of diagnostic or therapeutic interest and a transporter intended for the internalization of said polypeptide of interest into the target cells, said transporter being
- a peptide comprising the amino acid sequence SEQ ID NO: 1, or
- a peptide comprising an amino acid sequence having 80%, in particular 90%, particularly 95% sequence identity with the sequence SEQ ID NO: 1,
said polypeptide of interest being chosen from:
1) the eIf3-f protein, such as the mouse eIf3-f protein represented by the sequence SEQ ID NO: 19, or the human eIf3-f protein represented by the sequence SEQ ID NO: 20, or a polypeptide having 80%, in particular 90%, particularly 95% sequence identity with the sequence of a eIf3-f protein, or
2) the FERM protein, such as the human FERM protein, represented by the sequence SEQ ID NO: 27, or a protein having 80%, in particular 90%, particularly 95% sequence identity with the sequence represented by the sequence SEQ ID NO: 27.

9. Fusion peptide according to claim 8, in which the polypeptide of interest is fused to the N-terminal end of the transporter intended for the internalization of said polypeptide.

10. Fusion peptide according to claim 8, in which the polypeptide of interest is fused to the C-terminal end of the transporter intended for the internalization of said polypeptide.

11. Fusion peptide according to claim 8, chosen from:
1) the fusion peptide represented by the sequence SEQ ID NO: 35,
2) the fusion peptide represented by the sequence SEQ ID NO: 48,
3) the fusion peptide represented by the sequence SEQ ID NO: 40, or
4) the fusion peptide represented by the sequence SEQ ID NO: 53.

12. Nucleic acid encoding a fusion peptide according to one of claims 8 to 11.

13. Expression vector comprising nucleic acids encoding a fusion peptide according to one of claims 8 to 11.

14. Host cell comprising an expression vector according to claim 13.

15. Pharmaceutical composition comprising a polypeptide of interest and a transporter of the molecule of interest, said transporter being
- a peptide comprising the amino acid sequence SEQ ID NO: 1, or
- a peptide comprising an amino acid sequence having 80%, in particular 90%, particularly 95% sequence identity with the sequence SEQ ID NO: 1,
in combination with an excipient and/or a pharmaceutically acceptable vehicle;
said polypeptide of interest being chosen from:
1) the eIf3-f protein, such as the mouse eIf3-f protein represented by the sequence SEQ ID NO: 19, or the human eIf3-f protein represented by the sequence SEQ ID NO: 20, or a polypeptide having 80%, in particular 90%, particularly 95% sequence identity with the sequence of a eIf3-f protein, or
2) the FERM protein, such as the human FERM protein, represented by the sequence SEQ ID NO: 27, or a protein having 80%, in particular 90%, particularly 95% sequence identity with the sequence represented by the sequence SEQ ID NO: 27.

16. Pharmaceutical composition according to claim 15, comprising a fusion peptide according to one of claims 8 to 11.

17. Pharmaceutical composition according to claim 15 or 16, said composition being formulated for a daily administration of 1 mg/m² to 1000 mg/m².
